# EUROPÄISCHE PATENTSCHRIFT

(11) **EP 2 755 971 B1**
(45) Veröffentlichungstag und Bekanntmachung des Hinweises auf die Patenterteilung: **20.12.2017**
(21) Anmeldenummer: 12756492.0
(22) Anmeldetag: 11.09.2012
(51) Int. Cl.: C07D 417/14, A61K 31/454, A01N 43/78

(54) **PIPERIDINPYRAZOLE ALS FUNGIZIDE**
PIPERIDINE PYRAZOLES AS FUNGICIDES
PIPÉRIDINOPYRAZOLES À ACTION FONGICIDE

(30) Priorität: 15.09.2011 EP 11181383
(43) Veröffentlichungstag der Anmeldung: 23.07.2014
(73) Patentinhaber: Bayer Intellectual Property GmbH, 40789 Monheim (DE)
(72) Erfinder: HILLEBRAND, Stefan, 41462 Neuss (DE); TSUCHIYA, Tomoki, F-69009 Lyon (FR); HOFFMANN, Sebastian, 41470 Neuss (DE); CRISTAU, Pierre, F-69009 Lyon (FR); WASNAIRE, Pierre, 40225 Düsseldorf (DE); SEITZ, Thomas, 40764 Langenfeld (DE); BENTING, Jürgen, 42799 Leichlingen (DE); WACHENDORFF-NEUMANN, Ulrike, 56566 Neuwied (DE); SCHMIDT, Jan, Peter, 69230 Saint-Genis Laval (FR)
(74) Vertreter: BIP Patents
(86) Internationale Anmeldenummer: PCT/EP2012/067728
(87) Internationale Veröffentlichungsnummer: WO 2013/037768

(56) Entgegenhaltungen:
- WO-A1-2010/123791
- WO-A1-2011/076699
- WO-A2-2008/013622
- WO-A2-2008/013925

## Beschreibung

Die Erfindung betrifft Heteroarylpiperidin und -piperazinderivate, deren agrochemisch wirksame Salze, deren Verwendung sowie Verfahren und Mittel zur Bekämpfung von pflanzenpathogenen Schadpilzen in und/oder auf Pflanzen oder in und/oder auf Saatgut von Pflanzen, Verfahren zur Herstellung solcher Mittel und behandeltes Saatgut sowie deren Verwendung zur Bekämpfung von pflanzenpathogenen Schadpilzen in der Land-, Garten- und Forstwirtschaft, im Materialschutz sowie im Bereich Haushalt und Hygiene. Die vorliegende Erfindung betrifft weiterhin ein Verfahren zur Herstellung von Heteroarylpiperidin und -piperazinderivaten.

Es ist bereits bekannt, dass bestimmte heterocyclisch substituierte Thiazole als fungizide Pflanzenschutzmittel benutzt werden können (siehe WO 07/014290, WO 08/013925, WO 08/013622, WO 08/091594, WO 08/091580, WO 09/055514, WO 09/094407, WO 09/094445, WO 09/132785, WO 10/037479, WO 10/065579, WO10/066353, WO10/123791, WO 10/149275, WO 11/051243, WO 11/085170, WO 11/076699, WO 12/020060, WO 12/025557, WO 12/082580, WO 12/055837). Die fungizide Wirksamkeit dieser Verbindungen ist jedoch gerade bei niedrigeren Aufwandmengen nicht immer ausreichend.

Da sich die ökologischen und ökonomischen Anforderungen an moderne Pflanzenschutzmittel laufend erhöhen, beispielsweise was Wirkspektrum, Toxizität, Selektivität, Aufwandmenge, Rückstandsbildung und günstige Herstellbarkeit angeht, und außerdem z.B. Probleme mit Resistenzen auftreten können, besteht die ständige Aufgabe, neue Pflanzenschutzmittel, insbesondere Fungizide zu entwickeln, die zumindest in Teilbereichen Vorteile gegenüber den bekannten aufweisen.

Überraschenderweise wurde nun gefunden, dass die vorliegenden Heteroarylpiperidin und -piperazinderivate die genannten Aufgaben zumindest in Teilaspekten lösen und sich als Pflanzenschutzmittel, insbesondere als Fungizide eignen.

Gegenstand der Erfindung sind Verbindungen der Formel **(I),** 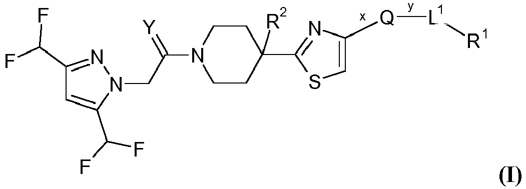 in welcher die Restedefinitionen folgende Bedeutungen haben:
- Y: steht für Sauerstoff oder Schwefel,
- R²: steht für Wassertoff oder Halogen
- Q: steht für 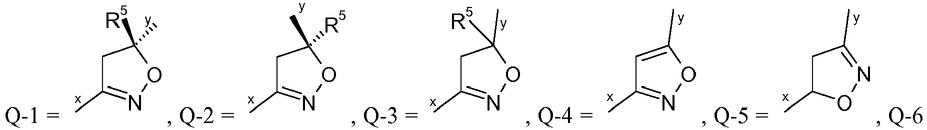 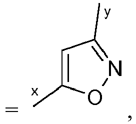
wobei die Bindung, die mit "x" identifiziert ist, direkt an das Thiazol gebunden ist, und wobei die Bindung, die mit "y" identifiziert ist, direkt an L¹ bzw. R¹ gebunden ist,
R⁵ steht für Wasserstoff, Cyano, C₁-C₃-Alkyl oder C₁-C₃-Halogenalkyl,
L¹ steht für eine direkte Bindung, -CH₂-, -(C=O)-, Schwefel oder Sauerstoff,
R¹ steht für Phenyl, das mindestens einen Substituenten Z⁴ enthält und zusätzlich zwei, drei oder vier weitere Substituenten, welche unabhängig ausgewählt sind aus Z⁴ und Z¹, enthält,
oder
R¹ steht für Naphthyl, Dihydronaphthalenyl, Tetrahydronaphthalenyl, Hexahydronaphthalenyl, Octahydronaphthalenyl oder Indenyl, das mindestens einen Substituenten Z⁵ enthält und zusätzlich zwei, drei oder vier weitere Substituenten, welche unabhängig ausgewählt sind aus Z⁵ und Z¹, enthält,
oder
R¹ steht für ein gegebenenfalls benzokondensiertes, substituiertes 5- oder 6-gliedriges Heteroaryl, das mindestens einen Substituenten Z⁶ emthält und zusätzlich zwei, drei oder vier weitere Substituenten, welche am Kohlenstoff unabhängig voneinander aus Z⁶ und Z¹ ausgewählt sind und am Stickstoff unabhängig voneinander ausgewählt sind aus Z⁶ und Z², enthält,
oder
R¹ steht für ein C₃-C₈-Cycloalkyl oder für ein C₅-C₈-Cycloalkenyl, das mindestens einen Substituenten Z⁷ enthält und zusätzlich zwei, drei oder vier weitere Substituenten, welche unabhängig voneinander ausgewählt sind aus Z⁷ und Z¹, enthält,
Z¹ steht für Halogen, Hydroxy, Nitro, Cyano, Alkyl, Alkenyl, Alkinyl, Halogenalkyl, Halogenalkenyl, Halogenalkinyl, Cycloalkyl, Halogencycloalkyl, Alkoxy, Alkoxyalkyl, Hydroxyalkyl, Halogenalkoxy, Alkylcarbonyl, Alkoxycarbonyl, Alkylcycloalkyl, Cycloalkoxyalkyl, Cycloalkylamino, Alkylthio, Halogenalkylthio, Cycloalkylthio, Cycloalkylalkyl, Alkylcarbonyloxy, Alkylcarbonylamino, Halogenalkylcarbonylamino, Alkylcarbonylthio, Alkylsulfinyl, Halogenalkylsulfinyl, Alkylsulfonyloxy, Halogenalkylsulfonyloxy, Alkylcycloalkylalkyl, -C(=O)NR³R⁴, -NR³R⁴, Tri(C₁-C₂-alkyl)silyl, oder -L³Z³,
- Z²: steht für Wasserstoff, Halogen, Hydroxy, Nitro, Cyano, Alkyl, Alkenyl, Alkinyl, Halogenalkyl, Halogenalkenyl, Halogenalkinyl, Cycloalkyl, Halogencycloalkyl, Alkoxy, Alkoxyalkyl, Hydroxyalkyl, Halogenalkoxy, Alkylcarbonyl, Alkoxycarbonyl, Alkylcycloalkyl, Cycloalkoxyalkyl, Cycloalkylamino, Alkylthio, Halogenalkylthio, Cycloalkylthio, Cycloalkylalkyl, Alkylcarbonyloxy, Alkylcarbonylamino, Halogenalkylcarbonylamino, Alkylcarbonylthio, Alkylsulfinyl, Halogenalkylsulfinyl, Alkylsulfonyloxy, Halogenalkylsulfonyloxy, Alkylcycloalkylalkyl, -C(=O)NR³R⁴, -NR³R⁴ oder Tri(C₁-C₂-alkyl)silyl,
- Z³: steht für einen Phenylrest, Naphthalenylrest oder einen 5- oder 6-gliedrigen Heteroarylrest, der jeweils 0, 1, 2 oder 3 Substituenten enthalten kann, wobei die Substituenten unabhängig voneinander ausgewählt sind aus folgender Liste:
Substituenten am Kohlenstoff: Halogen, Cyano, Nitro, Hydroxy, Amino, -SH, Alkyl, Alkenyl, Alkinyl, Halogenalkyl, Halogenalkenyl, Halogenalkinyl, Cycloalkyl, Halogencycloalkyl, Alkoxyalkyl, Alkylcarbonyl, Halogenalkylcarbonyl, Alkoxycarbonyl, Alkoxy, Halogenalkoxy, Cycloalkoxy, Halogencycloalkoxy, Alkenyloxy, Alkinyloxy, Alkoxyalkoxy, Alkylamino, Dialkylamino, Alkylthio, Halogenalkylthio, Alkylsulfinyl, Halogenalkylsulfinyl, Alkylsulfonyl, Halogenalkylsulfonyl, Trisilylalkyl oder Phenyl,
Substituenten am Stickstoff: Wasserstoff, -C(=O)H, Alkyl, Alkenyl, Alkinyl, Halogenalkyl, Halogenalkenyl, Halogenalkinyl, Cycloalkyl, Halogencycloalkyl, Alkylcycloalkyl, Cycloalkylalkyl, Alkoxyalkyl, Alkylsulfonyl, Halogenalkylsulfonyl, Cycloalkylsulfonyl, Phenylsulfonyl, Alkylcarbonyl, Halogenalkylcarbonyl, Alkoxycarbonyl, Halogenalkoxycarbonyl, Cycloalkoxycarbonyl, -C(=O)NR³R⁴, Phenyl oder Benzyl,
- Z⁴: steht für SH, C(=O)H, C₇-C₈-Cycloalkyl, C₇-C₈-Halogencycloalkyl, Cycloalkylcycloalkyl, Halogencycloalkylalkyl, Cycloalkenyl, Halogencycloalkenyl, C₅-C₆-Alkoxyalkyl, Alkoxyalkoxyalkyl, Alkylthioalkyl, Alkylsulfinylalkyl, Alkylsulfonylalkyl, Alkylaminoalkyl, Dialkylaminoalkyl, Halogenalkylaminoalkyl, Cycloalkylaminoalkyl, C₅-C₆-Alkylcarbonyl, Halogenalkylcarbonyl, Cycloalkylcarbonyl, Cycloalkoxycarbonyl, Cycloalkylalkoxycarbonyl, Cycloalkylaminocarbonyl, Halogenalkoxyalkyl, C₅-C₆-Hydroxyalkyl, C₅-C₆-Alkoxy, C₅-C₆-Halogenalkoxy, Cycloalkoxy, Halogencycloalkoxy, Cycloalkylalkoxy, Alkenyloxy, Halogenalkenyloxy, Alkinyloxy, Halogenalkinyloxy, Alkoxyalkoxy, Halogenalkylcarbonyloxy, Cycloalkylcarbonyloxy, Alkylcarbonylalkoxy, C₅-C₆-Alkylthio, C₅-C₆-Halogenalkylthio, C₅-C₆-Alkylsulfinyl, C₅-C₆-Halogenalkylsulfinyl, Alkylsulfonyl, C₁-C₄-Halogenalkylsulfonyl, Cycloalkylsulfonyl, Tri(C₃-C₄-alkyl)silyl, Alkylsulfonylamino oder Halogenalkylsulfonylamino,
- Z⁵: steht für Tri(C₂-C₄-alkyl)silyl, Benzyl, Phenyl, SH, C₅-C₆-Alkoxy, C₅-C₆-Halogenalkoxy, C₂-C₆-Alkenyloxy, C₂-C₆-Alkinyloxy, C₅-C₆-Alkylthio oder C₅-C₆-Halogenalkylthio,
- Z⁶: steht für
Substituenten am Kohlenstoff: SH, Cycloalkylcycloalkyl oder Tri(C₃-C₄-alkyl)silyl,
Substituenten am Stickstoff: Alkenyl, Alkinyl, Halogenalkyl, Halogenalkenyl, Halogenalkinyl, Cycloalkyl, Halogencycloalkyl, Alkylcycloalkyl, Cycloalkylalkyl, Cycloalkylcycloalkyl, Alkylsulfonyl, C(=O)H, Benzyl oder Phenyl,
- Z⁷: steht für Cyano, Halogen, Hydroxy, Oxo, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio oder Phenyl,
- L³: steht für eine direkte Bindung, -CH₂-, -C(=O)-, Schwefel, Sauerstoff, -C(=O)O-, -C(=O)NH-, -OC(=O)- oder -NHC(=O)-,
R³ und R⁴ stehen gleich oder verschieden und unabhängig voneinander für Wasserstoff, Alkyl, Alkenyl, Alkinyl, Halogenalkyl, Cycloalkyl, Benzyl oder Phenyl,
sowie Salze, Metallkomplexe und N-Oxide der Verbindungen der Formel **(I).**

Ein weiterer Gegenstand ist die Verwendung der Verbindungen der Formel **(I)** als Fungizide. Erfindungsgemäße Heteroarylpiperidin und -piperazinderivate der Formel **(I)** sowie deren Salze, Metallkomplexe und N-Oxide eignen sich sehr gut als zur Bekämpfung pflanzenpathogener Schadpilze. Die vorgenannten erfindungsgemäßen Verbindungen zeigen vor allem eine starke fungizide Wirksamkeit und lassen sich sowohl im Pflanzenschutz, im Bereich Haushalt und Hygiene als auch im Materialschutz verwenden.

Die Verbindungen der Formel **(I)** können sowohl in reiner Form als auch als Mischungen verschiedener möglicher isomerer Formen, insbesondere von Stereoisomeren, wie E- und Z-, threo- und erythro-, sowie optischen Isomeren, wie R- und S-Isomeren oder Atropisomeren, gegebenenfalls aber auch von Tautomeren vorliegen. Es werden sowohl die E- als auch die Z-Isomeren, wie auch die threo- und erythro-, sowie die optischen Isomeren, beliebige Mischungen dieser Isomeren, sowie die möglichen tautomeren Formen beansprucht.

Die Restedefinitionen der erfindungsgemäßen Verbindungen der Formel **(I)** haben bevorzugt, besonders bevorzugt und ganz besonders bevorzugt folgende Bedeutungen:
Y steht bevorzugt für Sauerstoff oder Schwefel und besonders bevorzugt für Sauerstoff,
R² steht bevorzugt für Wasserstoff oder Halogen, besonders bevorzugt für Wasserstoff oder Fluor und ganz besonders bevorzugt für Wasserstoff,
Q steht bevorzugt für Q-1, Q-2, Q-3, Q-4, Q-5 und Q-6, und besonders bevorzugt für Q-3 und Q-4,
wobei die Bindung, die mit "x" identifiziert ist, direkt an das Thiazol gebunden ist, und wobei die Bindung, die mit "y" identifiziert ist, direkt an L¹ bzw. R¹ gebunden ist,
- R⁵: steht bevorzugt für Wasserstoff, Cyano, Methyl, Ethyl, Trifluomethyl oder Difluormethyl, oder
- R⁵: steht besonders bevorzugt für Wasserstoff, Cyano, Methyl, Trifluomethyl oder Difluormethyl, oder
- R⁵: steht ganz besonders bevorzugt für Wasserstoff,
- L¹: steht bevorzugt für eine direkte Bindung oder Sauerstoff,
- L¹: steht besonders bevorzugt für eine direkte Bindung,
- R¹: steht bevorzugt für Phenyl, das mindestens einen Substituenten Z⁴ enthält und zusätzlich zwei oder drei weitere Substituenten, welche unabhängig voneinander ausgewählt sind aus Z⁴ und Z¹, enthält, oder
- R¹: steht besonders bevorzugt für Phenyl, das mindestens einen Substituenten Z⁴ enthält und zusätzlich zwei weitere Substituenten, welche unabhängig voneinander ausgewählt sind aus Z⁴ und Z¹, enthält, oder
- R¹: steht bevorzugt für Naphthyl, Dihydronaphthalenyl, Tetrahydronaphthalenyl, Hexahydronaphthalenyl, Octahydronaphthalenyl oder Indenyl, das mindestens einen Substituenten Z⁵ enthält und zusätzlich zwei oder drei weitere Substituenten, welche unabhängig voneinander ausgewählt sind aus Z⁵ und Z¹, enthält, oder
- R¹: steht besonders bevorzugt für Naphthyl, Dihydronaphthalenyl, Tetrahydronaphthalenyl oder Hexahydronaphthalenyl, das mindestens einen Substituenten Z⁵ enthält und zusätzlich zwei weitere Substituenten, welche unabhängig voneinander ausgewählt sind aus Z⁵ und Z¹, enthält, oder
- R¹: steht bevorzugt für ein gegebenenfalls benzokondensiertes, substituiertes 5- oder 6-gliedriges Heteroaryl, das mindestens einen Substituenten Z⁶ enthält und zusätzlich zwei oder drei weitere Substituenten, welche am Kohlenstoff unabhängig voneinander aus Z⁶ und Z¹ ausgewählt sind und am Stickstoff unabhängig voneinander ausgewählt sind aus Z⁶ und Z², enthält, oder
- R¹: steht besonders bevorzugt für Furan-2-yl, Furan-3-yl, Thiophen-2-yl, Thiophen-3-yl, Isoxazol-3-yl, Isoxazol-4-yl, Isoxazol-5-yl, Pyrrol-1-yl, Pyrrol-2-yl, Pyrrol-3-yl, Oxazol-2-yl, Oxazol-4-yl, Oxazol-5-yl, Thiazol-2-yl, Thiazol-4-yl, Thiazol-5-yl, Isothiazol-3-yl, Isothiazol-4-yl, Isothiazol-5-yl, Pyrazol-1-yl, Pyrazol-3-yl, Pyrazol-4-yl, Imidazol-1-yl, Imidazol-2-yl, Imidazol-4-yl, 1,2,4-Oxadiazol-3-yl, 1,2,4-Oxadiazol-5-yl, 1,3,4-Oxadiazol-2-yl, 1,2,4-Thiadiazol-3-yl, 1,2,4-Thiadiazol-5-yl, 1,3,4-Thiadiazol-2-yl, 1,2,3-Triazol-1-yl, 1,2,3-Triazol-2-yl, 1,2,3-Triazol-4-yl, 1,2,4-Triazol-1-yl, 1,2,4-Triazol-3-yl, 1,2,4-Triazol-4-yl, Pyridin-2-yl, Pyridin-3-yl, Pyridin-4-yl, Pyridazin-3-yl, Pyridazin-4-yl, Pyrimidin-2-yl, Pyrimidin-4-yl, Pyrimidin-5-yl oder Pyrazin-2-yl, das mindestens einen Substituenten Z⁶ enthält und zusätzlich zwei weitere Substituenten, welche am Kohlenstoff unabhängig voneinander aus Z⁶ und Z¹ ausgewählt sind und am Stickstoff unabhängig voneinander ausgewählt sind aus Z⁶ und Z², enthält, oder
- R¹: steht ganz besonders bevorzugt für Furan-2-yl, Furan-3-yl, Thiophen-2-yl, Thiophen-3-yl, Isoxazol-3-yl, Isoxazol-4-yl, Isoxazol-5-yl, Pyrrol-1-yl, Pyrrol-2-yl, Pyrrol-3-yl, Oxazol-2-yl, Oxazol-4-yl, Oxazol-5-yl, Thiazol-2-yl, Thiazol-4-yl, Thiazol-5-yl, Isothiazol-3-yl, Isothiazol-4-yl, Isothiazol-5-yl, Pyrazol-1-yl, Pyrazol-3-yl, Pyrazol-4-yl, Imidazol-1-yl, Imidazol-2-yl, Imidazol-4-yl, 1,2,4-Oxadiazol-3-yl, 1,2,4-Oxadiazol-5-yl, 1,3,4-Oxadiazol-2-yl, 1,2,4-Thiadiazol-3-yl, 1,2,4-Thiadiazol-5-yl, 1,3,4-Thiadiazol-2-yl, 1,2,3-Triazol-1-yl, 1,2,3-Triazol-2-yl, 1,2,3-Triazol-4-yl, 1,2,4-Triazol-1-yl, 1,2,4-Triazol-3-yl, 1,2,4-Triazol-4-yl, Pyridin-2-yl, Pyridin-3-yl, Pyridin-4-yl, Pyridazin-3-yl, Pyridazin-4-yl, Pyrimidin-2-yl, Pyrimidin-4-yl, Pyrimidin-5-yl oder Pyrazin-2-yl, welche jeweils 1 oder 2 Substituenten enthalten können, wobei ein Substituent ausgewählt ist aus Z⁶ und gegebenenfalls ein weiterer Substituent ausgewählt ist aus folgender Liste:
Substituenten am Kohlenstoff: Fluor, Chlor, Brom, Iod, Cyano, Nitro, Hydroxy, Methyl, Ethyl, *n-*Propyl, 1-Methylethyl, *n*-Butyl, 1,1-Dimethylethyl, 1,2-Dimethylethyl, Ethenyl, Ethinyl, Trifluormethyl, Difluormethyl, Trichlormethyl, Dichlormethyl, Cyclopropyl, Methoxy, Ethoxy, *n*-Propoxy, 1-Methylethoxy, 1,1-Dimethylethoxy, Methylcarbonyl, Ethylcarbonyl, Trifluormethylcarbonyl, Methoxycarbonyl, Ethoxycarbonyl, *n*-Propoxycarbonyl, 1-Methylethoxycarbonyl, 1,1-Dimethylethoxycarbonyl, 1-Ethenyloxy, 2-Propenyloxy, 2-Propinyloxy, Methylcarbonyloxy, Trifluoralkylcarbonyloxy, Chlormethylcarbonyloxy, Methylcarbonylamino, Trifluoralkylcarbonylamino, Chlormethylcarbonylamino, Methylthio, Ethylthio, Methylsulfinyl, Methylsulfonyl, Methylsulfonyloxy, Trifluoralkylsulfonyloxy, Methylsulfonylamino oder Trifluoromethylsulfonylamino,
   Substituenten am Stickstoff: Methyl, Ethyl, *n*-Propyl, -C(=O)H, Methylcarbonyl, Trifluormethylcarbonyl, Chlormethylcarbonyl, Methylsulfonyl, Trifluormethylsulfonyl, Phenylsulfonyl, Phenyl oder 2-Propinyl, oder
- R¹: steht besonders bevorzugt für Indol-1-yl, Indol-2-yl, Indol-3-yl, Indol-4-yl, Indol-5-yl, Indol-6-yl, Indol-7-yl, Benzimidazol-1-yl, Benzimidazol-2-yl, Benzimidazol-4-yl, Benzimidazol-5-yl, Indazol-1-yl, Indazol-3-yl, Indazol-4-yl, Indazol-5-yl, Indazol-6-yl, Indazol-7-yl, Indazol-2-yl, 1-Benzofuran-2-yl, 1-Benzofuran-3-yl, 1-Benzofuran-4-yl, 1-Benzofuran-5-yl, 1-Benzofuran-6-yl, 1-Benzofuran-7-yl, 1-Benzothiophen-2-yl, 1-Benzothiophen-3-yl, 1-Benzothiophen-4-yl, 1-Benzothiophen-5-yl, 1-Benzothiophen-6-yl, 1-Benzothiophen-7-yl, 1,3-Benzothiazol-2-yl, 1,3-Benzothiazol-4-yl, 1,3-Benzothiazol-5-yl, 1,3-Benzothiazol-6-yl, 1,3-Benzothiazol-7-yl, 1,3-Benzoxazol-2-yl, 1,3-Benzoxazol-4-yl, 1,3-Benzoxazol-5-yl, 1,3-Benzoxazol-6-yl, 1,3-Benzoxazol-7-yl, Chinolin-2-yl, Chinolin-3-yl, Chinolin-4-yl, Chinolin-5-yl, Chinolin-6-yl, Chinolin-7-yl, Chinolin-8-yl, Isochinolin-1-yl, Isochinolin-3-yl, Isochinolin-4-yl, Isochinolin-5-yl, Isochinolin-6-yl, Isochinolin-7-yl oder Isochinolin-8-yl, das mindestens einen Substituenten Z⁶ enthält und zusätzlich zwei weitere Substituenten, welche am Kohlenstoff unabhängig voneinander aus Z⁶ und Z¹ ausgewählt sind und am Stickstoff unabhängig voneinander ausgewählt sind aus Z⁶ und Z², enthält, oder
- R¹: steht besonders bevorzugt für Indol-1-yl, Indol-2-yl, Indol-3-yl, Indol-4-yl, Indol-5-yl, Indol-6-yl, Indol-7-yl, Benzimidazol-1-yl, Benzimidazol-2-yl, Benzimidazol-4-yl, Benzimidazol-5-yl, Indazol-1-yl, Indazol-3-yl, Indazol-4-yl, Indazol-5-yl, Indazol-6-yl, Indazol-7-yl, Indazol-2-yl, 1-Benzofuran-2-yl, 1-Benzofuran-3-yl, 1-Benzofuran-4-yl, 1-Benzofuran-5-yl, 1-Benzofuran-6-yl, 1-Benzofuran-7-yl, 1-Benzothiophen-2-yl, 1-Benzothiophen-3-yl, 1-Benzothiophen-4-yl, 1-Benzothiophen-5-yl, 1-Benzothiophen-6-yl, 1-Benzothiophen-7-yl, 1,3-Benzothiazol-2-yl, 1,3-Benzothiazol-4-yl, 1,3-Benzothiazol-5-yl, 1,3-Benzothiazol-6-yl, 1,3-Benzothiazol-7-yl, 1,3-Benzoxazol-2-yl, 1,3-Benzoxazol-4-yl, 1,3-Benzoxazol-5-yl, 1,3-Benzoxazol-6-yl, 1,3-Benzoxazol-7-yl, Chinolin-2-yl, Chinolin-3-yl, Chinolin-4-yl, Chinolin-5-yl, Chinolin-6-yl, Chinolin-7-yl, Chinolin-8-yl, Isochinolin-1-yl, Isochinolin-3-yl, Isochinolin-4-yl, Isochinolin-5-yl, Isochinolin-6-yl, Isochinolin-7-yl oder Isochinolin-8-yl, welche jeweils bis zu zwei Substituenten enthalten können, wobei die Substituenten unabhängig voneinander aus folgender Liste ausgewählt sind:
Substituenten am Kohlenstoff: Fluor, Chlor, Brom, Iod, Methyl, Methoxy, 2-Propinyloxy, 2-Propenyloxy,
Substituenten am Stickstoff: Methyl, Ethyl, *n*-Propyl, -C(=O)H, Methylcarbonyl, Trifluormethylcarbonyl, Chlormethylcarbonyl, Methylsulfonyl, Trifluormethylsulfonyl, Phenylsulfonyl, Phenyl oder 2-Propinyl,
- R¹: steht bevorzugt für ein C₃-C₈-Cycloalkyl oder für ein C₅-C₈-Cycloalkenyl, das mindestens einen Substituenten Z⁷ enthält und zusätzlich zwei oder drei weitere Substituenten, welche unabhängig voneinander ausgewählt sind aus Z⁷ und Z¹, enthält, oder
- R¹: steht besonders bevorzugt für ein C₃-C₈-Cycloalkyl oder für ein C₅-C₈-Cycloalkenyl, das mindestens einen Substituenten Z⁷ enthält und zusätzlich zwei weitere Substituenten, welche unabhängig voneinander ausgewählt sind aus Z⁷ und Z¹, enthält, oder
- Z¹: steht bevorzugt für Wasserstoff, Halogen, Cyano, Hydroxy, Nitro, -C(=O)NR³R⁴, -NR³R⁴, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₃-C₈-Halogencycloalkyl, C₁-C₆-Alkoxy-C₁-C₆-alkyl, C₁-C₆-Alkylcarbonyl, C₁-C₆-Alkoxycarbonyl, C₁-C₄-Alkoxy, C₁-C₄-Halogenalkoxy, C₁-C₆-Alkylcarbonyloxy, C₁-C₄-Alkylthio, C₁-C₄-Halogenalkylthio, C₃-C₆-Cycloalkylthio, Tri(C₁-C₂-alkyl)silyl, oder -L³Z³,
- Z¹: steht besonders bevorzugt für Wasserstoff, Chlor, Fluor, Brom, Cyano, Nitro, -CH₃,-CH₂CH₃, -CH₂CH₂CH₃, -CH(CH₃)₂, -CH₂CH₂CH₂CH₃, -CH(CH₃)CH₂CH₃,-CH₂CH(CH₃)CH₃, -C(CH₃)₃, -CH=CH₂, -CH=CHCH₃, -CH₂CH=CH₂, -CH=CHCH₂CH₃,-CH₂CH=CHCH₃, -CH₂CH₂CH=CH₂, -C≡CH, -C≡CCH₃, -CH₂C≡CH, -C≡CCH₂CH₃,-CH₂C≡CCH₃, -CH₂CH₂C≡CH, -CF₃, -CFH₂, -CF₂H, -CF₂CF₃, -CCl₃, Cyclopropyl, Cyclobutyl, Cyclopentyl, Cyclohexyl, -CH₂OCH₃, -CH₂OCH₂CH₃, -CH₂CH₂OCH₃,-CH₂OCH₂CH₂CH₃, -CH₂CH₂OCH₂CH₃, -CH₂CH₂CH₂OCH₃, -C(=O)CH₃, -C(=O)CH₂CH₃, C(=O)CH₂CH₂CH₃, C(=O)CH(CH₃)₂, -C(=O)CF₃, -C(=O)OCH₃, -C(=O)OCH₂CH₃,-C(=O)OCH₂CH₂CH₃, -C(=O)OCH(CH₃)₂, -OCH₃, -OCH₂CH₃, -OCH₂CH₂CH₃,-OCH(CH₃)₂, -OCH₂CH₂CH₂CH₃, -OCH₂CH(CH₃)₂, -OCH(CH₃)CH₂CH₃, -OC(CH₃)₃,-OCF₃, -OCF₂H, -OCH₂CF₃, -OCF₂CF₃, O-Cyclohexyl, O-Cyclopentyl, O-Cyclopropyl,-SCH₃, -SCH₂CH₃, -SCH₂CH₂CH₃, -SCH(CH₃)₂, -SCH₂CH₂CH₂CH₃, -SCH₂CH(CH₃)₂,-SCH(CH₃)CH₂CH₃, -SC(CH₃)₃, -SCF₃, -SCF₂H, -SCH₂CF₃, -SCF₂CF₃, -S(=O)Me, -S(O)CF₃, -S(=O)₂Me, -S(O)₂CF₃, -OCH₂CH=CH₂, -OCH₂C≡CH, -OCH₂OCH₃, -OCH₂OCH₂CH₃,-OCH₂CH₂OCH₃, -OCH₂OCH(CH₃)₂, Trimethylsilyl oder Phenyl, das 0, 1 oder 2 Substituenten enthalten kann, wobei die Substituenten unabhängig voneinander ausgewählt sind aus folgender Liste:
Chlor, Fluor, Brom, Cyano, Nitro, -CH₃, -CH₂CH₃, -CH₂CH₂CH₃, -CH(CH₃)₂,-CH₂CH₂CH₂CH₃, -CH(CH₃)CH₂CH₃, -CH₂CH(CH₃)CH₃, -C(CH₃)₃, -CH=CH₂,-CH₂CH=CH₂, -CH₂CH=CHCH₃, -CH₂CH₂CH=CH₂, -C≡CH, -C≡CCH₃, -CH₂C≡CH, -CF₃,-CF₂H, -CH₂OCH₃, -CH₂OCH₂CH₃, -CH₂CH₂OCH₃,-CH₂OCH₂CH₂CH₃, -CH₂CH₂OCH₂CH₃, -CH₂CH₂CH₂OCH₃, -C(=O)CH₃, -C(=O)CH₂CH₃, C(=O)CH₂CH₂CH₃, C(=O)CH(CH₃)₂, -C(=O)CF₃, -C(=O)OCH₃, -C(=O)OCH₂CH₃,-C(=O)OCH₂CH₂CH₃, -C(=O)OCH(CH₃)₂, -OCH₃, -OCH₂CH₃, -OCH₂CH₂CH₃,-OCH(CH₃)₂, -OCH₂CH₂CH₂CH₃, -OCH₂CH(CH₃)₂, -OCH(CH₃)CH₂CH₃, -OC(CH₃)₃,-OCF₃, -OCF₂H, -OCH₂CF₃, -OCFzCF₃, O-SCH₃, -SCH₂CH₃, -OCH₂C≡CH, -OCH₂OCH₃,
- Z²: steht bevorzugt für Wasserstoff, Halogen, Cyano, Hydroxy, Nitro, -C(=O)NR³R⁴, -NR³R⁴, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₃-C₈-Halogencycloalkyl, C₁-C₆-Alkoxy-C₁-C₆-alkyl, C₁-C₆-Alkoxycarbonyl, C₁-C₄-Alkoxy, C₁-C₄-Halogenalkoxy, C₁-C₆-Alkylcarbonyloxy, C₁-C₄-Alkylthio, C₁-C₄-Halogenalkylthio, C₃-C₆-Cycloalkylthio oder Tri(C₁-C₂-alkyl)silyl,
- Z²: steht besonders bevorzugt für Wasserstoff, Chlor, Fluor, Brom, Cyano, Nitro, -CH₃,-CH₂CH₃, -CH₂CH₂CH₃, -CH(CH₃)₂, -CH₂CH₂CH₂CH₃, -CH(CH₃)CH₂CH₃,-CH₂CH(CH₃)CH₃, -C(CH₃)₃, -CH=CH₂, -CH=CHCH₃, -CH₂CH=CH₂, -CH=CHCH₂CH₃,-CH₂CH=CHCH₃, -CH₂CH₂CH=CH₂, -C≡CH, -C≡CCH₃, -CH₂C≡CH, -C≡CCH₂CH₃,-CH₂C≡CCH₃, -CH₂CH₂C≡CH, -CF₃, -CFH₂, -CF₂H, -CF₂CF₃, -CCl₃, Cyclopropyl, Cyclobutyl, Cyclopentyl, Cyclohexyl, -CH₂OCH₃, -CH₂OCH₂CH₃, -CH₂CH₂OCH₃,-CH₂OCH₂CH₂CH₃, -CH₂CH₂OCH₂CH₃, -CH₂CH₂CH₂OCH₃, -C(=O)CH₃, -C(=O)CH₂CH₃, C(=O)CH₂CH₂CH₃, C(=O)CH(CH₃)₂, -C(=O)CF₃, -C(=O)OCH₃, -C(=O)OCH₂CH₃,-C(=O)OCH₂CH₂CH₃, -C(=O)OCH(CH₃)₂, -OCH₃, -OCH₂CH₃, -OCH₂CH₂CH₃,-OCH(CH₃)₂, -OCH₂CH₂CH₂CH₃, -OCH₂CH(CH₃)₂, -OCH(CH₃)CH₂CH₃, -OC(CH₃)₃, O-Cyclohexyl, O-Cyclopentyl, O-Cyclopropyl, -OCH₂CH=CH₂, -OCH₂C≡CH, -OCH₂OCH₃, -OCH₂OCH₂CH₃, -OCH₂CH₂OCH₃, -OCH₂OCH(CH₃)₂, Trimethylsilyl,
- Z³: steht bevorzugt für einen Phenylrest, Naphthalenyl oder einen 5- oder 6-gliedrigen Heteroarylrest, der bis zu zwei Substituenten enthalten kann, wobei die Substituenten unabhängig voneinander ausgewählt sind aus folgender Liste:
Substituenten am Kohlenstoff: Halogen, Cyano, Nitro, Hydroxy, Amino, -SH, C₁-C₄-Alkyl, C₂-C₄-Alkenyl, C₂-C₄-Alkinyl, C₁-C₄-Halogenalkyl, C₂-C₄-Halogenalkenyl, C₂-C₄-Halogenalkinyl, C₂-C₄-Alkoxyalkyl, C₁-C₆-Alkylcarbonyl, C₁-C₆-Halogenalkylcarbonyl, C₁-C₆-Alkoxycarbonyl, C₁-C₄-Alkoxy, C₁-C₄-Halogenalkoxy, C₂-C₆-Alkenyloxy, C₂-C₆-Alkinyloxy, C₁-C₄-Alkylthio, C₁-C₄-Halogenalkylthio, C₁-C₄-Alkylsulfonyl, C₁-C₄-Halogenalkylsulfonyl oder C₁-C₄-Alkylamino, Di(C₁-C₄-alkyl)amino,
Substituenten am Stickstoff: C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₁-C₄-Alkoxy-C₁-C₄-alkyl, Phenyl, Benzyl, C₁-C₄-Halogenalkylsulfonyl, C₁-C₆-Alkoxycarbonyl, C₁-C₆-Halogenalkoxycarbonyl, Phenylsulfonyl, C₁-C₄-Alkylsulfonyl, -C(=O)H, oder C₁-C₃-Alkylcarbonyl, und
- Z³: steht besonders bevorzugt für einen Phenylrest, der bis zu zwei Substituenten enthalten kann, wobei die Substituenten unabhängig voneinander ausgewählt sind aus folgender Liste:
Chlor, Brom, Iod, Fluor, Cyano, Nitro, Hydroxy, Amino, -SH, Methyl, Ethyl, *n*-Propyl, 1-Methylethyl, 1,1-Dimethylethyl, Ethenyl, Propen-2-yl, Ethinyl, Propin-2-yl, Trifluormethyl, Difluormethyl, Methoxymethyl, Methylcarbonyl, Ethylcarbonyl, Trifluormethylcarbonyl, Methoxycarbonyl, Ethoxycarbonyl, *n*-Propoxycarbonyl, 1-Methylethoxycarbonyl, 1,1-Dimethylethoxycarbonyl, Methoxy, Ethoxy, *n*-Propoxy, 1-Methylethoxy, 1,1-Dimethylethoxy, Trifluormethoxy, Ethenyloxy, 2-Propenyloxy, Ethinyloxy, 2-Propinyloxy, Methylthio, Ethylthio, Trifluormethylthio, Methylsulfonyl, Ethylsulfonyl, Propylthionyl, 1-Methylethylthio, Trifluormethylsulfonyl, Methylamino, Ethylamino, *n*-Propylamino, 1-Methylethylamino, 1,1-Dimethylethylamino oder Dimethylamino,
- Z⁴: steht bevorzugt für C(=O)H, C₇-C₈-Cycloalkyl, C₃-C₆-Cycloalkyl-C₃-C₆-cycloalkyl, C₃-C₆-Cycloalkenyl, C₄-C₆-Alkoxy-C₁-C₄-alkyl, C₃-C₆-Alkoxy-C₂-C₄-alkyl, C₁-C₃-Alkoxy-C₅-C₆-alkoxy-C₁-C₃-alkyl, C₁-C₄-Alkylthio-C₁-C₂-alkyl, C₁-C₄-Alkylsulfinyl-C₁-C₂-alkyl, C₁-C₄-Alkylsulfonyl-C₁-C₂-alkyl, C₁-C₄-Alkylamino-C₁-C₂-alkyl, C₁-C₂-Dialkylamino-C₁-C₂-alkyl, C₁-C₄-Halogenalkylamino-C₁-C₂-alkyl, C₃-C₆-Cycloalkylamino-C₁-C₂-alkyl, C₅-C₆-Alkylcarbonyl, C₁-C₄-Halogenalkylcarbonyl, C₃-C₆-Cycloalkylcarbonyl, C₃-C₆-Cycloalkoxycarbonyl, C₃-C₆-Cycloalkyl-C₁-C₂-alkoxycarbonyl, C₃-C₆-Cycloalkylaminocarbonyl, C₁-C₄-Halogenalkoxy-C₁-C₂-alkyl, C₅-C₆-Hydroxyalkyl, C₅-C₆-Alkoxy, C₅-C₆-Halogenalkoxy, C₃-C₆-Cycloalkoxy, C₃-C₆-Halogencycloalkoxy, C₃-C₆-Cycloalkylalkoxy, C₂-C₆-Alkenyloxy, C₂-C₆-Halogenalkenyloxy, C₂-C₆-Alkinyloxy, C₂-C₆-Halogenalkinyloxy, C₁-C₄-Alkoxy-C₁-C₄-alkoxy, C₁-C₄-Halogenalkylcarbonyloxy, C₃-C₆-Cycloalkylcarbonyloxy, C₁-C₄-Alkylcarbonyl-C₁-C₄-alkoxy, C₅-C₆-Alkylthio, C₅-C₆-Halogenalkylthio, C₅-C₆-Alkylsulfinyl, C₅-C₆-Halogenalkylsulfinyl, C₁-C₄-Alkylsulfonyl, C₁-C₄-Halogenalkylsulfonyl, C₃-C₆-Cycloalkylsulfonyl, Tri(C₃-C₄-alkyl)silyl, C₁-C₄-Alkylsulfonylamino oder C₁-C₄-Halogenalkylsulfonylamino,
- Z⁴: steht besonders bevorzugt für C(=O)H, Cycloheptyl, 2-Cyclopropylcycloproyl, Cyclohexenyl, *n*-Butoxymethyl, *n*-Propoxyethyl, Methoxyethoxymethyl, Ethoxyethoxymethyl, Methylthiomethyl, Ethylthiomethyl, Methylsulfinylmethyl, Ethylsulfinylmethyl, Methylsulfonylmethyl, Ethylsulfonylmethyl, Methylaminomethyl, Ethylaminomethyl, Dimethylaminomethyl, Trifluormethylaminomethyl, Cyclopropylaminomethyl, *n*-Butylcarbonyl, *n*-Pentylcarbonyl, Trifluormethylcarbonyl, Cyclopropylcarbonyl, Cyclohexylcarbonyl, Cyclopropoxycarbonyl, Cyclopentyloxycarbonyl, Cyclohexyloxycarbonyl, Cycloproylaminocarbonyl, Cyclopentylaminocarbonyl, Cyclohexylaminocarbonyl, Difluormethoxymethyl, Trifluormethoxymethyl, *n*-Pentoxy, Halogen-*n*-pentoxy, Cyclopropoxy, Cyclopentyloxy, Cyclohexyloxy, Cyclopropylmethoxy, Allyl, 3-Methylbut-2-en-1-yloxy, Prop-2-in-1-yloxy, But-2-in-1-yloxy, Pent-2-in-1-yloxy, Halogenalkinyloxy, Methoxyethoxy, Methoxypropoxy, Ethoxyethoxy, 3,3,3-Trifluorpropanyloxy, Trifluormethylcarbonyloxy, Cyclopropylcarbonyloxy, Cyclopentylcarbonyloxy, Cyclohexylcarbonyloxy, Methylcarbonylmethoxy, Pentylsulfonyl, Methylsulfonyl, Ethylsulfonyl, Trifluormethylsulfonyl, Methylsulfonylamino, Ethylsulfonylamino oder Trifluormethylsulfonylamino,
- Z⁵: steht bevorzugt für Benzyl, Phenyl, C₅-C₆-Alkoxy, C₅-C₆-Halogenalkoxy, C₂-C₄-Alkenyloxy, C₂-C₄-Alkinyloxy, C₅-C₆-Alkylthio oder C₅-C₆-Halogenalkylthio,
- Z⁶: steht bevorzugt für
Substituenten am Kohlenstoff: C₃-C₆-Cycloalkylcyclopropyl, C₃-C₆-Cycloalkylcyclohexyl oder Tri(C₃-C₄-alkyl)silyl,
Substituenten am Stickstoff: C₂-C₆-Alkenyl, C₂-C₆-Alkinyl, C₁-C₆-Halogenalkyl, C₃-C₆-Cycloalkyl, C₁-C₄-Alkyl-C₃-C₆-Cycloalkyl, C₃-C₆-Cycloalkylcyclopropyl, C₃-C₆-Cycloalkylcyclohexyl, C₃-C₆-Cycloalkyl-C₁-C₄-alkyl, C₁-C₄-Alkylsulfonyl, C(=O)H, Benzyl oder Phenyl,
- L³: steht bevorzugt für eine direkte Bindung, -CH₂-, Schwefel, Sauerstoff, und besonders bevorzugt für eine direkte Bindung,
- R³ und R⁴: stehen bevorzugt gleich oder verschieden und unabhängig voneinander für Wasserstoff, C₁-C₄-Alkyl, C₂-C₄-Alkenyl, C₂-C₄-Alkinyl, C₁-C₄-Halogenalkyl, C₅-C₆-Cycloalkyl, Benzyl oder Phenyl,
- R³ und R⁴: stehen besonders bevorzugt gleich oder verschieden und unabhängig voneinander für Wasserstoff, Methyl, Ethyl, Propyl, Prop-2-in-1-yl, 1,1,1-Trifluorethyl, Cyclohexyl, Benzyl oder Phenyl.

Die erfindungsgemäß verwendbaren Heteroarylpiperidin und -piperazinderivate sind durch die Formel **(I)** allgemein definiert. Die Restedefinitionen der vorstehenden und nachfolgend genannten Restedefinitionen der Formel **(I)** gelten für die Endprodukte der Formel **(I)** wie für alle Zwischenprodukte (siehe auch unten unter "Erläuterungen der Verfahren und Zwischenprodukte") gleichermaßen.

Die oben aufgeführten bzw. nachfolgenden allgemeinen oder in Vorzugsbereichen aufgeführten Restedefinitionen bzw. Erläuterungen können auch untereinander, also zwischen den jeweiligen Bereichen und Vorzugsbereichen beliebig kombiniert werden. Sie gelten für die Endprodukte sowie für die Vor- und Zwischenprodukte entsprechend. Außerdem können einzelne Definitionen entfallen.

Bevorzugt sind solche Verbindungen der Formel **(I),** in welcher alle Reste jeweils die oben genannten bevorzugten Bedeutungen haben.

Besonders bevorzugt sind solche Verbindungen der Formel **(I),** in welcher alle Reste jeweils die oben genannten besonders bevorzugten Bedeutungen haben.

Ganz besonders bevorzugt sind solche Verbindungen der Formel **(I),** in welcher alle Reste jeweils die oben genannten ganz besonders bevorzugten Bedeutungen haben.

Weiterhin bevorzugt sind Verbindungen der Formel **(I)** sowie agrochemisch wirksame Salze, Metallkomplexe und N-Oxide davon in welcher:
- Y: für Sauerstoff steht;
- R²: für Wasserstoff steht;
- Q: für Q-3 steht;
- R⁵: für Wasserstoff steht;
- L¹: für eine direkte Bindung steht;
- R¹: für 4,5-Dimethyl-2-(prop-2-in-1-yloxy)phenyl steht oder,
- R¹: für 2,6-Difluor-3-(prop-2-in-1-yloxy)phenyl steht oder,
- R¹: für 2,6-Difluor-4-(prop-2-in-1-yloxy)phenyl steht oder,
- R¹: für 2,6-Difluor-4-[(methylsulfonyl)amino]phenyl steht oder,
- R¹: für 3-(Allyloxy)-2,6-difluorphenyl steht.

Die zuvor genannten Reste-Definitionen können untereinander in beliebiger Weise kombiniert werden. Außerdem können einzelne Definitionen entfallen.

Je nach Art der oben definierten Substituenten weisen die Verbindungen der Formel **(I)** saure oder basische Eigenschaften auf und können mit anorganischen oder organischen Säuren oder mit Basen oder mit Metallionen Salze, gegebenenfalls auch innere Salze oder Addukte bilden. Tragen die Verbindungen der Formel **(I)** Amino, Alkylamino oder andere, basische Eigenschaften induzierende Gruppen, so können diese Verbindungen mit Säuren zu Salzen umgesetzt werden oder fallen durch die Synthese direkt als Salze an. Tragen die Verbindungen der Formel **(I)** Hydroxy, Carboxy oder andere, saure Eigenschaften induzierende Gruppen, so können diese Verbindungen mit Basen zu Salzen umgesetzt werden. Geeignete Basen sind beispielsweise Hydroxide, Carbonate, Hydrogencarbonate der Alkali- und Erdalkalimetalle, insbesondere die von Natrium, Kalium, Magnesium und Calcium, weiterhin Ammoniak, primäre, sekundäre und tertiäre Amine mit C₁-C₄-Alkyl-Gruppen, Mono-, Di- und Trialkanolamine von C₁-C₄-Alkanolen, Cholin sowie Chlorcholin.

Die so erhältlichen Salze weisen ebenfalls fungizide Eigenschaften auf.

Beispiele für anorganische Säuren sind Halogenwasserstoffsäuren wie Fluorwasserstoff, Chlorwasserstoff, Bromwasserstoff und Iodwasserstoff, Schwefelsäure, Phosphorsäure und Salpetersäure und saure Salze wie NaHSO₄ und KHSO₄. Als organische Säuren kommen beispielsweise Ameisensäure, Kohlensäure und Alkansäuren wie Essigsäure, Trifluoressigsäure, Trichloressigsäure und Propionsäure sowie Glycolsäure, Thiocyansäure, Milchsäure, Bernsteinsäure, Zitronensäure, Benzoesäure, Zimtsäure, Oxal-säure, gesättigte oder einfach oder doppelt ungesättigte C₆-C₂₀-Fettsäuren, Alkylschwefelsäuremonoester, Alkylsulfonsäuren (Sulfonsäuren mit geradkettigen oder verzweigten Alkylresten mit 1 bis 20 Kohlenstoffatomen), Arylsulfonsäuren oder Aryldisulfonsäuren (aromatische Reste wie Phenyl und Naphthyl welche ein oder zwei Sulfonsäuregruppen tragen), Alkylphosphonsäuren (Phosphonsäuren mit geradkettigen oder verzweigten Alkylresten mit 1 bis 20 Kohlenstoffatomen), Arylphosphonsäuren oder Aryldiphosphonsäuren (aromatische Reste wie Phenyl und Naphthyl welche ein oder zwei Phosphonsäurereste tragen), wobei die Alkyl- bzw. Arylreste weitere Substituenten tragen können, z.B. p-Toluolsulfonsäure, Salicylsäure, p-Aminosalicylsäure, 2-Phenoxybenzoesäure, 2-Acetoxybenzoesäure etc.

Als Metallionen kommen insbesondere die Ionen der Elemente der zweiten Hauptgruppe, insbesondere Calzium und Magnesium, der dritten und vierten Hauptgruppe, insbesondere Aluminium, Zinn und Blei, sowie der ersten bis achten Nebengruppe, insbesondere Chrom, Mangan, Eisen, Kobalt, Nickel, Kupfer, Zink und andere in Betracht. Besonders bevorzugt sind die Metallionen der Elemente der vierten Periode. Die Metalle können dabei in den verschiedenen ihnen zukommenden Wertigkeiten vorliegen.

Gegebenenfalls substituierte Gruppen können einfach oder mehrfach substituiert sein, wobei bei Mehrfachsubstitutionen die Substituenten gleich oder verschieden sein können.

Bei den in den vorstehenden Formeln angegebenen Definitionen der Symbole wurden Sammelbegriffe verwendet, die allgemein repräsentativ für die folgenden Substituenten stehen:
**Halogen:** Fluor, Chlor, Brom und Iod und vorzugsweise Fluor, Chlor, Brom und noch bevorzugter Fluor, Chlor.

**Alkyl:** gesättigte, geradkettige oder verzweigte Kohlenwasserstoffreste mit 1 bis 8 vorzugsweise 1 bis 6 und noch bevorzugter 1 bis 3 Kohlenstoffatomen, z.B. (aber nicht beschränkt auf) C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl,1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl. Diese Definition gilt auch für Alkyl as Bestandteil eines zusammengesetzen Substitutenten wie z.B. Cycloalkylalkyl, Hydroxyalkyl etc. sofern an anderer Stelle nicht definiert wie z.B. Alkylthio, Alkylsufinyl, Alkylsulfonyl, Halogenalkyl bzw. Halogenalkylthio. Steht das Alkyl am Ende eines zusammengesetzten Substituenten. wie z.B bei Alkylcylcloalkyl, so kann der am Anfang stehende Bestandteil des zusammengesetzten Substituenten, z.B. das Cycloalkyl, ein- bzw. mehrfach, gleich oder verschieden und unabhängig voneinander mit Alkyl substituiert sein. Das gleiche gilt auch für zusammengesetzte Substituenten bei denen andere Reste wie z.B. Alkenyl, Alkinyl, Hydroxy, Halogen, Formyl etc. am Ende stehen.

**Alkenyl:** ungesättigte, geradkettige oder verzweigte Kohlenwasserstoffreste mit 2 bis 8 vorzugsweise 2 bis 6 Kohlenstoffatomen und einer Doppelbindung in einer beliebigen Position, z.B. (aber nicht beschränkt auf) C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1,-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl. Diese Definition gilt auch für Alkenyl als Bestandteil eines zusammengesetzen Substitutenten wie z.B. Halogenalkenyl etc. sofern an anderer Stelle nicht definiert;

**Alkinyl:** geradkettige oder verzweigte Kohlenwasserstoffgruppen mit 2 bis 8 vorzugsweise 2 bis 6 Kohlenstoffatomen und einer Dreifachbindung in einer beliebigen Position, z.B. (aber nicht beschränkt auf) C₂-C₆-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl. Diese Definition gilt auch für Alkinyl als Bestandteil eines zusammengesetzen Substitutenten wie z.B. Halogenalkinyl etc. sofern an anderer Stelle nicht definiert;

**Alkoxy:** gesättigte, geradkettige oder verzweigte Alkoxyreste mit 1 bis 8 vorzugsweise 1 bis 6 und noch bevorzugter 1 bis 3 Kohlenstoffatomen, z.B. (aber nicht beschränkt auf) C1-C6-Alkoxy wie Methoxy, Ethoxy, Propoxy, 1-Methylethoxy, Butoxy, 1-Methyl-propoxy, 2-Methylpropoxy, 1,1-Dimethylethoxy, Pentoxy, 1-Methylbutoxy, 2-Methylbutoxy, 3-Methylbutoxy, 2,2-Dimethylpropoxy, 1-Ethylpropoxy, Hexoxy, 1,1-Dimethylpropoxy, 1,2-Dimethylpropoxy,1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methylpropoxy. Diese Definition gilt auch für Alkoxy als Bestandteil eines zusammengesetzen Substitutenten wie z.B. Halogenalkoxy, Alkinylalkoxy etc. sofern an anderer Stelle nicht definiert;

**Alkylthio:** gesättigte, geradkettige oder verzweigte Alkylthioreste mit 1 bis 8 vorzugsweise 1 bis 6 und noch bevorzugter 1 bis 3 Kohlenstoffatomen, z.B. (aber nicht beschränkt auf) C₁-C₆-Alkylthio wie Methylthio, Ethylthio, Propylthio, 1-Methylethylthio, Butylthio, 1-Methyl-propylthio, 2-Methylpropylthio, 1,1-Dimethylethylthio, Pentylthio, 1-Methylbutylthio, 2-Methylbutylthio, 3-Methylbutylthio, 2,2-Di-methylpropylthio, 1-Ethylpropylthio, Hexylthio, 1,1-Dimethylpropylthio, 1,2-Dimethylpropylthio,1-Methylpentylthio, 2-Methylpentylthio, 3-Methyl-pentylthio, 4-Methylpentylthio, 1,1-Dimethylbutylthio, 1,2-Dimethylbutylthio, 1,3-Dimethyl-butylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropylthio und 1-Ethyl-2-methylpropylthio. Diese Definition gilt auch für Alkylthio als Bestandteil eines zusammengesetzen Substitutenten wie z.B. Halogenalkylthio etc. sofern an anderer Stelle nicht definiert;

**Alkoxycarbonyl:** eine Alkoxygruppe mit 1 bis 6 bevorzugt 1 bis 3 Kohlenstoffatomen (wie vorstehend genannt), welche über eine Carbonylgruppe (-CO-) an das Gerüst gebunden ist. Diese Definition gilt auch für Alkoxycarbonyl als Bestandteil eines zusammengesetzen Substitutenten wie z.B. Cycloalkylalkoxycarbonyl etc. sofern an anderer Stelle nicht definiert;

**Alkylsulfinyl:** gesättigte, geradkettige oder verzweigte Alkylsulfinylreste mit 1 bis 8 vorzugsweise 1 bis 6 und noch bevorzugter 1 bis 3 Kohlenstoffatomen, z.B. (aber nicht beschränkt auf) C₁-C₆-Alkylsulfinyl wie Methylsulfinyl, Ethylsulfinyl, Propylsulfinyl, 1-Methylethylsulfinyl, Butylsulfinyl, 1-Methyl-propylsulfinyl, 2-Methylpropylsulfinyl, 1,1-Dimethylethylsulfinyl, Pentylsulfinyl, 1-Methylbutylsulfinyl, 2-Methylbutylsulfinyl, 3-Methylbutylsulfinyl, 2,2-Dimethylpropylsulfinyl, 1-Ethylpropylsulfinyl, Hexylsulfinyl, 1,1-Dimethylpropylsulfinyl, 1,2-Dimethylpropylsulfinyl,1-Methylpentylsulfinyl, 2-Methylpentyl-sulfinyl, 3-Methylpentylsulfinyl, 4-Methylpentylsulfinyl, 1,1-Dimethylbutylsulfinyl, 1,2-Dimethylbutylsulfinyl, 1,3-Dimethylbutylsulfinyl, 2,2-Dimethylbutylsulfinyl, 2,3-Dimethylbutylsulfinyl, 3,3-Dimethylbutylsulfinyl, 1-Ethylbutylsulfinyl, 2-Ethylbutylsulfinyl, 1,1,2-Trimethylpropylsulfinyl, 1,2,2-Trimethylpropylsulfinyl, 1-Ethyl-1-methylpropylsulfinyl und 1-Ethyl-2-methylpropylsulfinyl. Diese Definition gilt auch für Alkylsulfinyl als Bestandteil eines zusammengesetzen Substitutenten wie z.B. Halogenalkylsulfinyl etc. sofern an anderer Stelle nicht definiert;

**Alkylsulfonyl:** gesättigte, geradkettige oder verzweigte Alkylsulfonylreste mit 1 bis 8 vorzugsweise 1 bis 6 und noch bevorzugter 1 bis 3 Kohlenstoffatomen, z.B. (aber nicht beschränkt auf) C₁-C₆-Alkylsulfonyl wie Methylsulfonyl, Ethylsulfonyl, Propylsulfonyl, 1-Methylethylsulfonyl, Butylsulfonyl, 1-Methyl-propylsulfonyl, 2-Methylpropylsulfonyl, 1,1-Dimethylethylsulfonyl, Pentylsulfonyl, 1-Methylbutylsulfonyl, 2-Methylbutylsulfonyl, 3-Methylbutylsulfonyl, 2,2-Dimethylpropylsulfonyl, 1-Ethylpropylsulfonyl, Hexylsulfonyl, 1,1-Dimethylpropylsulfonyl, 1,2-Dimethylpropylsulfonyl,1-Methylpentylsulfonyl, 2-Methylpentyl-sulfonyl, 3-Methylpentylsulfonyl, 4-Methylpentylsulfonyl, 1,1-Dimethylbutylsulfonyl, 1,2-Dimethylbutylsulfonyl, 1,3-Dimethylbutylsulfonyl, 2,2-Dimethylbutylsulfonyl, 2,3-Dimethylbutylsulfonyl, 3,3-Dimethylbutylsulfonyl, 1-Ethylbutylsulfonyl, 2-Ethylbutylsulfonyl, 1,1,2-Trimethylpropylsulfonyl, 1,2,2-Trimethylpropylsulfonyl, 1-Ethyl-1-methylpropylsulfonyl und 1-Ethyl-2-methylpropylsulfonyl. Diese Definition gilt auch für Alkylsulfonyl als Bestandteil eines zusammengesetzen Substitutenten wie z.B. Alkylsulfonylalkyl etc. sofern an anderer Stelle nicht definiert;

**Cycloalkyl:** monocyclische, gesättigte Kohlenwasserstoffgruppen mit 3 bis 10 vorzugsweise 3 bis 8 und noch bevorzugter 3 bis 6 Kohlenstoffringgliedern, z.B. (aber nicht beschränkt auf) Cyclopropyl, Cyclopentyl und Cyclohexyl. Diese Definition gilt auch für Cycloalkyl als Bestandteil eines zusammengesetzen Substitutenten wie z.B. Cycloalkylalkyl etc. sofern an anderer Stelle nicht definiert;

**Cycloalkenyl:** monocyclische, partiell ungesättigter Kohlenwasserstoffgruppen mit 3 bis 10 vorzugsweise 3 bis 8 und noch bevorzugter 3 bis 6 Kohlenstoffringgliedern, z.B. (aber nicht beschränkt auf) Cyclopropenyl, Cyclopenentyl und Cyclohexenyl. Diese Definition gilt auch für Cycloalkenyl als Bestandteil eines zusammengesetzen Substitutenten wie z.B. Cycloalkyenlalkyl etc. sofern an anderer Stelle nicht definiert;

**Cycloalkoxy:** monocyclische, gesättigte Cycloalkyloxyreste mit 3 bis 10 vorzugsweise 3 bis 8 und noch bevorzugter 3 bis 6 Kohlenstoffringgliedern, z.B. (aber nicht beschränkt auf) Cyclopropyloxy, Cyclopentyloxy und Cyclohexyloxy. Diese Definition gilt auch für Cycloalkoxy als Bestandteil eines zusammengesetzen Substitutenten wie z.B. Cycloalkoxyalkyl etc. sofern an anderer Stelle nicht definiert;

**Halogenalkyl:** geradkettige oder verzweigte Alkylgruppen mit 1 bis 8 vorzugsweise 1 bis 6 und noch bevorzugter 1 bis 3 Kohlenstoffatomen (wie vorstehend genannt), wobei in diesen Gruppen teilweise oder vollständig die Wasserstoffatome durch Halogenatome wie vorstehend genannt ersetzt sein können, z.B. (aber nicht beschränkt auf) C₁-C₃-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor,2-difluorethyl, 2,2-Dichlor-2-fluorethyl, 2,2,2-Trichlorethyl, Pentafluorethyl und 1,1,1-Trifluorprop-2-yl. Diese Definition gilt auch für Halogenalkyl als Bestandteil eines zusammengesetzen Substitutenten wie z.B. Halogenalkylaminoalkyl etc. sofern an anderer Stelle nicht definiert;
Halogenalkenyl und Halogenalkinyl sind analog wie Halogenalkyl definiert, wobei anstatt Alkylgruppen, Alkenyl und Alkinylgruppen als Bestandteil des Substituenten vorliegen.

**Halogenalkoxy:** geradkettige oder verzweigte Alkoxygruppen mit 1 bis 8 vorzugsweise 1 bis 6 und noch bevorzugter 1 bis 3 Kohlenstoffatomen (wie vorstehend genannt), wobei in diesen Gruppen teilweise oder vollständig die Wasserstoffatome durch Halogenatome wie vorstehend genannt ersetzt sein können, z.B. (aber nicht beschränkt auf) C₁-C₃-Halogenalkoxy wie Chlormethoxy, Brommethoxy, Dichlormethoxy, Trichlormethoxy, Fluormethoxy, Difluormethoxy, Trifluormethoxy, Chlorfluormethoxy, Dichlorfluormethoxy, Chlordifluormethoxy, 1-Chlorethoxy, 1-Bromethoxy, 1-Fluorethoxy, 2-Fluorethoxy, 2,2-Difluorethoxy, 2,2,2-Trifluorethoxy, 2-Chlor-2-fluorethoxy, 2-Chlor,2-difluorethoxy, 2,2-Dichlor-2-fluorethoxy, 2,2,2-Trichlorethoxy, Pentafluorethoxy und 1,1,1-Trifluorprop-2-oxy. Diese Definition gilt auch für Halogenalkoxy als Bestandteil eines zusammengesetzen Substitutenten wie z.B. Halogenalkoxyalkyl etc. sofern an anderer Stelle nicht definiert;

**Halogenalkylthio:** geradkettige oder verzweigte Alkylthiogruppen mit 1 bis 8 vorzugsweise 1 bis 6 und noch bevorzugter 1 bis 3 Kohlenstoffatomen (wie vorstehend genannt), wobei in diesen Gruppen teilweise oder vollständig die Wasserstoffatome durch Halogenatome wie vorstehend genannt ersetzt sein können, z.B. (aber nicht beschränkt auf) C₁-C₃-Halogenalkylthio wie Chlormethylthio, Brommethylthio, Dichlormethylthio, Trichlormethylthio, Fluormethylthio, Difluormethylthio, Trifluormethylthio, Chlorfluormethylthio, Dichlorfluor-methylthio, Chlordifluormethylthio, 1-Chlorethylthio, 1-Bromethylthio, 1-Fluorethylthio, 2-Fluorethylthio, 2,2-Difluorethylthio, 2,2,2-Trifluorethylthio, 2-Chlor-2-fluorethylthio, 2-Chlor,2-difluorethylthio, 2,2-Dichlor-2-fluorethylthio, 2,2,2-Trichlorethylthio, Pentafluorethylthio und 1,1,1-Trifluorprop-2-ylthio. Diese Definition gilt auch für Halogenalkylthio als Bestandteil eines zusammengesetzen Substitutenten wie z.B. Halogenalkylthioalkyl etc. sofern an anderer Stelle nicht definiert;

**Heteroaryl:** 5 oder 6-gliedriges, vollständig ungesättigtes monocyclisches Ringsystem, enthaltend ein bis vier Heteroatome aus der Gruppe Sauerstoff, Stickstoff oder Schwefel, enthält der Ring mehrere Sauerstoffatome, so stehen diese nicht direkt benachbart;

**5-gliedriges Heteroaryl: enthaltend ein bis vier Stickstoffatome oder ein bis drei Stickstoffatome und ein Schwefel- oder Sauerstoffatom:** 5-Ring Heteroarylgruppen, welche neben Kohlenstoffatomen ein bis vier Stickstoffatome oder ein bis drei Stickstoffatome und ein Schwefel- oder Sauerstoffatom als Ringglieder enthalten können, z.B. (aber nicht beschränkt auf) 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5- Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl und 1,3,4-Triazol-2-yl;

**über Stickstoff gebundenes 5-gliedriges Heteroaryl, enthaltend ein bis vier Stickstoffatome, oder über Stickstoff gebundenes benzokondensiertes 5-gliederiges Heteroaryl, enthaltend ein bis drei Stickstoffatome:** 5-Ring Heteroarylgruppen, welche neben Kohlenstoffatomen ein bis vier Stickstoffatome bzw. ein bis drei Stickstoffatome als Ringglieder enthalten können, und in welchen zwei benachbarte Kohlenstoffringglieder oder ein Stickstoff- und ein benachbartes Kohlenstoffringglied durch eine Buta-1,3-dien-1,4-diylgruppe verbrückt sein können, in der ein oder zwei C-Atome durch N-Atome ersetzt sein können, in der ein oder zwei C-Atome durch N-Atome ersetzt sein können, wobei diese Ringe über eines der Stickstoffringglieder an das Gerüst gebunden sind, z.B. (aber nicht beschränkt auf) 1-Pyrrolyl, 1-Pyrazolyl, 1,2,4-Triazol-1-yl, 1-Imidazolyl, 1,2,3-Triazol-1-yl und 1,3,4-Triazol-1-yl;

**6-gliedriges Heteroaryl, enthaltend ein bis vier Stickstoffatome:** 6-Ring Heteroarylgruppen, welche neben Kohlenstoffatomen ein bis drei bzw. ein bis vier Stickstoffatome als Ringglieder enthalten können, beispielsweise (aber nicht beschränkt auf) 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl und 1,2,4,5-Tetrazin-3-yl;

**Benzokondensiertes 5-gliedriges Heteroaryl, enthaltend ein bis drei Stickstoffatome oder ein Stickstoffatom und ein Sauerstoff- oder Schwefelatom:** z.B. (aber nicht beschränkt auf) Indol-1-yl, Indol-2-yl, Indol-3-yl, Indol-4-yl, Indol-5-yl, Indol-6-yl, Indol-7-yl, Benzimidazol-1-yl, Benzimidazol-2-yl, Benzimidazol-4-yl, Benzimidazol-5-yl, Indazol-1-yl, Indazol-3-yl, Indazol-4-yl, Indazol-5-yl, Indazol-6-yl, Indazol-7-yl, Indazol-2-yl, 1-Benzofuran-2-yl, 1-Benzofuran-3-yl, 1-Benzofuran-4-yl, 1-Benzofuran-5-yl, 1-Benzofuran-6-yl, 1-Benzofuran-7-yl, 1-Benzothiophen-2-yl, 1-Benzothiophen-3-yl, 1-Benzothiophen-4-yl, 1-Benzothiophen-5-yl, 1-Benzothiophen-6-yl, 1-Benzothiophen-7-yl, 1,3-Benzothiazol-2-yl, 1,3-Benzothiazol-4-yl, 1,3-Benzothiazol-5-yl, 1,3-Benzothiazol-6-yl, 1,3-Benzothiazol-7-yl, 1,3-Benzoxazol-2-yl, 1,3-Benzoxazol-4-yl, 1,3-Benzoxazol-5-yl, 1,3-Benzoxazol-6-yl und 1,3-Benzoxazol-7-yl,

**Benzokondensiertes 6-gliedriges Heteroaryl, enthaltend ein bis drei Stickstoffatome: :** z.B. (aber nicht beschränkt auf) Chinolin-2-yl, Chinolin-3-yl, Chinolin-4-yl, Chinolin-5-yl, Chinolin-6-yl, Chinolin-7-yl, Chinolin-8-yl, Isochinolin-1-yl, Isochinolin-3-yl, Isochinolin-4-yl, Isochinolin-5-yl, Isochinolin-6-yl, Isochinolin-7-yl, und Isochinolin-8-yl;

Diese Definition gilt auch für Heteroaryl als Bestandteil eines zusammengesetzen Substitutenten wie z.B. Heteroarylalkyl etc. sofern an anderer Stelle nicht definiert;

**Heterocyclyl:** drei- bis fünfzehngliedriger vorzugsweise ein drei- bis neungliedriger gesättigter oder partiell ungesättigter Heterocyclus, enthaltend ein bis vier Heteroatome aus der Gruppe Sauerstoff, Stickstoff oder Schwefel: mono-, bi- oder tricyclische Heterocyclen enthaltend neben Kohlenstoffringgliedern ein bis drei Stickstoffatome und/oder ein Sauerstoff- oder Schwefelatom oder ein oder zwei Sauerstoff- und/oder Schwefelatome; enthält der Ring mehrere Sauerstoffatome, so stehen diese nicht direkt benachbart; wie z.B. (aber nicht beschränkt auf) Oxiranyl, Aziridinyl, 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,4-Dihydrofur-2-yl, 2,4-Dihydrofur-3-yl, 2,3-Dihydrothien-2-yl, 2,3-Dihydrothien-3-yl, 2,4-Dihydrothien-2-yl, 2,4-Dihydrothien-3-yl, 2-Pyrrolin-2-yl, 2-Pyrrolin-3-yl, 3-Pyrrolin-2-yl, 3-Pyrrolin-3-yl, 2-Isoxazolin-3-yl, 3-Isoxazolin-3-yl, 4-Isoxazolin-3-yl, 2-Isoxazolin-4-yl, 3-Isoxazolin-4-yl, 4-Isoxazolin-4-yl, 2-Isoxazolin-5-yl, 3-Isoxazolin-5-yl, 4-Isoxazolin-5-yl, 2-Isothiazolin-3-yl, 3-Isothiazolin-3-yl, 4-Isothiazolin-3-yl, 2-Isothiazolin-4-yl, 3-Isothiazolin-4-yl, 4-Isothiazolin-4-yl, 2-Isothiazolin-5-yl, 3-Isothiazolin-5-yl, 4-Isothiazolin-5-yl, 2,3-Dihydropyrazol-1-yl, 2,3-Dihydropyrazol-2-yl, 2,3-Dihydropyrazol-3-yl, 2,3-Dihydropyrazol-4-yl, 2,3-Dihydropyrazol-5-yl, 3,4-Dihydropyrazol-1-yl, 3,4-Dihydropyrazol-3-yl, 3,4-Dihydropyrazol-4-yl, 3,4-Dihydropyrazol-5-yl, 4,5-Dihydroopyrazol-1-yl, 4,5-Dihydropyrazol-3-yl, 4,5-Dihydropyrazol-4-yl, 4,5-Dihydropyrazol-5-yl, 2,3-Dihydrooxazol-2-yl, 2,3-Dihydrooxazol-3-yl, 2,3-Dihydrooxazol-4-yl, 2,3-Dihydrooxazol-5-yl, 3,4-Dihydrooxazol-2-yl, 3,4-Dihydrooxazol-3-yl, 3,4-Dihydrooxazol-4-yl, 3,4-Dihydrooxazol-5-yl, 3,4-Dihydrooxazol-2-yl, 3,4-Dihydrooxazol-3-yl, 3,4-Dihydrooxazol-4-yl, 2-Piperidinyl, 3-Piperidinyl, 4-Piperidinyl, 1,3-Dioxan-5-yl, 2-Tetrahydropyranyl, 4-Tetrahydropyranyl, 2-Tetrahydrothienyl, 3-Hexahydro-pyridazinyl, 4-Hexahydropyridazinyl, 2-Hexahydropyrimidinyl, 4-Hexahydropyrimidinyl, 5-Hexahydropyrimidinyl-Hexahydropyrimidinyl, 2-Piperazinyl, 1,3,5-Hexahydro-triazin-2-yl und 1,2,4-Hexahydrotriazin-3-yl. Diese Definition gilt auch für Heterocyclyl als Bestandteil eines zusammengesetzen Substitutenten wie z.B. Heterocyclylalkyl etc. sofern an anderer Stelle nicht definiert;

**Abgangsgruppe:** S_{N}1 oder S_{N}2-Abgangsgruppe, beispielsweise Chlor, Brom, Iod, Alkylsulfonate (-OSO₂-Alkyl, z.B. -OSO₂CH₃, -OSO₂CF₃) oder Arylsulfonate (-OSO₂-Aryl, z.B. -OSO₂Ph,-OSO₂PhMe);

Nicht umfasst sind solche Kombinationen, die den Naturgesetzen widersprechen und die der Fachmann daher aufgrund seines Fachwissens ausgeschlossen hätte. Beispielsweise sind Ringstrukturen mit drei oder mehreren benachbarten O-Atomen ausgeschlossen.

### Erläuterung der Herstellverfahren und Zwischenprodukte

Die Piperidinpyrazole der Formel **(I)** lassen sich auf unterschiedliche Weise herstellen. Im Folgenden sind die möglichen Verfahren zunächst schematisch dargestellt. Wenn nicht anders angegeben haben die angegebenen Reste die oben angegebenen Bedeutungen.

Die erfindungsgemäßen Verfahren zur Herstellung von Verbindungen der Formel (I) werden gegebenenfalls unter Verwendung eines oder mehrerer Reaktionshilfsmittel durchgeführt.

Als Reaktionshilfsmittel kommen gegebenenfalls anorganische oder organische Basen oder Säureakzeptoren in Betracht. Hierzu gehören vorzugsweise Alkalimetall- oder Erdalkalimetall- -acetate, -amide, -carbonate, -hydrogencarbonate, -hydride, -hydroxide oder -alkanolate, wie beispielsweise Natrium-, Kalium- oder Calcium-acetat, Lithium-, Natrium-, Kalium- oder Calcium-amid, Natrium-, Kalium- oder Calcium-carbonat, Natrium-, Kalium- oder Calcium-hydrogencarbonat, Lithium-, Natrium-, Kalium- oder Calcium-hydrid, Lithium-, Natrium-, Kalium- oder Calciumhydroxid, Natrium- oder Kalium-methanolat, -ethanolat, -n- oder -i-propanolat, -n-, -i-, -s- oder - tButanolat; weiterhin auch basische organische Stickstoffverbindungen, wie beispielsweise Trimethylamin, Triethylamin, Tripropylamin, Tributylamin, Ethyl-diisopropylamin, N,N-Dimethylcyclohexylamin, Dicyclohexylamin, Ethyl-dicyclohexylamin, N,N-Dimethyl-anilin, N,N-Dimethyl-benzylamin, Pyridin, 2-Methyl-, 3-Methyl-, 4-Methyl-, 2,4-Dimethyl-, 2,6-Dimethyl-, 3,4-Dimethyl-und 3,5-Dimethyl-pyridin, 5-Ethyl-2-methyl-pyridin, 4-Dimethylamino-pyridin, N-Methyl-piperidin, 1,4-Diazabicyclo[2,2,2]-octan (DABCO), 1,5-Diazabicyclo[4,3,0]-non-5-en (DBN), oder 1,8 Diazabicyclo[5,4,0]-undec-7-en (DBU).

Die erfindungsgemäßen Verfahren werden gegebenenfalls unter Verwendung eines oder mehrerer Verdünnungsmittel durchgeführt. Als Verdünnungsmittel kommen praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl-und Dibutylether, Glykoldimethylether und Diglykoldimethylether, Tetrahydrofuran und Dioxan, Ketone wie Aceton, Methylethyl-, Methyl-isopropyl-oder Methyl-isobutyl-keton, Ester wie Essigsäuremethylester oder - ethylester, Nitrile wie z.B. Acetonitril oder Propionitril, Amide wie z.B. Dimethylformamid, Dimethylacetamid und N-Methyl-pyrrolidon, sowie Dimethylsulfoxid, Tetramethylensulfon und Hexamethylphosphorsäuretriamid und DMPU.

Die Reaktionstemperaturen können bei den erfindungsgemäßen Verfahren in einem größeren Bereich variiert werden. Im Allgemeinen arbeitet man bei Temperaturen zwischen 0 °C und 250 °C, vorzugsweise bei Temperaturen zwischen 10 °C und 185 °C.

Die Reaktionszeit variiert in Abhängigkeit vom Maßstab der Reaktion und der Reaktionstemperatur, liegt aber im Allgemeinen zwischen einigen Minuten und 48 Stunden.

Die erfindungsgemäßen Verfahren werden im Allgemeinen unter Normaldruck durchgeführt. Es ist jedoch auch möglich, unter erhöhtem oder vermindertem Druck zu arbeiten.

Zur Durchführung der erfindungsgemäßen Verfahren werden die jeweils benötigten Ausgangsstoffe im Allgemeinen in angenähert äquimolaren Mengen eingesetzt. Es ist jedoch auch möglich, eine der jeweils eingesetzten Komponenten in einem größeren Überschuss zu verwenden.

### Verfahren A

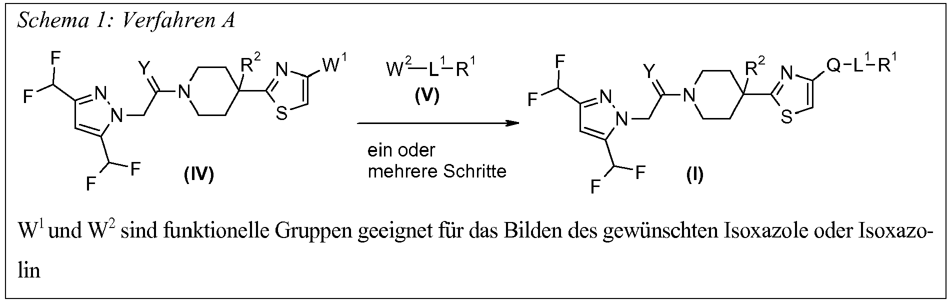

Im Allgemeinen ist es möglich Verbindungen der Formel **(I)** aus entsprechenden Verbindungen **(IV)** und **(V)** mit geeigneten funktionellen Gruppen W¹ und W² **(I)** herzustellen (s. Schema 1, *Verfahren A*)*.* Die möglichen funktionellen Gruppen für W¹ und W² sind in der Lage unter geeigneten Reaktionsbedingungen den gewünschten Isoxazol- oder Isoxazolin-Ring **Q** zu bilden (z.B. Aldehyde, Ester, Carbonsäuren, Amide, Nitrile, Alkohole, Oxime, Oximchlorid, Halide, Alkine, Alkene, Alkylhalide, Methansulfonate, Trifluormethansulfonate, Boronsäuren und Boronate). In der Literatur finden sich zahlreiche Methoden für die Darstellung von Isoxazolen oder Isoxazolinen (s. WO 2008/013622; Comprehensive Heterocyclic Chemistry Vol. 4-6, A. R. Katritzky and C. W. Rees editors, Pergamon Press, New York, 1984; Comprehensive Heterocyclic Chemistry II, Vol 2-4, A. R. Katritzky, C. W. Rees and E. F: Scriven editors, Pergamon Press, New York, 1996; The Chemistry of Heterocyclic Compounds, E. C. Taylor, editor, Wiley, New York; Rodd's Chemistry of Carbon Compounds, Vol. 2-4, Elsevier, New York; Synthesis, 1982, 6, 508-509; Tetrahedron, 2000, 56, 1057-1094; und darin zitierte Literatur).

### Verfahren B

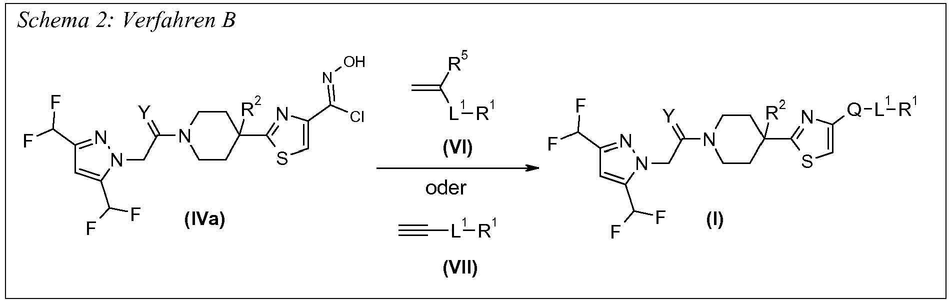

Eine bestimmte Möglichkeit, Verbindungen der Formel **(I)** aus entsprechenden Verbindungen **(IVa)** durch Reaktion mit den Verbindungen **(VI)** oder **(VII)** darzustellen, ist in Schema 2 gezeigt.

Eine Verbindung der allgemeinen Formel **(IVa)** wird durch Kondensation eines Aldehyds der Formel **(XIX)** mit Hydroxylamin und anschließender Chlorierung erhalten (s. z.B. WO 05/0040159, WO 08/013622 und Synthesis, 1987, 11, 998-1001). Aldehyd **(XIX)** und Hydroxylamin zur Reaktion werden zunächst gebracht (Schema 3, Schritt (a)). Das korrespondierende Oxim **(XVIII)** wird anschließend in Gegenwart eines geeingeneten Chlorierungsmittels chloriert. Bevorzugte Chlorierungsreagenzien sind N-Chlorsuccinimid, HClO, NaOCl, und Chlor. Nach dem Schritt (a) kann die Reaktionsmischung nach üblichen Methoden aufgearbeitet oder direkt im Schritt (b) weiter umgesetzt werden.

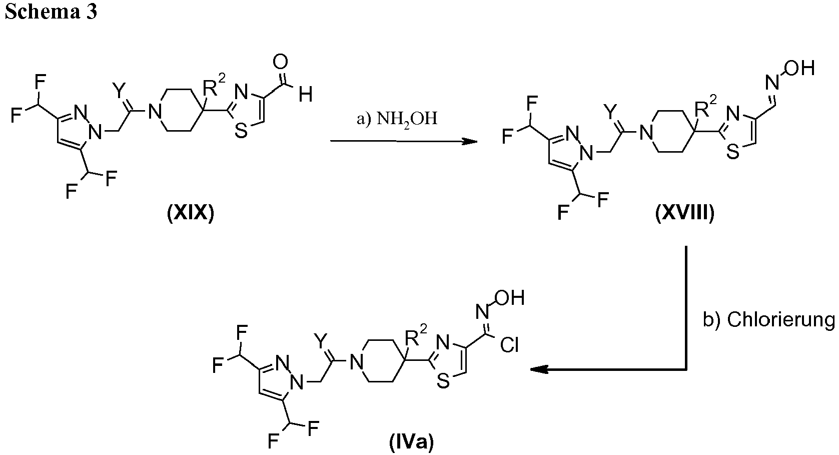

Die Alkene **(VI)** und Alkine **(VII)** sind kommerziell verfügbar oder können aus kommerziell erhältlichen Vorstufen nach literaturbeschriebenen Vorschriften hergestellt werden (z.B. aus Ketone oder Aldehyde durch eine Wittig oder Horner-Wadsworth-Emmons Olefinierung: Chem. Rev. 1989, 89, 863-927 und Julia Olefinierung: Tetrahedron Lett., 1973, 14, 4833-4836; Peterson-Olefinierung: J. Org. Chem. 1968, 33, 780; mit Bestmann-Ohira's Reagenz: Synthesis 2004, 1, 59-62).

Eine Verbindung der allgemeinen Formel **(I)** wird aus einem Alken der allgemeinen Formel **(VI)** oder aus einem Alkin der Formel **(VII)** und Verbindung **(IVa)** durch eine Zykloadditionsreaktion erhalten (s. z.B. WO 08/013622 und Synthesis, 1987, 11, 998-1001).

Das *Verfahren B* wird in Gegenwart einer geeigneten Base durchgeführt. Bevorzugte Basen sind tertiäre Amine (z.B. Triethylamin), Alkalimetall- oder Erdalkalimetallcarbonate (z.B. Natrium- oder Kaliumcarbonat), Hydrogencarbonate und Phosphate.

Das *Verfahren B* wird vorzugsweise unter Verwendung eines oder mehrer Verdünnungsmittel durchgeführt. Bei der Durchführung des *Verfahrens B* kommen vorzugsweise inerte organische Lösungsmittel infrage (wie z.B. Ethylacetat, Tetrahydrofuran, DMF). Ebenso kommt Wasser als Lösungsmittel infrage. Alternativ kann das Verfahren B in einem Überschuß des Alkens **(VI)** oder des Alkins **(VII)** durch-geführt werden.

Die Aufarbeitung erfolgt nach üblichen Methoden. Falls notwendig werden die Verbindungen durch Umkristallisation oder Chromatographie gereinigt.

### Verfahren C

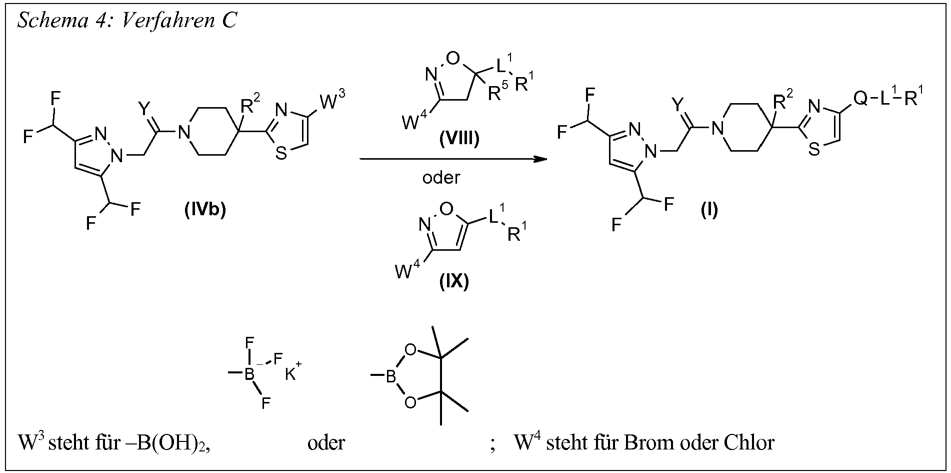

Eine bestimmte Möglichkeit, Verbindungen der Formel **(I)** aus entsprechenden Verbindungen **(IVb)** durch Reaktion mit den Verbindungen **(VIII)** oder **(IX)** durch Palladium-katalysierte Kupplungsreaktionen, wie zum Beispiel der Suzuki-Reaktion (Angew. Chem. Int. Ed. Engl., 1998, 27, 2046; A. Syn. Commun., 1981, 11, 7, 513) darzustellen, ist in Schema 4 (*Verfahren C*) gezeigt.

Das Zwischenprodukt der allgemeinen Formel **(IVb)** wird durch Borylierung oder durch Metall-Halogenaustausch mit nachfolgender Bor-Transmetallierung aus Verbindungen der Formel **(XXII)** erhalten (s. z.B. Chemical Communications, 2011, 460-462; European Journal of Organic Chemistry, 2007, 3212-3218; Tetrahedron, 2010, 8051-8059). Die Verbindungen der Formel **(XXII)** können aus kommerziell erhältlichen Vorstufen nach literaturbeschriebenen Vorschriften hergestellt werden (s. z.B. WO 2011/076699). 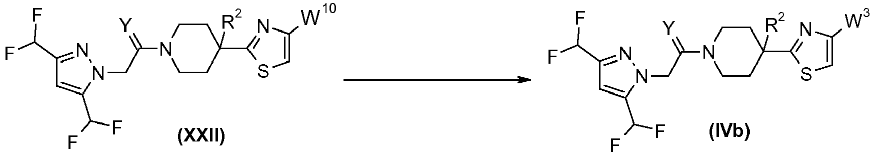 W³ steht für -B(OH)₂, 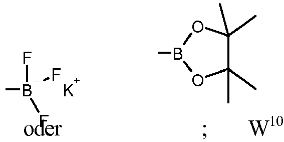 steht für Brom, Iod

Die Isoxazole oder Isoxazoline der allgemeinen Formel **(VIII)** können aus kommerziell erhältlichen Vorstufen (z.B. aus Hydroxycarbonimidoicdibromid oder (Hydroxyimino)essigsäure) durch eine Zykloadditionsreaktion mit einem Alken **(VI)** oder einem Alkin **(VII)** hergestellt werden (s. Organic Letters, 2009, 1159-1162; Liebigs Annalen der Chemie, 1989, 985-90).

Als Lösungsmittel im *Verfahren C* können alle üblichen, unter den Reaktionsbedingungen inerten Lösungsmittel verwendet werden und die Reaktion kann in Mischungen von zwei oder mehrerer dieser Lösungsmittel ausgeführt werden. Die bevorzugten Lösungsmittel sind *N,N-*Dimethylformamid, Dichlormethan, DMSO und Tetrahydrofuran.

Die Reaktion kann in Gegenwart der folgenden Additive ausgeführt werden: Phosphine, wie z.B. 2-Dicyclohexylphosphinobiphenyl, Trocknungsmitteln, z.B. 4 Å Molekularsieb, und geeigneten Basen, z.B. Triethylamin, Pyridin, Natriumcarbonat, Natriumethoxid oder Kaliumphosphat.

Es können zahlreiche kommerziell erhältliche Kupfer(II)-Katalysatoren, Palladium(0)-katalysatoren oder Palladium(II)katalysatoren in der Reaktion verwendet werden, aber bevorzugt wird in der Reaktion Kupfer(II)acetat, Tetrakistriphenylphosphinpalladium(0), 1,1-Bis(diphenylphosphino)ferrocene]dichlorpalladium(II), oder Palladium(II)acetat verwendet. Die Aufwandmenge an Katalysator beträgt mindestens 1% bis zu einem Überschuss in Abhängigkeit von der Ausgangsverbindung **(IVb).**

Die Aufarbeitung erfolgt nach üblichen Methoden. Falls notwendig werden die Verbindungen durch Umkristallisation oder Chromatographie gereinigt.

### Verfahren D

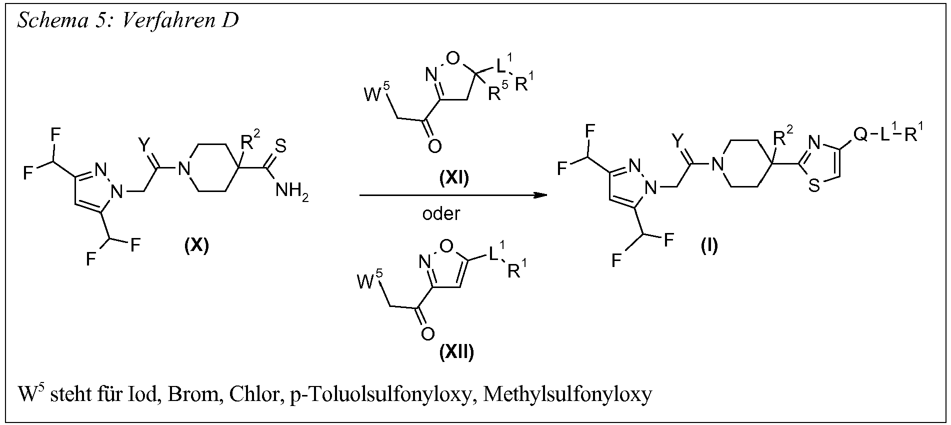

Eine bestimmte Möglichkeit zur Synthese von Verbindungen der Formel **(I)** aus Verbindungen **(X)** mit den Verbindungen **(XI)** oder **(XII)** ist in Schema 5 (*Verfahren D*) gezeigt.

Thiocarboxamide **(X)** sind nach literaturbekannten Methoden erhältlich, beispielsweise durch Schwefelung des kommerziell erhältlichen entsprechenden Carboxamids durch Verwendung von z.B. Lawesson's Reagenz (WO2008/013622, Org. Synth. Vol. 7, 1990, 372).

α-Haloketone oder entsprechende Äquivalente (z.B. p-Toluolsulfonyloxy) sind auch nach literaturbekannten Methoden erhältlich (Beispiele s. WO2008/013622), (Schema 6). 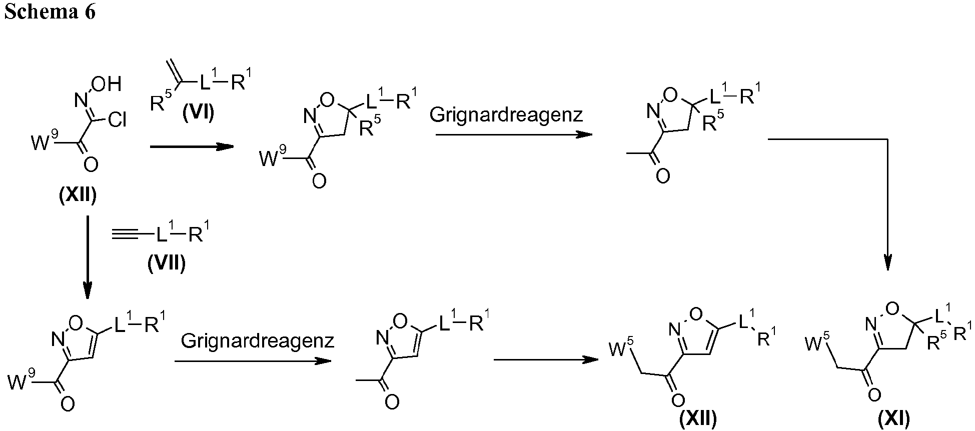 W⁹ steht für N,N-Dimethylamino, N-Methoxy-N-methylamino oder Morpholin-1-yl

Die Thiazole **(I)** werden durch eine Hantzsch-Thiazol-Synthese aus den Thiocarboxamiden **(X)** und α-Haloketonen oder entsprechende Äquivalente **(XI)** oder **(XII)** erhalten (siehe z.B. "Comprehensive Heterocyclic Chemistry", Pergamon Press, 1984; Vol 6, Seite 235-363, "Comprehensive Heterocyclic Chemistry II", Pergamon Press, 1996; Vol 3, Seite 373-474 und darin zitierte Referenzen, und WO 07/014290).

Das *Verfahren D* wird vorzugsweise unter Verwendung eines oder mehrer Verdünnungsmittel durchgeführt. Bei der Durchführung des *Verfahrens D* kommen vorzugsweise inerte organische Lösungsmittel infrage (wie z.B. sind N,N-Dimethylformamid und Ethanol).

Gegebenenfalls wird eine Hilfsbase, wie beispielsweise Triethylamin, verwendet.

Falls notwendig werden die Verbindungen durch Umkristallisation oder Chromatographie gereinigt oder können gegebenenfalls auch im nächsten Schritt ohne vorhergehende Reinigung eingesetzt werden.

### Verfahren E

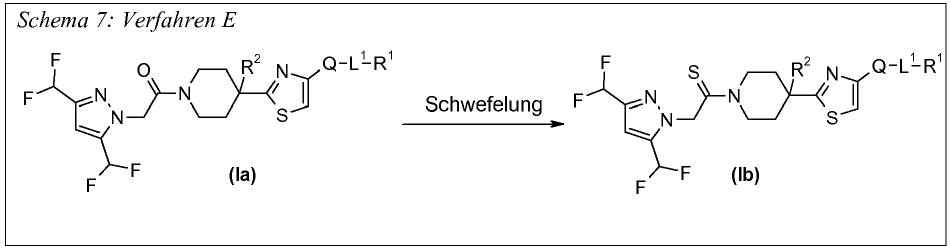

Die bei der Durchführung des erfindungsgemäßen *Verfahrens E* (Schema 7) erhaltenen Amide **(Ia)** lassen sich mittels literaturbeschriebener Methoden in die korrespondierenden Thioamide **(Ib)** überführen (z.B. Bioorganic & Medicinal Chemistry Letters, 2009, 19(2), 462-468). Hierbei werden die Verbindungen der Formel **(Ia)** typischerweise mit Phosphorpentasulfid oder 2,4-Bis(4-methoxyphenyl)-1,3-dithia-2,4-diphosphetan-2,4-disulfid (Lawesson Reagenz) umgesetzt.

Das erfindungsgemäße *Verfahren E* wird vorzugsweise unter Verwendung eines oder mehrerer Verdünnungsmittel durchgeführt. Die bevorzugten Lösungsmittel sind Toluol, Tetrahydrofuran und 1,2-Dimethoxyethan.

Nach Beendigung der Reaktion werden die Verbindungen **(Ib)** von der Reaktionsmischung durch eine der üblichen Trenntechniken getrennt. Falls notwendig werden die Verbindungen durch Umkristallisation oder Chromatographie gereinigt.

### Verfahren F

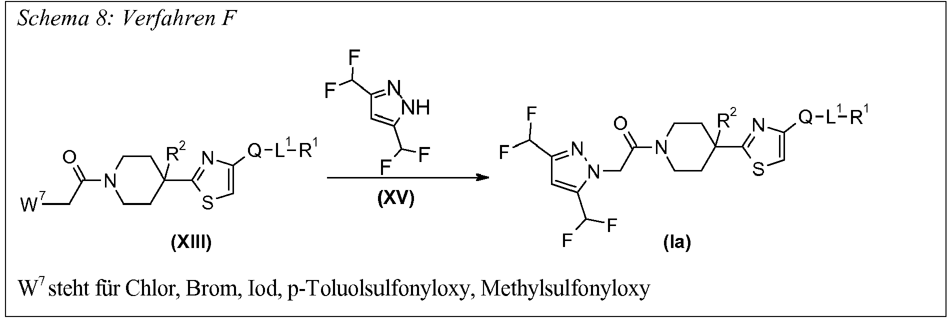

Eine Möglichkeit, Verbindungen der Formel **(Ia)** aus entsprechenden Verbindungen **(XIII)** mit den Verbindungen **(XV)** darzustellen, ist in Schema 8 (*Verfahren F*) gezeigt

Die Ausgangstoffe **(XIII),** worin W⁷ für eine Abgangsgruppe (z.B. Chlor, Brom, Iod, p-Toluolsulfonyloxy, Methylsulfonyloxy) steht, lassen sich mittels literaturbeschriebener Methoden aus Verbindungen **(XX), (XXI)** oder **(III)** (s. **Abbildung 1**) herstellen (s. z.B. Mesylierung: Organic Letters, 2003, 2539-2541; Tosylierung: JP60156601; Halogenierung: Australian Journal of Chemistry, 1983, 2095-2110;). Typischerweise werden die Verbindungen der Formel **(XIIIa,** W⁷ = Chlor) ausgehend von einem Amid der Formel **(III)** und Chloracetylchlorid hergestellt. Die Verbindungen **(XX)** in **(Abbildung 1)** werden analog zu *Verfahren F* mit Glycolsäure oder Hydroxyacetylchlorid aus **(III)** hergestellt (s. z.B. WO2007103187, WO2006117521, Bioorganic & Medicinal Chemistry Letters, 2007, 6326-6329).

### Abbildung 1

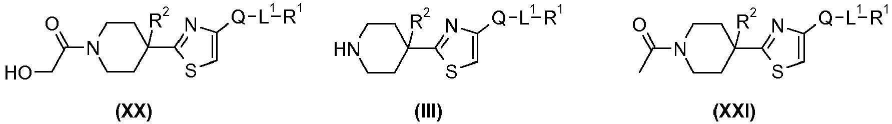

3,5-Bis(difluormethyl)-1H-pyrazol **(XV)** ist kommerziell verfügbar oder kann aus kommerziell erhältlichen Vorstufen durch literaturbeschriebene Prozesse hergestellt werden (Zhurnal Vsesoyuznogo Khimicheskogo Obshchestva im. D. I. Mendeleeva, 1981, 105-7).

In *Verfahren F* wird Wenigstens ein Äquivalent einer Base (z.B. Natriumhydrid, Kaliumcarbonat) im Verhältnis zum Startmaterial der allgemeinen Formel **(XIII)** verwendet.

Nach Beendigung der Reaktion werden die Verbindungen **(Ia)** von der Reaktionsmischung durch eine der üblichen Trenntechniken getrennt. Falls notwendig werden die Verbindungen durch Umkristallisation oder Chromatographie gereinigt.

### Verfahren G

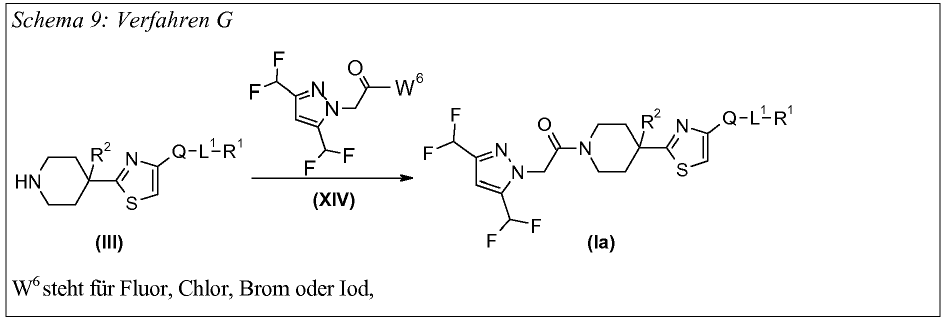

Eine Möglichkeit, Verbindungen der Formel **(Ia)** aus entsprechenden Verbindungen **(III)** mit den Verbindungen **(XIV)** darzustellen, ist in Schema 9 (*Verfahren G*) gezeigt.

Verbindungen der Formel **XXIV** sind bekannt oder durch in der Literatur beschriebene Prozesse der analogen Verbindungen darstellbar (s. z.B. WO 2008/ 091580, WO 2007/014290 und WO 2008/091594).

Eine Verbindung mit der allgemeinen Formel **(Ia)** kann analog zu in der Literatur beschriebenen Vorschriften (s. z.B. WO 2007/147336) durch eine Kupplungsreaktion einer Verbindung mit der entsprechenden allgemeinen Formel **(III)** mit einem Substrat der allgemeinen Formel **(XIV),** wobei W⁶ für Fluor, Chlor, Brom, Iod steht, gegebenenfalls in der Gegenwart eines Säurefängers / Base synthetisiert werden.

Wenigstens ein Äquivalent eines Säurefängers/einer Base (z.B. Hünig Base, Triethylamin oder kommerziell erhältliche polymere Säurefänger) wird im Verhältnis zum Startmaterial der allgemeinen Formel **(III)** verwendet. Ist das Startmaterial ein Salz, werden wenigstens zwei Äquivalente des Säurefängers benötigt.

Alternativ kann eine Verbindung der Formel **(Ia),** auch aus der entsprechenden Verbindung der Formel **(III)** mit einem Substrat der Formel **(XIV),** wobei W⁶ für Hydroxy steht, in Gegenwart eines Kupplungsreagenzes analog zu in der Literatur beschriebenen Vorschriften synthetisiert werden (z.B. Tetrahedron 2005, 61, 10827-10852, und darin zitierte Referenzen).

Geeignete Kupplungsreagenzien sind beispielsweise Peptidkupplungsreagenzien zum Beispiel, N-(3-Dimethylaminopropyl)-N'-ethyl-carbodiimid gemischt mit 4-Dimethylamino-pyridin, N-(3-Dimethylaminopropyl)-N'-ethyl-carbodiimid gemischt mit 1-Hydroxy-benzotriazol, Bromtripyrrolidinophosphonium-hexafluorophosphat, O-(7-Azabenzotriazol-1-yl)-N,N,N',N'-tetramethyluroniumhexafluorophosphat, etc.

Nach Beendigung der Reaktion, werden die Verbindungen **(Ia)** von der Reaktionsmischung durch eine der üblichen Trenntechniken getrennt. Falls notwendig werden die Verbindungen durch Umkristallisation oder Chromatographie gereinigt.

### Verfahren H

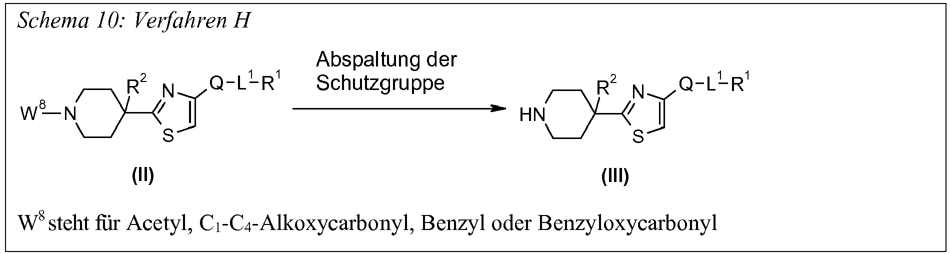

Eine Möglichkeit Verbindungen der Formel **(III)** aus entsprechenden Verbindungen **(II)** darzustellen ist in Schema 10 (*Verfahren H*) gezeigt.

Eine Verbindung der Formel **(II)** wird in eine Verbindung der Formel **(III)** durch geeignete Methoden zur Entfernung von Schutzgruppen, die in der Literatur beschrieben sind ("Protective Groups in Organic Synthesis"; Theodora W. Greene, Peter G. M. Wuts; Wiley-Interscience; Third Edition; 1999; 494-653), überführt.

*tert*-Butoxycarbonyl- und Benzyloxycarbonylschutzgruppen können im sauren Medium entfernt werden (z.B mit Salzsäure oder der Trifluoressigsäure). Acetylschutzgruppen können unter basischen Bedingungen (z.B. mit Kaliumcarbonat oder Cäsiumcarbonat) entfernt werden. Benzylische Schutzgruppen können hydrogenolytisch mit Wasserstoff in Gegenwart eines Katalysators (z.B. Palladium auf Aktivkohle) entfernt werden.

Nach Beendigung der Reaktion werden die Verbindungen **(III)** von der Reaktionsmischung durch eine der üblichen Trenntechniken getrennt. Falls notwendig werden die Verbindungen durch Umkristallisation oder Chromatographie gereinigt oder können, wenn gewünscht, auch im nächsten Schritt ohne vorhergehende Reinigung eingesetzt werden. Es ist außerdem möglich, die Verbindung der allgemeinen Formel **(III)** als Salz zu isolieren, z.B. als Salz der Salzsäure oder der Trifluoressigsäure.

### Verfahren I

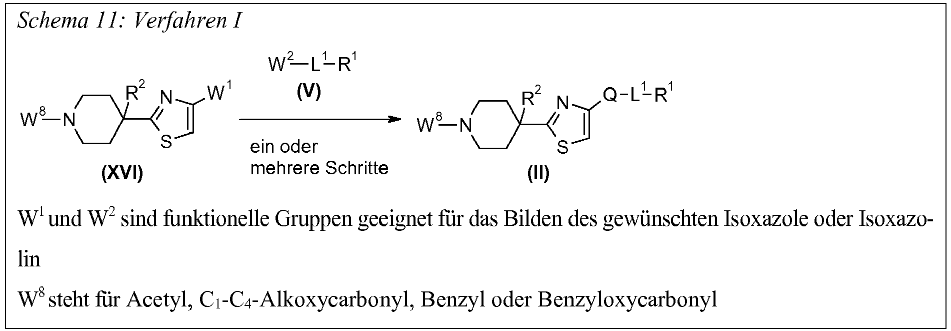

Eine Möglichkeit, das Zwischenprodukt der Formel **(II)** aus entsprechenden Verbindungen **(XVI)** herzustellen, ist in Schema 11 (*Verfahren I*) gezeigt. Die möglichen funktionellen Gruppen für W¹ und W² sind in der Lage unter geeigneten Reaktionsbedingungen den gewünschten Isoxazol- oder Isoxazolin-Ring **Q** zu bilden (z.B. Aldehyde, Ester, Carbonsäuren, Amide, Nitrile, Alkohole, Oxime, Oximchlorid, Halide, Alkine, Alkene, Alkylhalide, Methansulfonate, Trifluormethansulfonate, Boronsäuren und Boronate). *Verfahren I* wird analog *Verfahren A* (Schema 1) durchgeführt.

### Verfahren J

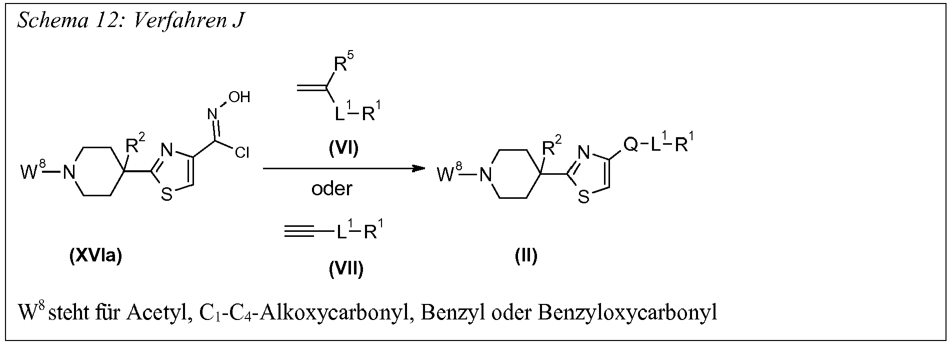

Eine bestimmte Möglichkeit, das Zwischenprodukt der Formel **(II)** aus entsprechenden Verbindungen **(XVIa)** durch Reaktion mit den Verbindungen **(VI)** oder **(VII)** darzustellen, ist in Schema 12 (*Verfahren J*) gezeigt. *Verfahren J* wird analog *Verfahren B* (Schema 2) durchgeführt.

### Verfahren K

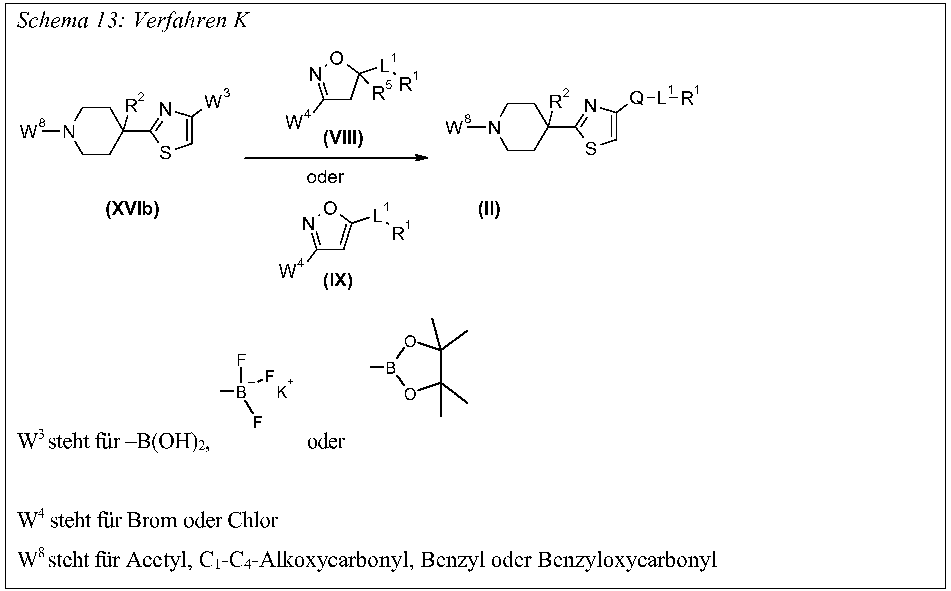

Eine andere bestimmte Möglichkeit, das Zwischenprodukt der Formel **(II)** aus entsprechenden Verbindungen **(XVIb)** durch Reaktion mit den Verbindungen **(VIII)** oder **(IX)** darzustellen, ist in Schema 13 (*Verfahren K*) gezeigt. *Verfahren J* wird analog *Verfahren* C (Schema 4) durchgeführt.

### Verfahren L

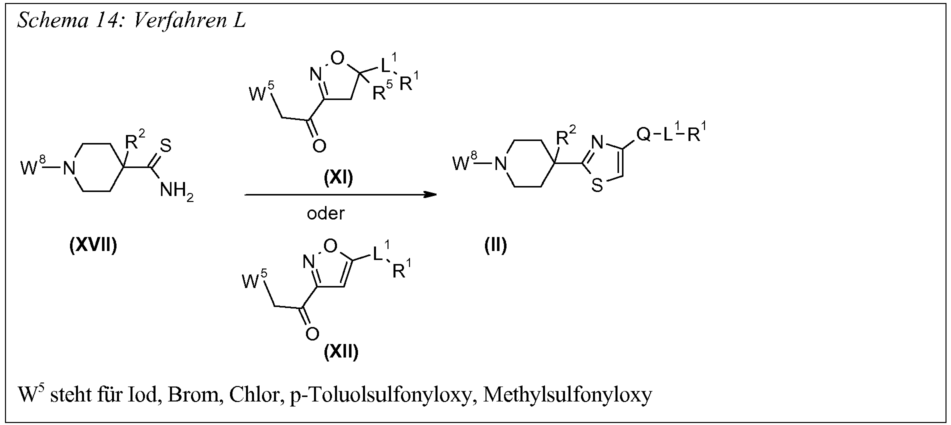

Eine bestimmte Möglichkeit, das Zwischenprodukt der Formel **(II)** aus entsprechenden Verbindungen **(XVII)** durch Reaktion mit den Verbindungen **(XI)** oder **(XII)** darzustellen, ist in Schema 14 (*Verfahren L*) gezeigt. *Verfahren L* wird analog *Verfahren D* (Schema 5) durchgeführt.

Es wird erkannt, daß einige Reagenzien und Reaktionsbegindungen, beschrieben vorstehend zum Herstellen von Verbindungen der Formel **(I),** nicht mit bestimmten Funktionalitäten, vorhanden in den Zwischenverbindungen, kompatibel sein können. In diesen Fällen hilft die Einbringung von Schutz-/Entschützungssequenzen oder gegenseitiger Umwandlungen funktioneller Gruppen in die Synthese, um die gewünschten Produkte zu erhalten. Die Verwendung und Auswahl der schützenden Gruppen ist für den Fachmann in der chemischen Synthese offensichtlich (s. z.B. *"*Protective Groups in Organic Synthesis"; Third Edition; 494-653, und dort zitierte Literatur). Der Fachmann wird erkennen, dass es in einigen Fällen nach der Einführung eines gegebenen Reagenzes, wie es in einem individuellen Schema dargestellt ist, notwendig sein kann, zusätzliche routinemäßige Syntheseschritte durchzuführen, die nicht im einzelnen beschrieben sind, um die Synthese von Verbindungen der Formel **(I)** zu vervollständigen. Der Fachmann wird ebenfalls erkennen, daß es notwendig sein kann, eine Kombination der Schritte, veranschaulicht in den vorstehenden Schemata, in einer anderen Reihenfolge als der durch die speziell dargestellte implizierte Sequenz durchzuführen, um die Verbindungen der Formel **(I)** herzustellen.

Die vorliegende Erfindung betrifft weiterhin ein Mittel, zum Bekämpfen von unerwünschten Mikroorganismen, umfassend die erfindungsgemäßen Wirkstoffe. Vorzugsweise handelt es sich um fungizide Mittel, welche landwirtschaftlich verwendbare Hilfsmittel, Solventien, Trägerstoffe, oberflächenaktive Stoffe oder Streckmittel enthalten.

Außerdem betrifft die Erfindung ein Verfahren zum Bekämpfen unerwünschter Mikroorganismen, dadurch gekennzeichnet, dass man die erfindungsgemäßen Wirkstoffe auf die phytopathogenen Pilze und/oder deren Lebensraum ausbringt.

Erfindungsgemäß bedeutet Trägerstoff eine natürliche oder synthetische, organische oder anorganische Substanz, mit welchen die Wirkstoffe zur besseren Anwendbarkeit, v.a. zum Aufbringen auf Pflanzen oder Pflanzenteile oder Saatgut, gemischt oder verbunden sind. Der Trägerstoff, welcher fest oder flüssig sein kann, ist im Allgemeinen inert und sollte in der Landwirtschaft verwendbar sein.

Als feste oder flüssige Trägerstoffe kommen infrage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und natürliche oder synthetische Silikate, Harze, Wachse, feste Düngemittel, Wasser, Alkohole, besonders Butanol, organische Solventien, Mineral- und Pflanzenöle sowie Derivate hiervon. Mischungen solcher Trägerstoffe können ebenfalls verwendet werden. Als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstängel.

Als verflüssigte gasförmige Streckmittel oder Trägerstoffe kommen solche Flüssigkeiten infrage, welche bei normaler Temperatur und unter Normaldruck gasförmig sind, z.B. Aerosol-Treibgase, wie Halogenkohlenwasserstoffe, sowie Butan, Propan, Stickstoff und Kohlendioxid.

Es können in den Formulierungen Haftmittel wie Carboxymethylcellulose, natürliche und synthetische pulverige, körnige oder latexförmige Polymere verwendet werden, wie Gummiarabikum, Polyvinylalkohol, Polyvinylacetat, sowie natürliche Phospholipide, wie Kephaline und Lecithine, und synthetische Phospholipide. Weitere Additive können mineralische und vegetabile Öle sein.

Im Falle der Benutzung von Wasser als Streckmittel können z.B. auch organische Lösungsmittel als Hilfslösungsmittel verwendet werden. Als flüssige Lösungsmittel kommen im Wesentlichen infrage: Aromaten, wie Xylol, Toluol oder Alkylnaphthaline, chlorierte Aromaten oder chlorierte aliphatische Kohlenwasserstoffe, wie Chlorbenzole, Chlorethylene oder Dichlormethan, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone, wie Aceton, Methylethylketon, Methylisobutylketon oder Cyclohexanon, stark polare Lösungsmittel wie Dimethylformamid und Dimethylsulfoxid, sowie Wasser.

Die erfindungsgemäßen Mittel können zusätzlich weitere Bestandteile enthalten, wie z.B. oberflächenaktive Stoffe. Als oberflächenaktive Stoffe kommen Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe infrage. Beispiele hierfür sind Salze von Polyacrylsäure, Salze von Lignosulphonsäure, Salze von Phenolsulphonsäure oder Naphthalinsulphonsäure, Polykondensate von Ethylenoxid mit Fettalkoholen oder mit Fettsäuren oder mit Fettaminen, substituierten Phenolen (vorzugsweise Alkylphenole oder Arylphenole), Salze von Sulphobernsteinsäureestern, Taurinderivate (vorzugsweise Alkyltaurate), Phosphorsäureester von polyethoxylierten Alkoholen oder Phenole, Fettsäureester von Polyolen, und Derivate der Verbindungen enthaltend Sulphate, Sulphonate und Phosphate, z.B. Alkylarylpolyglycolether, Alkylsulfonate, Alkylsulfate, Arylsulfonate, Eiweißhydrolysate, Lignin-Sulfitablaugen und Methylcellulose. Die Anwesenheit einer oberflächenaktiven Substanz ist notwendig, wenn einer der Wirkstoff und/oder einer der inerten Trägerstoffe nicht in Wasser löslich ist und wenn die Anwendung in Wasser erfolgt. Der Anteil an oberflächenaktiven Stoffen liegt zwischen 5 und 40 Gewichtsprozent des erfindungsgemäßen Mittels.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Gegebenenfalls können auch andere zusätzliche Komponenten enthalten sein, z.B. schützende Kolloide, Bindemittel, Klebstoffe, Verdicker, thixotrope Stoffe, Penetrationsförderer, Stabilisatoren, Sequestiermittel, Komplexbildner. Im Allgemeinen können die Wirkstoffe mit jedem festen oder flüssigen Additiv, welches für Formulierungszwecke gewöhnlich verwendet wird, kombiniert werden.

Im Allgemeinen enthalten die erfindungsgemäßen Mittel und Formulierungen zwischen 0,05 und 99 Gew.-%, 0,01 und 98 Gew.-%, vorzugsweise zwischen 0,1 und 95 Gew.-%, besonders bevorzugt zwischen 0,5 und 90 % Wirkstoff, ganz besonders bevorzugt zwischen 10 und 70 Gewichtsprozent.

Die erfindungsgemäßen Wirkstoffe bzw. Mittel können als solche oder in Abhängigkeit von ihren jeweiligen physikalischen und/oder chemischen Eigenschaften in Form ihrer Formulierungen oder den daraus bereiteten Anwendungsformen, wie Aerosole, Kapselsuspensionen, Kaltnebelkonzentrate, Heißnebelkonzentrate, verkapselte Granulate, Feingranulate, fließfähige Konzentrate für die Behandlung von Saatgut, gebrauchsfertige Lösungen, verstäubbare Pulver, emulgierbare Konzentrate, Öl-in-Wasser-Emulsionen, Wasser-in-Öl-Emulsionen, Makrogranulate, Mikrogranulate, Öl dispergierbare Pulver, Öl mischbare fließfähige Konzentrate, Öl mischbare Flüssigkeiten, Schäume, Pasten, Pestizid ummanteltes Saatgut, Suspensionskonzentrate, Suspensions-Emulsions-Konzentrate, lösliche Konzentrate, Suspensionen, Spritzpulver, lösliche Pulver, Stäubemittel und Granulate, wasserlösliche Granulate oder Tabletten, wasserlösliche Pulver für Saatgutbehandlung, benetzbare Pulver, Wirkstoff-imprägnierte Natur- und synthetische Stoffe sowie Feinstverkapselungen in polymeren Stoffen und in Hüllmassen für Saatgut, sowie ULV-Kalt- und Warmnebel-Formulierungen eingesetzt werden.

Die genannten Formulierungen können in an sich bekannter Weise hergestellt werden, z.B. durch Vermischen der Wirkstoffe mit mindestens einem üblichen Streckmittel, Lösungs- bzw. Verdünnungsmittel, Emulgator, Dispergier- und/oder Binde- oder Fixiermittels, Netzmittel, Wasser-Repellent, gegebenenfalls Sikkative und UV-Stabilisatoren und gegebenenfalls Farbstoffen und Pigmenten, Entschäumer, Konservierungsmittel, sekundäre Verdickungsmittel, Kleber, Gibberelline sowie weiteren Verarbeitungshilfsmitteln.

Die erfindungsgemäßen Mittel umfassen nicht nur Formulierungen, welche bereits anwendungsfertig sind und mit einer geeigneten Apparatur auf die Pflanze oder das Saatgut ausgebracht werden können, sondern auch kommerzielle Konzentrate, welche vor Gebrauch mit Wasser verdünnt werden müssen.

Die erfindungsgemäßen Wirkstoffe können als solche oder in ihren (handelsüblichen) Formulierungen sowie in den aus diesen Formulierungen bereiteten Anwendungsformen in Mischung mit anderen (bekannten) Wirkstoffen, wie Insektiziden, Lockstoffen, Sterilantien, Bakteriziden, Akariziden, Nematiziden, Fungiziden, Wachstumsregulatoren, Herbiziden, Düngemitteln, Safener bzw. Semiochemicals vorliegen.

Die erfindungsgemäße Behandlung der Pflanzen und Pflanzenteile mit den Wirkstoffen bzw. Mitteln erfolgt direkt oder durch Einwirkung auf deren Umgebung, Lebensraum oder Lagerraum nach den üblichen Behandlungsmethoden, z.B. durch Tauchen, (Ver-)Spritzen, (Ver-)Sprühen, Berieseln, Verdampfen, Zerstäuben, Vernebeln, (Ver-)Streuen, Verschäumen, Bestreichen, Verstreichen, Gießen (drenchen), Tröpfchenbewässerung und bei Vermehrungsmaterial, insbesondere bei Samen, weiterhin durch Trockenbeizen, Nassbeizen, Schlämmbeizen, Inkrustieren, ein- oder mehrschichtiges Umhüllen usw. Es ist ferner möglich, die Wirkstoffe nach dem Ultra-Low-Volume-Verfahren auszubringen oder die Wirkstoffzubereitung oder den Wirkstoff selbst in den Boden zu injizieren.

Die Erfindung umfasst weiterhin ein Verfahren zur Behandlung von Saatgut.

Die Erfindung betrifft weiterhin Saatgut, welches gemäß einem der im vorherigen Absatz beschriebenen Verfahren behandelt wurde. Die erfindungsgemäßen Saatgüter finden Anwendung in Verfahren zum Schutz von Saatgut vor unerwünschten Mikroorganismen. Bei diesen wird ein mit wenigstens einem erfindungsgemäßen Wirkstoff behandeltes Saatgut verwendet.

Die erfindungsgemäßen Wirkstoffe bzw. Mittel sind auch geeignet für die Behandlung von Saatgut. Ein großer Teil des durch Schadorganismen hervorgerufenen Schadens an Kulturpflanzen wird durch den Befall des Saatguts während der Lagerung oder nach der Aussaat sowie während und nach der Keimung der Pflanze ausgelöst. Diese Phase ist besonders kritisch, weil die Wurzeln und Schösslinge der wachsenden Pflanze besonders empfindlich sind und auch nur eine kleine Schädigung zum Tod der Pflanze führen kann. Es besteht daher ein großes Interesse daran, das Saatgut und die keimende Pflanze durch Einsatz geeigneter Mittel zu schützen.

Die Bekämpfung von phytopathogenen Pilzen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufrieden stellend gelöst werden können. So ist es erstrebenswert, Verfahren zum Schutz des Saatguts und der keimenden Pflanze zu entwickeln, die das zusätzliche Ausbringen von Pflanzenschutzmitteln nach der Saat oder nach dem Auflaufen der Pflanzen überflüssig machen oder zumindest deutlich verringern. Es ist weiterhin erstrebenswert, die Menge des eingesetzten Wirkstoffs dahingehend zu optimieren, dass das Saatgut und die keimende Pflanze vor dem Befall durch phytopathogene Pilze bestmöglich geschützt werden, ohne jedoch die Pflanze selbst durch den eingesetzten Wirkstoff zu schädigen. Insbesondere sollten Verfahren zur Behandlung von Saatgut auch die intrinsischen fungiziden Eigenschaften transgener Pflanzen einbeziehen, um einen optimalen Schutz des Saatguts und der keimenden Pflanze bei einem minimalen Aufwand an Pflanzenschutzmitteln zu erreichen.

Die vorliegende Erfindung bezieht sich daher auch auf ein Verfahren zum Schutz von Saatgut und keimenden Pflanzen vor dem Befall von phytopathogenen Pilzen, indem das Saatgut mit einem erfindungsgemäßen Mittel behandelt wird. Die Erfindung bezieht sich ebenfalls auf die Verwendung der erfindungsgemäßen Mittel zur Behandlung von Saatgut zum Schutz des Saatguts und der keimenden Pflanze vor phytopathogenen Pilzen. Weiterhin bezieht sich die Erfindung auf Saatgut, welches zum Schutz vor phytopathogenen Pilzen mit einem erfindungsgemäßen Mittel behandelt wurde.

Die Bekämpfung von phytopathogenen Pilzen, die Pflanzen nach dem Auflaufen schädigen, erfolgt in erster Linie durch die Behandlung des Bodens und der oberirdischen Pflanzenteile mit Pflanzenschutzmitteln. Aufgrund der Bedenken hinsichtlich eines möglichen Einflusses der Pflanzenschutzmittel auf die Umwelt und die Gesundheit von Menschen und Tieren gibt es Anstrengungen, die Menge der ausgebrachten Wirkstoffe zu vermindern.

Einer der Vorteile der vorliegenden Erfindung ist es, dass aufgrund der besonderen systemischen Eigenschaften der erfindungsgemäßen Wirkstoffe bzw. Mittel die Behandlung des Saatguts mit diesen Wirkstoffen bzw. Mitteln nicht nur das Saatgut selbst, sondern auch die daraus hervorgehenden Pflanzen nach dem Auflaufen vor phytopathogenen Pilzen schützt. Auf diese Weise kann die unmittelbare Behandlung der Kultur zum Zeitpunkt der Aussaat oder kurz danach entfallen.

Ebenso ist es als vorteilhaft anzusehen, dass die erfindungsgemäßen Wirkstoffe bzw. Mittel insbesondere auch bei transgenem Saatgut eingesetzt werden können, wobei die aus diesem Saatgut wachsende Pflanze in der Lage ist, ein Protein zu exprimieren, welches gegen Schädlinge wirkt. Durch die Behandlung solchen Saatguts mit den erfindungsgemäßen Wirkstoffen bzw. Mitteln können bereits durch die Expression des beispielsweise insektiziden Proteins bestimmte Schädlinge bekämpft werden. Überraschenderweise kann dabei ein weiterer synergistischer Effekt beobachtet werden, welcher zusätzlich die Effektivität zum Schutz gegen den Schädlingsbefall vergrößert.

Die erfindungsgemäßen Mittel eignen sich zum Schutz von Saatgut jeglicher Pflanzensorte, die in der Landwirtschaft, im Gewächshaus, in Forsten oder im Garten- und Weinbau eingesetzt wird. Insbesondere handelt es sich dabei um Saatgut von Getreide (wie Weizen, Gerste, Roggen, Triticale, Hirse und Hafer), Mais, Baumwolle, Soja, Reis, Kartoffeln, Sonnenblume, Bohne, Kaffee, Rübe (z.B. Zuckerrübe und Futterrübe), Erdnuss, Raps, Mohn, Olive, Kokosnuss, Kakao, Zuckerrohr, Tabak, Gemüse (wie Tomate, Gurke, Zwiebeln und Salat), Rasen und Zierpflanzen (siehe auch unten). Besondere Bedeutung kommt der Behandlung des Saatguts von Getreide (wie Weizen, Gerste, Roggen, Triticale und Hafer), Mais und Reis zu.

Wie auch weiter unten beschrieben, ist die Behandlung von transgenem Saatgut mit den erfindungsgemäßen Wirkstoffen bzw. Mitteln von besonderer Bedeutung. Dies betrifft das Saatgut von Pflanzen, die wenigstens ein heterologes Gen enthalten, das die Expression eines Polypeptids oder Proteins mit insektiziden Eigenschaften ermöglicht. Das heterologe Gen in transgenem Saatgut kann z.B. aus Mikroorganismen der Arten Bacillus, Rhizobium, Pseudomonas, Serratia, Trichoderma, Clavibacter, Glomus oder Gliocladium stammen. Bevorzugt stammt dieses heterologe Gen aus Bacillus sp., wobei das Genprodukt eine Wirkung gegen den Maiszünsler (European corn borer) und/oder Western Corn Rootworm besitzt. Besonders bevorzugt stammt das heterologe Gen aus Bacillus thuringiensis.

Im Rahmen der vorliegenden Erfindung wird das erfindungsgemäße Mittel alleine oder in einer geeigneten Formulierung auf das Saatgut aufgebracht. Vorzugsweise wird das Saatgut in einem Zustand behandelt, in dem so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde. So kann zum Beispiel Saatgut verwendet werden, das geerntet, gereinigt und bis zu einem Feuchtigkeitsgehalt von unter 15 Gew.-% getrocknet wurde. Alternativ kann auch Saatgut verwendet werden, das nach dem Trocknen z.B. mit Wasser behandelt und dann erneut getrocknet wurde.

Im Allgemeinen muss bei der Behandlung des Saatguts darauf geachtet werden, dass die Menge des auf das Saatgut aufgebrachten erfindungsgemäßen Mittels und/oder weiterer Zusatzstoffe so gewählt wird, dass die Keimung des Saatguts nicht beeinträchtigt bzw. die daraus hervorgehende Pflanze nicht geschädigt wird. Dies ist v.a. bei Wirkstoffen zu beachten, die in bestimmten Aufwandmengen phytotoxische Effekte zeigen können.

Die erfindungsgemäßen Mittel können ummittelbar aufgebracht werden, also ohne weitere Komponenten zu enthalten und ohne verdünnt worden zu sein. In der Regel ist es vorzuziehen, die Mittel in Form einer geeigneten Formulierung auf das Saatgut aufzubringen. Geeignete Formulierungen und Verfahren für die Saatgutbehandlung sind dem Fachmann bekannt und werden z.B. in den folgenden Dokumenten beschrieben: US 4,272,417 A, US 4,245,432 A, US 4,808,430 A, US 5,876,739 A, US 2003/0176428 A1, WO 2002/080675 A1, WO 2002/028186 A2.

Die erfindungsgemäß verwendbaren Wirkstoffe können in die üblichen Beizmittel-Formulierungen überführt werden, wie Lösungen, Emulsionen, Suspensionen, Pulver, Schäume, Slurries oder andere Hüllmassen für Saatgut, sowie ULV-Formulierungen.

Diese Formulierungen werden in bekannter Weise hergestellt, indem man die Wirkstoffe mit üblichen Zusatzstoffen vermischt, wie zum Beispiel übliche Streckmittel sowie Lösungs- oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser.

Als Farbstoffe, die in den erfindungsgemäß verwendbaren Beizmittel-Formulierungen enthalten sein können, kommen alle für derartige Zwecke üblichen Farbstoffe in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C.I. Pigment Red 112 und C.I. Solvent Red 1 bekannten Farbstoffe.

Als Netzmittel, die in den erfindungsgemäß verwendbaren Beizmittel-Formulierungen enthalten sein können, kommen alle zur Formulierung von agrochemischen Wirkstoffen üblichen, die Benetzung fördernden Stoffe in Frage. Vorzugsweise verwendbar sind Alkylnaphthalin-Sulfonate, wie Diisopropyl- oder Diisobutyl-naphthalin-Sulfonate.

Als Dispergiermittel und/oder Emulgatoren, die in den erfindungsgemäß verwendbaren Beizmittel-Formulierungen enthalten sein können, kommen alle zur Formulierung von agrochemischen Wirkstoffen üblichen nichtionischen, anionischen und kationischen Dispergiermittel in Betracht. Vorzugsweise verwendbar sind nichtionische oder anionische Dispergiermittel oder Gemische von nichtionischen oder anionischen Dispergiermitteln. Als geeignete nichtionische Dispergiermittel sind insbesondere Ethylenoxid-Propylenoxid Blockpolymere, Alkylphenolpolyglykolether sowie Tristryrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate.

Als Entschäumer können in den erfindungsgemäß verwendbaren Beizmittel-Formulierungen alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können in den erfindungsgemäß verwendbaren Beizmittel-Formulierungen alle für derartige Zwecke in agrochemischen Mitteln einsetzbaren Stoffe vorhanden sein. Beispielhaft genannt seien Dichlorophen und Benzylalkoholhemiformal.

Als sekundäre Verdickungsmittel, die in den erfindungsgemäß verwendbaren Beizmittel-Formulierungen enthalten sein können, kommen alle für derartige Zwecke in agrochemischen Mitteln einsetzbaren Stoffe in Frage. Vorzugsweise in Betracht kommen Cellulosederivate, Acrylsäurederivate, Xanthan, modifizierte Tone und hochdisperse Kieselsäure.

Als Kleber, die in den erfindungsgemäß verwendbaren Beizmittel-Formulierungen enthalten sein können, kommen alle üblichen in Beizmitteln einsetzbaren Bindemittel in Frage. Vorzugsweise genannt seien Polyvinylpyrrolidon, Polyvinylacetat, Polyvinylalkohol und Tylose.

Als Gibberelline, die in den erfindungsgemäß verwendbaren Beizmittel-Formulierungen enthalten sein können, kommen vorzugsweise die Gibberelline A1, A3 (= Gibberellinsäure), A4 und A7 infrage, besonders bevorzugt verwendet man die Gibberellinsäure. Die Gibberelline sind bekannt (vgl. R. Wegler "Chemie der Pflanzenschutz- und Schädlingsbekämpfungsmittel", Bd. 2, Springer Verlag, 1970, S. 401-412).

Die erfindungsgemäß verwendbaren Beizmittel-Formulierungen können entweder direkt oder nach vorherigem Verdünnen mit Wasser zur Behandlung von Saatgut der verschiedensten Art, auch von Saatgut transgener Pflanzen, eingesetzt werden. Dabei können im Zusammenwirken mit den durch Expression gebildeten Substanzen auch zusätzliche synergistische Effekte auftreten.

Zur Behandlung von Saatgut mit den erfindungsgemäß verwendbaren Beizmittel-Formulierungen oder den daraus durch Zugabe von Wasser hergestellten Zubereitungen kommen alle üblicherweise für die Beizung einsetzbaren Mischgeräte in Betracht. Im einzelnen geht man bei der Beizung so vor, dass man das Saatgut in einen Mischer gibt, die jeweils gewünschte Menge an Beizmittel-Formulierungen entweder als solche oder nach vorherigem Verdünnen mit Wasser hinzufügt und bis zur gleichmäßigen Verteilung der Formulierung auf dem Saatgut mischt. Gegebenenfalls schließt sich ein Trocknungsvorgang an.

Die erfindungsgemäßen Wirkstoffe bzw. Mittel weisen eine starke mikrobizide Wirkung auf und können zur Bekämpfung von unerwünschten Mikroorganismen, wie Pilzen und Bakterien, im Pflanzenschutz und im Materialschutz eingesetzt werden.

Fungizide lassen sich Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

Die erfindungsgemäßen fungiziden Mittel können zur Bekämpfung von phytopathogenen Pilzen kurativ oder protektiv eingesetzt werden. Die Erfindung betrifft daher auch kurative und protektive Verfahren zum Bekämpfen von phytopathogenen Pilzen durch die Verwendung der erfindungsgemäßen Wirkstoffe oder Mittel, welche auf das Saatgut, die Pflanze oder Pflanzenteile, die Früchten oder den Boden, in welcher die Pflanzen wachsen, ausgebracht wird.

Die erfindungsgemäßen Mittel zum Bekämpfen von phytopathogenen Pilzen im Pflanzenschutz umfassen eine wirksame, aber nicht-phytotoxische Menge der erfindungsgemäßen Wirkstoffe. "Wirksame, aber nicht-phytotoxische Menge" bedeutet eine Menge des erfindungsgemäßen Mittels, die ausreichend ist, um die Pilzerkrankung der Pflanze ausreichend zu kontrollieren oder ganz abzutöten und die gleichzeitig keine nennenswerten Symptome von Phytotoxizität mit sich bringt. Diese Aufwandmenge kann im Allgemeinen in einem größeren Bereich variieren. Sie hängt von mehreren Faktoren ab, z.B. vom zu bekämpfenden Pilz, der Pflanze, den klimatischen Verhältnissen und den Inhaltsstoffen der erfindungsgemäßen Mittel.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes. Sie können vorzugsweise als Pflanzenschutzmittel eingesetzt werden. Sie sind gegen normal sensible und resistente Arten sowie gegen alle oder einzelne Entwicklungsstadien wirksam.

Als Pflanzen, welche erfindungsgemäß behandelt werden können, seien folgende Hauptanbaupflanzen erwähnt: Mais, Sojabohne, Baumwolle, *Brassica* Ölsaaten wie *Brassica napus* (z.B. Canola), *Brassica rapa, B. juncea* (z.B. (Acker-)Senf) und *Brassica carinata,* Reis, Weizen Zuckerrübe, Zurckerrohr, Hafer, Roggen, Gerste, Hirse, Triticale, Flachs, Wein und verschiedene Früchte und Gemüse von verschiedenen botanischen Taxa wie z.B. *Rosaceae sp.* (beispielsweise Kernfrüchte wie Apfel und Birne, aber auch Steinfrüchte wie Aprikosen, Kirschen, Mandeln und Pfirsiche und Beerenfrüchte wie Erdbeeren), *Ribesioidae sp., Juglandaceae sp., Betulaceae sp., Anacardiaceae sp., Fagaceae sp., Moraceae sp., Oleaceae sp., Actinidaceae sp., Lauraceae sp., Musaceae sp.* (beispielsweise Bananenbäume und - plantagen), *Rubiaceae sp.* (beispielsweise Kaffee), *Theaceae sp., Stereuliceae sp., Rutaceae sp.* (beispielsweise Zitronen, Organen und Grapefruit); *Solanaceae sp.* (beispielsweise Tomaten, Kartoffeln, Pfeffer, Auberginen), *Liliaceae sp., Compositae sp.* (beispielsweise Salat, Artischocke and Chicoree - einschließlich Wurzelchicoree, Endivie oder gemeinen Chicoree), *Umbelliferae sp.* (beispielsweise Karrotte, Petersilie, Stangensellerie und Knollensellerie), *Cucurbitaceae sp.* (beispielsweise Gurke - einschließlich Gewürzgurke, Kürbis, Wassermelone, Flaschenkürbis und Melonen), *Alliaceae* sp. (beispielsweise Lauch und Zwiebel), *Cruciferae sp.* (beispielsweise Weißkohl, Rotkohl, Brokkoli, Blumenkohl, Rosenkohl, Pak Choi, Kohlrabi, Radieschen, Meerrettich, Kresse, Senf, Raps und Chinakohl), *Leguminosae sp.* (beispielsweise Erdnüsse, Erbsen, und Bohnen - wie z.B. Stangenbohne und Ackerbohne), *Chenopodiaceae sp.* (beispielsweise Mangold, Futterrübe, Spinat, Rote Rübe), *Malvaceae* (beispielsweise Okra), *Asparagaceae* (beispielsweise Spargel); Nutzpflanzen und Zierpflanzen in Garten und Wald; sowie jeweils genetisch modifizierte Arten dieser Pflanzen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert. Besonders bevorzugt werden erfindungsgemäß Pflanzen der jeweils handelsüblichen oder in Gebrauch befmdlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio- und Genotypen sein.

Das erfindungsgemäße Behandlungsverfahren kann für die Behandlung von genetisch modifizierten Organismen (GMOs), z. B. Pflanzen oder Samen, verwendet werden. Genetisch modifizierte Pflanzen (oder transgene Pflanzen) sind Pflanzen, bei denen ein heterologes Gen stabil in das Genom integriert worden ist. Der Begriff "heterologes Gen" bedeutet im wesentlichen ein Gen, das außerhalb der Pflanze bereitgestellt oder assembliert wird und das bei Einführung in das Zellkerngenom, das Chloroplastengenom oder das Mitochondriengenom der transformierten Pflanze dadurch neue oder verbesserte agronomische oder sonstige Eigenschaften verleiht, dass es ein interessierendes Protein oder Polypeptid exprimiert oder dass es ein anderes Gen, das in der Pflanze vorliegt bzw. andere Gene, die in der Pflanze vorliegen, herunterreguliert oder abschaltet (zum Beispiel mittels Antisense-Technologie, Cosuppressionstechnologie oder RNAi-Technologie [RNA Interference]). Ein heterologes Gen, das im Genom vorliegt, wird ebenfalls als Transgen bezeichnet. Ein Transgen, das durch sein spezifisches Vorliegen im Pflanzengenom definiert ist, wird als Transformations- bzw. transgenes Event bezeichnet.

In Abhängigkeit von den Pflanzenarten oder Pflanzensorten, ihrem Standort und ihren Wachstumsbedingungen (Böden, Klima, Vegetationsperiode, Ernährung) kann die erfindungsgemäße Behandlung auch zu überadditiven ("synergistischen") Effekten führen. So sind zum Beispiel die folgenden Effekte möglich, die über die eigentlich zu erwartenden Effekte hinausgehen: verringerte Aufwandmengen und/oder erweitertes Wirkungsspektrum und/oder erhöhte Wirksamkeit der Wirkstoffe und Zusammensetzungen, die erfindungsgemäß eingesetzt werden können, besseres Pflanzenwachstum, erhöhte Toleranz gegenüber hohen oder niedrigen Temperaturen, erhöhte Toleranz gegenüber Trockenheit oder Wasser- oder Bodensalzgehalt, erhöhte Blühleistung, Ernteerleichterung, Reifebeschleunigung, höhere Erträge, größere Früchte, größere Pflanzenhöhe, intensiver grüne Farbe des Blatts, frühere Blüte, höhere Qualität und/oder höherer Nährwert der Ernteprodukte, höhere Zuckerkonzentration in den Früchten, bessere Lagerfähigkeit und/oder Verarbeitbarkeit der Ernteprodukte.

In gewissen Aufwandmengen können die erfindungsgemäßen Wirkstoffkombinationen auch eine stärkende Wirkung auf Pflanzen ausüben. Sie eignen sich daher für die Mobilisierung des pflanzlichen Abwehrsystems gegen Angriff durch unerwünschte phytopathogene Pilze und/oder Mikroorganismen und/oder Viren. Dies kann gegebenenfalls einer der Gründe für die erhöhte Wirksamkeit der erfindungsgemäßen Kombinationen sein, zum Beispiel gegen Pilze. Pflanzenstärkende (resistenzinduzierende) Substanzen sollen im vorliegenden Zusammenhang auch solche Substanzen oder Substanzkombinationen bedeuten, die fähig sind, das pflanzliche Abwehrsystem so zu stimulieren, dass die behandelten Pflanzen, wenn sie im Anschluss daran mit unerwünschten phytopathogenen Pilzen inokuliert wurde, einen beträchtlichen Resistenzgrad gegen diese unerwünschten phytopathogenen Pilze aufweisen. Die erfindungsgemäßen Substanzen lassen sich daher zum Schutz von Pflanzen gegen Angriff durch die erwähnten Pathogene innerhalb eines gewissen Zeitraums nach der Behandlung einsetzen. Der Zeitraum, über den eine Schutzwirkung erzielt wird, erstreckt sich im Allgemeinen von 1 bis 10 Tagen, vorzugsweise 1 bis 7 Tagen, nach der Behandlung der Pflanzen mit den Wirkstoffen.

Zu Pflanzen und Pflanzensorten, die vorzugsweise erfindungsgemäß behandelt werden, zählen alle Pflanzen, die über Erbgut verfügen, das diesen Pflanzen besonders vorteilhafte, nützliche Merkmale verleiht (egal, ob dies durch Züchtung und/oder Biotechnologie erzielt wurde).

Pflanzen und Pflanzensorten, die ebenfalls vorzugsweise erfindungsgemäß behandelt werden, sind gegen einen oder mehrere biotische Stressfaktoren resistent, d.h. diese Pflanzen weisen eine verbesserte Abwehr gegen tierische und mikrobielle Schädlinge wie Nematoden, Insekten, Milben, phytopathogene Pilze, Bakterien, Viren und/oder Viroide auf.

Beispiele für Nematoden-resistente Pflanzen sind z.B. folgenden US Patentanmeldungen beschrieben: 11/765,491, 11/765,494, 10/926,819, 10/782,020, 12/032,479, 10/783,417, 10/782,096, 11/657,964, 12/192,904, 11/396,808, 12/166,253, 12/166,239, 12/166,124, 12/166,209, 11/762,886, 12/364,335, 11/763,947, 12/252,453, 12/209,354, 12/491,396 und 12/497,221.

Pflanzen und Pflanzensorten, die ebenfalls erfindungsgemäß behandelt werden können, sind solche Pflanzen, die gegen einen oder mehrere abiotische Stressfaktoren resistent sind. Zu den abiotischen Stressbedingungen können zum Beispiel Dürre, Kälte- und Hitzebedingungen, osmotischer Stress, Staunässe, erhöhter Bodensalzgehalt, erhöhtes Ausgesetztsein an Mineralien, Ozonbedingungen, Starklichtbedingungen, beschränkte Verfügbarkeit von Stickstoffnährstoffen, beschränkte Verfügbarkeit von Phosphornährstoffen oder Vermeidung von Schatten zählen.

Pflanzen und Pflanzensorten, die ebenfalls erfindungsgemäß behandelt werden können, sind solche Pflanzen, die durch erhöhte Ertragseigenschaften gekennzeichnet sind. Ein erhöhter Ertrag kann bei diesen Pflanzen z.B. auf verbesserter Pflanzenphysiologie, verbessertem Pflanzenwuchs und verbesserter Pflanzenentwicklung, wie Wasserverwertungseffizienz, Wasserhalteeffizienz, verbesserter Stickstoffverwertung, erhöhter Kohlenstoffassimilation, verbesserter Photosynthese, verstärkter Keimkraft und beschleunigter Abreife beruhen. Der Ertrag kann weiterhin durch eine verbesserte Pflanzenarchitektur (unter Stress- und Nicht-Stress-Bedingungen) beeinflusst werden, darunter frühe Blüte, Kontrolle der Blüte für die Produktion von Hybridsaatgut, Keimpflanzenwüchsigkeit, Pflanzengröße, Internodienzahl und -abstand, Wurzelwachstum, Samengröße, Fruchtgröße, Schotengröße, Schoten- oder Ährenzahl, Anzahl der Samen pro Schote oder Ähre, Samenmasse, verstärkte Samenfüllung, verringerter Samenausfall, verringertes Schotenplatzen sowie Standfestigkeit. Zu weiteren Ertragsmerkmalen zählen Samenzusammensetzung wie Kohlenhydratgehalt, Proteingehalt, Ölgehalt und Ölzusammensetzung, Nährwert, Verringerung der nährwidrigen Verbindungen, verbesserte Verarbeitbarkeit, verbesserte Lagerfähigkeit.

Pflanzen, die erfindungsgemäß behandelt werden können, sind Hybridpflanzen, die bereits die Eigenschaften der Heterosis bzw. des Hybrideffekts exprimieren, was im Allgemeinen zu höherem Ertrag, höherer Wüchsigkeit, besserer Gesundheit und besserer Resistenz gegen biotische und abiotische Stressfaktoren führt. Solche Pflanzen werden typischerweise dadurch erzeugt, dass man eine ingezüchtete pollensterile Elternlinie (den weiblichen Kreuzungspartner) mit einer anderen ingezüchteten pollenfertilen Elternlinie (dem männlichen Kreuzungspartner) kreuzt. Das Hybridsaatgut wird typischerweise von den pollensterilen Pflanzen geerntet und an Vermehrer verkauft. Pollensterile Pflanzen können manchmal (z. B. beim Mais) durch Entfahnen (d.h. mechanischem Entfernen der männlichen Geschlechtsorgane bzw. der männlichen Blüten), produziert werden; es ist jedoch üblicher, dass die Pollensterilität auf genetischen Determinanten im Pflanzengenom beruht. In diesem Fall, insbesondere dann, wenn es sich bei dem gewünschten Produkt, da man von den Hybridpflanzen ernten will, um die Samen handelt, ist es üblicherweise günstig, sicherzustellen, dass die Pollenfertilität in Hybridpflanzen, die die für die Pollensterilität verantwortlichen genetischen Determinanten enthalten, völlig restoriert wird. Dies kann erreicht werden, indem sichergestellt wird, dass die männlichen Kreuzungspartner entsprechende Fertilitätsrestorergene besitzen, die in der Lage sind, die Pollenfertilität in Hybridpflanzen, die die genetischen Determinanten, die für die Pollensterilität verantwortlich sind, enthalten, zu restorieren. Genetische Determinanten für Pollensterilität können im Cytoplasma lokalisiert sein. Beispiele für cytoplasmatische Pollensterilität (CMS) wurden zum Beispiel für Brassica-Arten beschrieben. Genetische Determinanten für Pollensterilität können jedoch auch im Zellkerngenom lokalisiert sein. Pollensterile Pflanzen können auch mit Methoden der pflanzlichen Biotechnologie, wie Gentechnik, erhalten werden. Ein besonders günstiges Mittel zur Erzeugung von pollensterilen Pflanzen ist in WO 89/10396 beschrieben, wobei zum Beispiel eine Ribonuklease wie eine Barnase selektiv in den Tapetumzellen in den Staubblättern exprimiert wird. Die Fertilität kann dann durch Expression eines Ribonukleasehemmers wie Barstar in den Tapetumzellen restoriert werden.

Pflanzen oder Pflanzensorten (die mit Methoden der Pflanzenbiotechnologie, wie der Gentechnik, erhalten werden), die erfindungsgemäß behandelt werden können, sind herbizidtolerante Pflanzen, d. h. Pflanzen, die gegenüber einem oder mehreren vorgegebenen Herbiziden tolerant gemacht worden sind. Solche Pflanzen können entweder durch genetische Transformation oder durch Selektion von Pflanzen, die eine Mutation enthalten, die solch eine Herbizidtoleranz verleiht, erhalten werden.

Herbizidtolerante Pflanzen sind zum Beispiel glyphosatetolerante Pflanzen, d. h. Pflanzen, die gegenüber dem Herbizid Glyphosate oder dessen Salzen tolerant gemacht worden sind. Pflanzen können mit verschiedenen Methoden tolerant gegenüber Glyphosate gemacht werden. So können zum Beispiel glyphosatetolerante Pflanzen durch Transformation der Pflanze mit einem Gen, das für das Enzym 5-Enolpyruvylshikimat-3-phosphatsynthase (EPSPS) kodiert, erhalten werden. Beispiele für solche EPSPS-Gene sind das AroA-Gen (Mutante CT7) des Bakterium *Salmonella typhimurium* (Comai et al., 1983, Science 221, 370-371), das CP4-Gen des Bakteriums *Agrobacterium sp.* (Barry et al., 1992, Curr. Topics Plant Physiol. 7, 139-145), die Gene, die für eine EPSPS aus der Petunie (Shah et al., 1986, Science 233, 478-481), für eine EPSPS aus der Tomate (Gasser et al., 1988, J. Biol. Chem. 263, 4280-4289) oder für eine EPSPS aus Eleusine (WO 01/66704) kodieren. Es kann sich auch um eine mutierte EPSPS handeln. Glyphosate-tolerante Pflanzen können auch dadurch erhalten werden, dass man ein Gen exprimiert, das für ein Glyphosate-Oxidoreduktase-Enzym kodiert. Glyphosate-tolerante Pflanzen können auch dadurch erhalten werden, dass man ein Gen exprimiert, das für ein Glyphosate-acetyltransferase-Enzym kodiert. Glyphosatetolerante Pflanzen können auch dadurch erhalten werden, dass man Pflanzen, die natürlich vorkommende Mutationen der oben erwähnten Gene selektiert. Pflanzen, die EPSPS Gene, welche Glyphosate-Toleranz verleihen, exprimieren, sind beschrieben. Pflanzen, welche andere Gene, die Glyphosate-Toleranz verleihen, z.B. Decarboxylase-Gene, sind beschrieben.

Sonstige herbizidresistente Pflanzen sind zum Beispiel Pflanzen, die gegenüber Herbiziden, die das Enzym Glutaminsynthase hemmen, wie Bialaphos, Phosphinotricin oder Glufosinate, tolerant gemacht worden sind. Solche Pflanzen können dadurch erhalten werden, dass man ein Enzym exprimiert, das das Herbizid oder eine Mutante des Enzyms Glutaminsynthase, das gegenüber Hemmung resistent ist, entgiftet. Solch ein wirksames entgiftendes Enzym ist zum Beispiel ein Enzym, das für ein Phosphinotricin-acetyltransferase kodiert (wie zum Beispiel das bar- oder pat-Protein aus Streptomyces-Arten). Pflanzen, die eine exogene Phosphinotricin-acetyltransferase exprimieren, sind beschrieben.

Weitere herbizidtolerante Pflanzen sind auch Pflanzen, die gegenüber den Herbiziden, die das Enzym Hydroxyphenylpyruvatdioxygenase (HPPD) hemmen, tolerant gemacht worden sind. Bei den Hydroxyphenylpyruvatdioxygenasen handelt es sich um Enzyme, die die Reaktion, in der para-Hydroxyphenylpyruvat (HPP) zu Homogentisat umgesetzt wird, katalysieren. Pflanzen, die gegenüber HPPD-Hemmern tolerant sind, können mit einem Gen, das für ein natürlich vorkommendes resistentes HPPD-Enzym kodiert, oder einem Gen, das für ein mutiertes oder chimäres HPPD-Enzym kodiert, transformiert werden, wie in WO 96/38567, WO 99/24585, WO 99/24586, WO 2009/144079, WO 2002/046387 oder US 6,768,044 beschrieben. Eine Toleranz gegenüber HPPD-Hemmern kann auch dadurch erzielt werden, dass man Pflanzen mit Genen transformiert, die für gewisse Enzyme kodieren, die die Bildung von Homogentisat trotz Hemmung des nativen HPPD-Enzyms durch den HPPD-Hemmer ermöglichen. Solche Pflanzen sind in WO 99/34008 und WO 02/36787 beschrieben. Die Toleranz von Pflanzen gegenüber HPPD-Hemmern kann auch dadurch verbessert werden, dass man Pflanzen zusätzlich zu einem Gen, das für ein HPPD-tolerantes Enzym kodiert, mit einem Gen transformiert, das für ein Prephenatdehydrogenase-Enzym kodiert, wie in WO 2004/024928 beschrieben ist. Außerdem können Pflanzen noch toleranter gegen HPPD-Hemmern gemacht werden, indem man ein Gen in ihr Genom einfügt, welches für ein Enzym kodiert, das HPPD-Hemmer metabolisiert oder abbaut, wie z.B. CYP450 Enzyme (siehe WO 2007/103567 und WO 2008/150473).

Weitere herbizidresistente Pflanzen sind Pflanzen, die gegenüber Acetolactatsynthase (ALS)-Hemmern tolerant gemacht worden sind. Zu bekannten ALS-Hemmern zählen zum Beispiel Sulfonylharnstoff, Imidazolinon, Triazolopyrimidine, Pyrimidinyloxy(thio)benzoate und/oder Sulfonylaminocarbonyltriazolinon-Herbizide. Es ist bekannt, dass verschiedene Mutationen im Enzym ALS (auch als Acetohydroxysäure-Synthase, AHAS, bekannt) eine Toleranz gegenüber unterschiedlichen Herbiziden bzw. Gruppen von Herbiziden verleihen wie z.B. in Tranel und Wright (Weed Science 2002, 50, 700-712) beschrieben ist. Die Herstellung von sulfonylharnstofftoleranten Pflanzen und imidazolinontoleranten Pflanzen ist beschrieben. Weitere sulfonylharnstoff- und imidazolinontolerante Pflanzen sind auch beschrieben.

Weitere Pflanzen, die gegenüber Imidazolinon und/oder Sulfonylhamstoff tolerant sind, können durch induzierte Mutagenese, Selektion in Zellkulturen in Gegenwart des Herbizids oder durch Mutationszüchtung erhalten werden (vgl. z.B. für Sojabohne US 5,084,082, für Reis WO 97/41218, für Zuckerrübe US 5,773,702 und WO 99/057965, für Salat US 5,198,599 oder für Sonnenblume WO 01/065922).

Pflanzen oder Pflanzensorten (die nach Methoden der pflanzlichen Biotechnologie, wie der Gentechnik, erhalten wurden), die ebenfalls erfindungsgemäß behandelt werden können, sind insektenresistente transgene Pflanzen, d.h. Pflanzen, die gegen Befall mit gewissen Zielinsekten resistent gemacht wurden. Solche Pflanzen können durch genetische Transformation oder durch Selektion von Pflanzen, die eine Mutation enthalten, die solch eine Insektenresistenz verleiht, erhalten werden.

Der Begriff "insektenresistente transgene Pflanze" umfasst im vorliegenden Zusammenhang jegliche Pflanze, die mindestens ein Transgen enthält, das eine Kodiersequenz umfasst, die für folgendes kodiert:
1) ein insektizides Kristallprotein aus *Bacillus thuringiensis* oder einen insektiziden Teil davon, wie die insektiziden Kristallproteine, aufgelistet von Crickmore et al. (Microbiology and Molecular Biology Reviews 1998, 62, 807-813), aktualisiert von Crickmore et al. (2005) bei der *Bacillus thuringiensis* Toxin Nomenclatur, online bei: http://www.lifesci.sussex.ac.uk/Home/Neil_Crickmore/Bt/),
   oder insektizide Teile davon, z.B. Proteine der Cry-Proteinklassen Cry1Ab, Cry1Ac, Cry1B, Cry1C, Cry1D, Cry1F, Cry2Ab, Cry3Aa, or Cry3Bb oder insektizide Teile davon (z.B. EP-A 1999141 und WO 2007/107302), oder solche Proteine, kodiert durch synthetische Gene wie in US Patentanmeldung 12/249,016 beschrieben ist; oder
2) ein Kristallprotein aus *Bacillus thuringiensis* oder einen Teil davon, der in Gegenwart eines zweiten, anderen Kristallproteins als *Bacillus thuringiensis* oder eines Teils davon insektizid wirkt, wie das binäre Toxin, das aus den Kristallproteinen Cy34 und Cy35 besteht (Nat. Biotechnol. 2001, 19, 668-72; Applied Environm. Microbiol. 2006, 71, 1765-1774) oder das binäre Toxin, das aus den Cry1A oder Cry1F Proteinen besteht und die Cry2Aa oder Cry2Ab oder Cry2Ae Proteine (US Patentanmeldung 12/214,022 und EP08010791.5); oder
3) ein insektizides Hybridprotein, das Teile von zwei unterschiedlichen insektiziden Kristallproteinen aus *Bacillus thuringiensis* umfasst, wie zum Beispiel ein Hybrid aus den Proteinen von 1) oben oder ein Hybrid aus den Proteinen von 2) oben, z. B. das Protein Cry1A.105, das von dem Mais-Event MON98034 produziert wird (WO 2007/027777); oder
4) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden, wie das Protein Cry3Bb1 in Mais-Events MON863 oder MON88017 oder das Protein Cry3A im Mais-Event MIR 604;
5) ein insektizides sezerniertes Protein aus *Bacillus thuringiensis* oder *Bacillus cereus* oder einen insektiziden Teil davon, wie die vegetativ wirkenden insektentoxischen Proteine (vegetative insekticidal proteins, VIP), die unter http://www.lifesci.sussex.ac.uk/Home/Neil_Crickmore/Bt/vip.html angeführt sind, z. B. Proteine der Proteinklasse VIP3Aa; oder
6) ein sezerniertes Protein aus *Bacillus thuringiensis* oder *Bacillus cereus,* das in Gegenwart eines zweiten sezernierten Proteins aus *Bacillus thuringiensis* oder *B. cereus* insektizid wirkt, wie das binäre Toxin, das aus den Proteinen VIP1A und VIP2A besteht (WO 94/21795); oder
7) ein insektizides Hybridprotein, das Teile von verschiedenen sezernierten Proteinen von *Bacillus thuringiensis* oder *Bacillus cereus* umfasst, wie ein Hybrid der Proteine von 1) oder ein Hybrid der Proteine von 2) oben; oder
8) ein Protein gemäß einem der Punkte 5) bis 7) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden (wobei die Kodierung für ein insektizides Protein erhalten bleibt), wie das Protein VIP3Aa im Baumwoll-Event COT 102; oder
9) ein sezerniertes Protein aus *Bacillus thuringiensis* oder *Bacillus cereus,* das in Gegenwart eines Kristallproteins von *Bacillus thuringiensis* insektizid wirkt, wie das binäre Toxin, das aus den Proteinen VIP3 und Cry1A oder Cry1F besteht (US Patentanmeldungen 61/126083 und 61/195019), oder das binäre Toxin, das aus dem VIP3 Protein und den Cry2Aa oder Cry2Ab oder Cry2Ae Proteinen besteht (US Patentanmeldung 12/214,022 und EP 08010791.5); oder
10) ein Protein gemäß Punkt 9) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden (wobei die Kodierung für ein insektizides Protein erhalten bleibt).

Natürlich zählt zu den insektenresistenten transgenen Pflanzen im vorliegenden Zusammenhang auch jegliche Pflanze, die eine Kombination von Genen umfasst, die für die Proteine von einer der oben genannten Klassen 1 bis 10 kodieren. In einer Ausführungsform enthält eine insektenresistente Pflanze mehr als ein Transgen, das für ein Protein nach einer der oben genannten 1 bis 10 kodiert, um das Spektrum der entsprechenden Zielinsektenarten zu erweitern oder um die Entwicklung einer Resistenz der Insekten gegen die Pflanzen dadurch hinauszuzögern, dass man verschiedene Proteine einsetzt, die für dieselbe Zielinsektenart insektizid sind, jedoch eine unterschiedliche Wirkungsweise, wie Bindung an unterschiedliche Rezeptorbindungsstellen im Insekt, aufweisen.

Eine "insekten-resistente transgene Pflanze" umfasst im vorliegenden Zusammenhang weiterhin jede Pflanze, die wenigstens ein Transgen enthält, welches eine Sequenz zur Herstellung einer Doppelstrang-RNA umfasst, die nach Nahrungsaufnahme durch einen Insektenschädling das Wachstum dieses Schädlings hindert.

Pflanzen oder Pflanzensorten (die nach Methoden der pflanzlichen Biotechnologie, wie der Gentechnik, erhalten wurden), die ebenfalls erfindungsgemäß behandelt werden können, sind gegenüber abiotischen Stressfaktoren tolerant. Solche Pflanzen können durch genetische Transformation oder durch Selektion von Pflanzen, die eine Mutation enthalten, die solch eine Stressresistenz verleiht, erhalten werden. Zu besonders nützlichen Pflanzen mit Stresstoleranz zählen folgende:
a. Pflanzen, die ein Transgen enthalten, das die Expression und/oder Aktivität des Gens für die Poly(ADP-ribose)polymerase (PARP) in den Pflanzenzellen oder Pflanzen zu reduzieren vermag.
b. Pflanzen, die ein stresstoleranzförderndes Transgen enthalten, das die Expression und/oder Aktivität der für PARG kodierenden Gene der Pflanzen oder Pflanzenzellen zu reduzieren vermag;
c. Pflanzen, die ein stresstoleranzförderndes Transgen enthalten, das für ein in Pflanzen funktionelles Enzym des Nicotinamidadenindinukleotid-Salvage-Biosynthesewegs kodiert, darunter Nicotinamidase, Nicotinatphosphoribosyltransferase, Nicotinsäuremononukleotidadenyltransferase, Nicotinamidadenindinukleotidsynthetase oder Nicotinamidphosphoribosyltransferase.

Pflanzen oder Pflanzensorten (die nach Methoden der pflanzlichen Biotechnologie, wie der Gentechnik, erhalten wurden), die ebenfalls erfindungsgemäß behandelt werden können, weisen eine veränderte Menge, Qualität und/oder Lagerfähigkeit des Ernteprodukts und/oder veränderte Eigenschaften von bestimmten Bestandteilen des Ernteprodukts auf, wie zum Beispiel:
1) Transgene Pflanzen, die eine modifizierte Stärke synthetisieren, die bezüglich ihrer chemischphysikalischen Eigenschaften, insbesondere des Amylosegehalts oder des Amylose/Amylopektin-Verhältnisses, des Verzweigungsgrads, der durchschnittlichen Kettenlänge, der Verteilung der Seitenketten, des Viskositätsverhaltens, der Gelfestigkeit, der Stärkekorngröße und/oder Stärkekornmorphologie im Vergleich mit der synthetisierten Stärke in Wildtyppflanzenzellen oder -pflanzen verändert ist, so dass sich diese modifizierte Stärke besser für bestimmte Anwendungen eignet.
2) Transgene Pflanzen, die Nichtstärkekohlenhydratpolymere synthetisieren, oder Nichtstärkekohlenhydratpolymere, deren Eigenschaften im Vergleich zu Wildtyppflanzen ohne genetische Modifikation verändert sind. Beispiele sind Pflanzen, die Polyfructose, insbesondere des Inulin- und Levantyps, produzieren, Pflanzen, die alpha-1,4-Glucane produzieren, Pflanzen, die alpha-1,6-verzweigte alpha-1,4-Glucane produzieren und Pflanzen, die Alternan produzieren.
3) Transgene Pflanzen, die Hyaluronan produzieren.
4) Transgene Pflanzen oder Hybridpflanzen wie Zwiebeln mit bestimmten Eigenschaften wie "hohem Anteil an löslichen Feststoffen" (,high soluble solids content'), geringe Schärfe (,low pungency', LP) und/oder lange Lagerfähigkeit (,long storage', LS).

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Baumwollpflanzen mit veränderten Fasereigenschaften. Solche Pflanzen können durch genetische Transformation oder durch Selektion von Pflanzen, die eine Mutation enthalten, die solche veränderten Fasereigenschaften verleiht, erhalten werden; dazu zählen:
a) Pflanzen wie Baumwollpflanzen, die eine veränderte Form von Cellulosesynthasegenen enthalten,
b) Pflanzen wie Baumwollpflanzen, die eine veränderte Form von rsw2- oder rsw3-homologen Nukleinsäuren enthalten, wie Baumwollpflanzen mit einer erhöhten Expression der Saccharosephosphatsynthase;
c) Pflanzen wie Baumwollpflanzen mit einer erhöhten Expression der Saccharosesynthase;
d) Pflanzen wie Baumwollpflanzen bei denen der Zeitpunkt der Durchlaßsteuerung der Plasmodesmen an der Basis der Faserzelle verändert ist, z. B. durch Herunterregulieren der faserselektiven β-1,3-Glucanase;
e) Pflanzen wie Baumwollpflanzen mit Fasern mit veränderter Reaktivität, z. B. durch Expression des N-Acetylglucosamintransferasegens, darunter auch nodC, und von Chitinsynthasegenen.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Raps oder verwandte Brassica-Pflanzen mit veränderten Eigenschaften der Ölzusammensetzung. Solche Pflanzen können durch genetische Transformation oder durch Selektion von Pflanzen, die eine Mutation enthalten, die solche veränderten Öleigenschaften verleiht, erhalten werden; dazu zählen:
a) Pflanzen wie Rapspflanzen, die Öl mit einem hohen Ölsäuregehalt produziere;
b) Pflanzen wie Rapspflanzen, die Öl mit einem niedrigen Linolensäuregehalt produzieren.
c) Pflanzen wie Rapspflanzen, die Öl mit einem niedrigen gesättigten Fettsäuregehalt produzieren.

Pflanzen oder Pflanzensorten (die nach Methoden der pflanzlichen Biotechnologie, wie der Gentechnik, erhalten werden können), die ebenfalls erfindungsgemäß behandelt werden können, sind Pflanzen wie Kartoffeln, welche Virus-resistent sind z.B. gegen den Kartoffelvirus Y (Event SY230 und SY233 von Tecnoplant, Argentinien), oder welche resistent gegen Krankheiten wie die Kraut- und Knollenfäule (potato late blight) (z.B. RB Gen), oder welche eine verminderte kälteinduzierte Süße zeigen (welche die Gene Nt-Inh, II-INV tragen) oder welche den Zwerg-Phänotyp zeigen (Gen A-20 Oxidase).

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Raps oder verwandte Brassica-Pflanzen mit veränderten Eigenschaften im Samenausfall (seed shattering). Solche Pflanzen können durch genetische Transformation oder durch Selektion von Pflanzen, die eine Mutation enthalten, die solche veränderten Eigenschaften verleihen, und umfassen Pflanzen wie Raps mit verzögertem oder vermindertem Samenausfall.

Besonders nützliche transgene Pflanzen, die erfindungsgemäß behandelt werden können, sind Pflanzen mit Transformationsevents oder Kombinationen von Transformationsevent, welche in den USA beim Animal and Plant Health Inspection Service (APHIS) of the United States Department of Agriculture (USDA) Gegenstand von erteilten oder anhängigen Petitionen für den nichtregulierten Status sind. Die Information hierzu ist jederzeit beim APHIS (4700 River Road Riverdale, MD 20737, USA) erhältlich, z.B. über die Internetseite http://www.aphis.usda.gov/brs/not_reg.html. Am Anmeldetag dieser Anmeldung waren beim APHIS die Petitionen mit folgenden Informationen entweder erteilt oder anhängig:
- Petition: Identifikationsnummer der Petition. Die Technische Beschreibung des Transformationsevents kann im einzelnen Petitionsdokument erhältlich von APHIS auf der Website über die Petitionsnummer gefunden werden. Diese Beschreibungen sind hiermit per Referenz offenbart.
- Erweiterung einer Petition: Referenz zu einer frühere Petition, für die eine Erweiterung oder Verlängerung beantragt wird.
- Institution: Name der die Petition einreichenden Person.
- Regulierter Artikel: die betroffen Pflanzenspecies.
- Transgener Phänotyp: die Eigenschaft ("Trait"), die der Pflanze durch das Transformationsevent verliehen wird.
- Transformationevent oder -linie: der Name des oder der Events (manchmal auch als Linie(n) bezeichnet), für die der nicht-regulierte Status beantragt ist.
- APHIS Documente: verschiedene Dokumente, die von APHIS bzgl. der Petition veröffentlicht warden oder von APHIS auf Anfrage erhalten werden können.

Besonders nützliche transgene Pflanzen, die erfindungsgemäß behandelt werden können, sind Pflanzen mit einem oder mehreren Genen, die für ein oder mehrere Toxine kodieren, sind die transgenen Pflanzen, die unter den folgenden Handelsbezeichnungen angeboten werden: YIELD GARD® (zum Beispiel Mais, Baumwolle, Sojabohnen), KnockOut® (zum Beispiel Mais), BiteGard® (zum Beispiel Mais), BT-Xtra® (zum Beispiel Mais), StarLink® (zum Beispiel Mais), Bollgard® (Baumwolle), Nucotn® (Baumwolle), Nucotn 33B® (Baumwolle), NatureGard® (zum Beispiel Mais), Protecta® und NewLeaf® (Kartoffel). Herbizidtolerante Pflanzen, die zu erwähnen sind, sind zum Beispiel Maissorten, Baumwollsorten und Sojabohnensorten, die unter den folgenden Handelsbezeichnungen angeboten werden: Roundup Ready® (Glyphosatetoleranz, zum Beispiel Mais, Baumwolle, Sojabohne), Liberty Link® (Phosphinotricintoleranz, zum Beispiel Raps), IMI® (Imidazolinontoleranz) und SCS® (Sylfonylharnstofftoleranz), zum Beispiel Mais. Zu den herbizidresistenten Pflanzen (traditionell auf Herbizidtoleranz gezüchtete Pflanzen), die zu erwähnen sind, zählen die unter der Bezeichnung Clearfield® angebotenen Sorten (zum Beispiel Mais).

Besonders nützliche transgene Pflanzen, die erfindungsgemäß behandelt werden können, sind Pflanzen, die Transformations-Events, oder eine Kombination von Transformations-Events, enthalten und die zum Beispiel in den Dateien von verschiedenen nationalen oder regionalen Behörden angeführt sind (siehe zum Beispiel http://gmoinfo.jrc.it/gmp_browse.aspx und http://cera-gmc.org/index.php?evidcode=&hstIDXCode=&gType=&AbbrCode=&atCode=&stCode=& coIDCode=&action=gm_crop_database&mode=Submit).

Die erfindungsgemäßen Wirkstoffe bzw. Mittel können außerdem im Materialschutz zum Schutz von technischen Materialien gegen Befall und Zerstörung durch unerwünschte Mikroorganismen, wie z.B. Pilzen und Insekten, eingesetzt werden.

Weiter können die erfindungsgemäßen Verbindungen allein oder in Kombinationen mit anderen Wirkstoffen als Antifouling-Mittel eingesetzt werden.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier, Wandpappe und Karton, Textilien, Teppiche, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen und Gebäuden, z.B. Kühlwasserkreisläufe, Kühl- und Heizsysteme und Belüftungs- und Klimaanlagen, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz. Die erfindungsgemäßen Wirkstoffe bzw. Mittel können nachteilige Effekte wie Vermodern, Verfall, Ver-, Entfärbung oder Verschimmeln verhindern. Außerdem können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Das erfindungsgemäße Verfahren zum Bekämpfen von unerwünschten Pilzen kann auch zum Schutz von so genannten Storage Goods verwendet werden. Unter "Storage Goods" werden dabei natürliche Substanzen pflanzlichen oder tierischen Ursprungs oder deren Verarbeitungsprodukte, welche der Natur entnommen wurden und für die Langzeitschutz gewünscht ist, verstanden. Storage Goods pflanzlichen Ursprungs, wie z.B. Pflanzen oder Pflanzenteile, wie Stiele, Blätter, Knollen, Samen, Früchte, Körner, können in frisch geerntetem Zustand oder nach Verarbeitung durch (Vor-)Trocknen, Befeuchten, Zerkleinern, Mahlen, Pressen oder Rösten, geschützt werden. Storage Goods umfasst auch Nutzholz, sei es unverarbeitet, wie Bauholz, Stromleitungsmasten und Schranken, oder in Form fertiger Produkte, wie Möbel. Storage Goods tierischen Ursprungs sind beispielsweise Felle, Leder, Pelze und Haare. Die erfindungsgemäßen Wirkstoffe können nachteilige Effekte wie Vermodern, Verfall, Ver-, Entfärbung oder Verschimmeln verhindern.

Beispielhaft, aber nicht begrenzend, seien einige Erreger von pilzlichen Erkrankungen, die erfindungsgemäß behandelt werden können, genannt:
Erkrankungen, hervorgerufen durch Erreger des Echten Mehltaus wie z.B. Blumeria-Arten, wie beispielsweise Blumeria graminis; Podosphaera-Arten, wie beispielsweise Podosphaera leucotricha; Sphaerotheca-Arten, wie beispielsweise Sphaerotheca fuliginea; Uncinula-Arten, wie beispielsweise Uncinula necator;
Erkrankungen, hervorgerufen durch Erreger von Rostkrankheiten wie z.B. Gymnosporangium-Arten, wie beispielsweise Gymnosporangium sabinae; Hemileia-Arten, wie beispielsweise Hemileia vastatrix; Phakopsora-Arten, wie beispielsweise Phakopsora pachyrhizi und Phakopsora meibomiae; Puccinia-Arten, wie beispielsweise Puccinia recondita oder Puccinia triticina; Uromyces-Arten, wie beispielsweise Uromyces appendiculatus;
Erkrankungen, hervorgerufen durch Erreger der Gruppe der Oomyceten wie z.B. Bremia-Arten, wie beispielsweise Bremia lactucae; Peronospora-Arten, wie beispielsweise Peronospora pisi oder P. brassicae; Phytophthora-Arten, wie beispielsweise Phytophthora infestans; Plasmopara-Arten, wie beispielsweise Plasmopara viticola; Pseudoperonospora-Arten, wie beispielsweise Pseudoperonospora humuli oder Pseudoperonospora cubensis; Pythium-Arten, wie beispielsweise Pythium ultimum;
Blattfleckenkrankheiten und Blattwelken, hervorgerufen durch z.B. Alternaria-Arten, wie beispielsweise Alternaria solani; Cercospora-Arten, wie beispielsweise Cercospora beticola; Cladiosporum-Arten, wie beispielsweise Cladiosporium cucumerinum; Cochliobolus-Arten, wie beispielsweise Cochliobolus sativus (Konidienform: Drechslera, Syn: Helminthosporium); Colletotrichum-Arten, wie beispielsweise Colletotrichum lindemuthanium; Cycloconium-Arten, wie beispielsweise Cycloconium oleaginum; Diaporthe-Arten, wie beispielsweise Diaporthe citri; Elsinoe-Arten, wie beispielsweise Elsinoe fawcettii; Gloeosporium-Arten, wie beispielsweise Gloeosporium laeticolor; Glomerella-Arten, wie beispielsweise Glomerella cingulata; Guignardia-Arten, wie beispielsweise Guignardia bidwelli; Leptosphaeria-Arten, wie beispielsweise Leptosphaeria maculans; Magnaporthe-Arten, wie beispielsweise Magnaporthe grisea; Microdochium-Arten, wie beispielsweise Microdochium nivale; Mycosphaerella-Arten, wie beispielsweise Mycosphaerella graminicola und M. fijiensis; Phaeosphaeria-Arten, wie beispielsweise Phaeosphaeria nodorum; Pyrenophora-Arten, wie beispielsweise Pyrenophora teres; Ramularia-Arten, wie beispielsweise Ramularia collo-cygni; Rhynchosporium-Arten, wie beispielsweise Rhynchosporium secalis; Septoria-Arten, wie beispielsweise Septoria apii; Typhula-Arten, wie beispielsweise Typhula incarnata; Venturia-Arten, wie beispielsweise Venturia inaequalis;
Wurzel- und Stängelkrankheiten, hervorgerufen durch z.B. Corticium-Arten, wie beispielsweise Corticium graminearum; Fusarium-Arten, wie beispielsweise Fusarium oxysporum; Gaeumannomyces-Arten, wie beispielsweise Gaeumannomyces graminis; Rhizoctonia-Arten, wie beispielsweise Rhizoctonia solani; Tapesia-Arten, wie beispielsweise Tapesia acuformis; Thielaviopsis-Arten, wie beispielsweise Thielaviopsis basicola;
Ähren- und Rispenerkrankungen (inklusive Maiskolben), hervorgerufen durch z.B. Alternaria-Arten, wie beispielsweise Alternaria spp.; Aspergillus-Arten, wie beispielsweise Aspergillus flavus; Cladosporium-Arten, wie beispielsweise Cladosporium cladosporioides; Claviceps-Arten, wie beispielsweise Claviceps purpurea; Fusarium-Arten, wie beispielsweise Fusarium culmorum; Gibberella-Arten, wie beispielsweise Gibberella zeae; Monographella-Arten, wie beispielsweise Monographella nivalis; Septoria-Arten, wie beispielsweise Septoria nodorum;
Erkrankungen, hervorgerufen durch Brandpilze wie z.B. Sphacelotheca-Arten, wie beispielsweise Sphacelotheca reiliana; Tilletia-Arten, wie beispielsweise Tilletia caries, T. controversa; Urocystis-Arten, wie beispielsweise Urocystis occulta; Ustilago-Arten, wie beispielsweise Ustilago nuda, U. nuda tritici;
Fruchtfäule hervorgerufen durch z.B. Aspergillus-Arten, wie beispielsweise Aspergillus flavus; Botrytis-Arten, wie beispielsweise Botrytis cinerea; Penicillium-Arten, wie beispielsweise Penicillium expansum und P. purpurogenum; Sclerotinia-Arten, wie beispielsweise Sclerotinia sclerotiorum;
Verticilium-Arten, wie beispielsweise Verticilium alboatrum;
Samen- und bodenbürtige Fäulen und Welken, sowie Sämlingserkrankungen, hervorgerufen durch z.B. Fusarium-Arten, wie beispielsweise Fusarium culmorum; Phytophthora Arten, wie beispielsweise Phytophthora cactorum; Pythium-Arten, wie beispielsweise Pythium ultimum; Rhizoctonia-Arten, wie beispielsweise Rhizoctonia solani; Sclerotium-Arten, wie beispielsweise Sclerotium rolfsii;
Krebserkrankungen, Gallen und Hexenbesen, hervorgerufen durch z.B. Nectria-Arten, wie beispielsweise Nectria galligena;
Welkeerkrankungen hervorgerufen durch z.B. Monilinia-Arten, wie beispielsweise Monilinia laxa;
Deformationen von Blättern, Blüten und Früchten, hervorgerufen durch z.B. Taphrina-Arten, wie beispielsweise Taphrina deformans;
Degenerationserkrankungen holziger Pflanzen, hervorgerufen durch z.B. Esca-Arten, wie beispielsweise Phaemoniella clamydospora und Phaeoacremonium aleophilum und Fomitiporia mediterranea;
Blüten- und Samenerkrankungen, hervorgerufen durch z.B. Botrytis-Arten, wie beispielsweise Botrytis cinerea;
Erkrankungen von Pflanzenknollen, hervorgerufen durch z.B. Rhizoctonia-Arten, wie beispielsweise Rhizoctonia solani; Helminthosporium-Arten, wie beispielsweise Helminthosporium solani;
Erkrankungen, hervorgerufen durch bakterielle Erreger wie z.B. Xanthomonas-Arten, wie beispielsweise Xanthomonas campestris pv. oryzae; Pseudomonas-Arten, wie beispielsweise Pseudomonas syringae pv. lachrymans; Erwinia-Arten, wie beispielsweise Erwinia amylovora;
Bevorzugt können die folgenden Krankheiten von Soja-Bohnen bekämpft werden:
   Pilzkrankheiten an Blättern, Stängeln, Schoten und Samen verursacht durch z.B. Alternaria leaf spot (Alternaria spec. atrans tenuissima), Anthracnose (Colletotrichum gloeosporoides dematium var. truncatum), Brown spot (Septoria glycines), Cercospora leaf spot and blight (Cercospora kikuchii), Choanephora leaf blight (Choanephora infundibulifera trispora (Syn.)), Dactuliophora leaf spot (Dactuliophora glycines), Downy Mildew (Peronospora manshurica), Drechslera blight (Drechslera glycini), Frogeye Leaf spot (Cercospora sojina), Leptosphaerulina Leaf Spot (Leptosphaerulina trifolii), Phyllostica Leaf Spot (Phyllosticta sojaecola), Pod and Stem Blight (Phomopsis sojae), Powdery Mildew (Microsphaera diffusa), Pyrenochaeta Leaf Spot (Pyrenochaeta glycines), Rhizoctonia Aerial, Foliage, and Web Blight (Rhizoctonia solani), Rust (Phakopsora pachyrhizi, Phakopsora meibomiae), Scab (Sphaceloma glycines), Stemphylium Leaf Blight (Stemphylium botryosum), Target Spot (Corynespora cassiicola).

Pilzkrankheiten an Wurzeln und der Stängelbasis verursacht durch z.B. Black Root Rot (Calonectria crotalariae), Charcoal Rot (Macrophomina phaseolina), Fusarium Blight or Wilt, Root Rot, and Pod and Collar Rot (Fusarium oxysporum, Fusarium orthoceras, Fusarium semitectum, Fusarium equiseti), Mycoleptodiscus Root Rot (Mycoleptodiscus terrestris), Neocosmospora (Neocosmopspora vasinfecta), Pod and Stem Blight (Diaporthe phaseolorum), Stem Canker (Diaporthe phaseolorum var. caulivora), Phytophthora Rot (Phytophthora megasperma), Brown Stem Rot (Phialophora gregata), Pythium Rot (Pythium aphanidermatum, Pythium irregulare, Pythium debaryanum, Pythium myriotylum, Pythium ultimum), Rhizoctonia Root Rot, Stem Decay, and Damping-Off (Rhizoctonia solani), Sclerotinia Stem Decay (Sclerotinia sclerotiorum), Sclerotinia Southern Blight (Sclerotinia rolfsii), Thielaviopsis Root Rot (Thielaviopsis basicola).

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, Holz verfärbende und Holz zerstörende Pilze (Basidiomyceten) sowie gegen Schleimorganismen und Algen. Es seien beispielsweise Mikroorganismen der folgenden Gattungen genannt: Alternaria, wie Alternaria tenuis; Aspergillus, wie Aspergillus niger; Chaetomium, wie Chaetomium globosum; Coniophora, wie Coniophora puetana; Lentinus, wie Lentinus tigrinus; Penicillium, wie Penicillium glaucum; Polyporus, wie Polyporus versicolor; Aureobasidium, wie Aureobasidium pullulans; Sclerophoma, wie Sclerophoma pityophila; Trichoderma, wie Trichoderma viride; Escherichia, wie Escherichia coli; Pseudomonas, wie Pseudomonas aeruginosa; Staphylococcus, wie Staphylococcus aureus.

Darüber hinaus weisen die erfindungsgemäßen Wirkstoffe auch sehr gute antimykotische Wirkungen auf. Sie besitzen ein sehr breites antimykotisches Wirkungsspektrum, insbesondere gegen Dermatophyten und Sprosspilze, Schimmel und diphasische Pilze (z.B. gegen Candida-Spezies wie Candida albicans, Candida glabrata) sowie Epidermophyton floccosum, Aspergillus-Spezies wie Aspergillus niger und Aspergillus fumigatus, Trichophyton-Spezies wie Trichophyton mentagrophytes, Microsporon-Spezies wie Microsporon canis und audouinii. Die Aufzählung dieser Pilze stellt keinesfalls eine Beschränkung des erfassbaren mykotischen Spektrums dar, sondern hat nur erläuternden Charakter.

Die erfindungsgemäßen Wirkstoffe können daher sowohl in medizinischen als auch in nicht-medizinischen Anwendungen eingesetzt werden.

Beim Einsatz der erfindungsgemäßen Wirkstoffe als Fungizide können die Aufwandmengen je nach Applikationsart innerhalb eines größeren Bereiches variiert werden. Die Aufwandmenge der erfindungsgemäßen Wirkstoffe beträgt
- bei der Behandlung von Pflanzenteilen, z.B. Blättern: von 0,1 bis 10 000 g/ha, bevorzugt von 10 bis 1 000 g/ha, besonders bevorzugt von 50 bis 300g/ha (bei Anwendung durch Gießen oder Tropfen kann die Aufwandmenge sogar verringert werden, vor allem wenn inerte Substrate wie Steinwolle oder Perlit verwendet werden);
- bei der Saatgutbehandlung: von 2 bis 200 g pro 100 kg Saatgut, bevorzugt von 3 bis 150 g pro 100 kg Saatgut, besonders bevorzugt von 2,5 bis 25 g pro 100 kg Saatgut, ganz besonders bevorzugt von 2,5 bis 12,5 g pro 100 kg Saatgut;
- bei der Bodenbehandlung: von 0,1 bis 10 000 g/ha, bevorzugt von 1 bis 5 000 g/ha.

Diese Aufwandmengen seien nur beispielhaft und nicht limitierend im Sinne der Erfindung genannt.

Die erfindungsgemäßen Wirkstoffe bzw. Mittel können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im Allgemeinen auf 1 bis 28 Tage, bevorzugt auf 1 bis 14 Tage, besonders bevorzugt auf 1 bis 10 Tage, ganz besonders bevorzugt auf 1 bis 7 Tage nach der Behandlung der Pflanzen mit den Wirkstoffen bzw. auf bis zu 200 Tage nach einer Saatgutbehandlung.

Darüber hinaus kann durch die erfindungsgemäße Behandlung der Mykotoxingehalt im Erntegut und den daraus hergestellten Nahrungs- und Futtermitteln verringert werden. Besonders, aber nicht ausschließlich sind hierbei folgende Mykotoxine zu nennen: Deoxynivalenol (DON), Nivalenol, 15-Ac-DON, 3-Ac-DON, T2- und HT2- Toxin, Fumonisine, Zearalenon, Moniliformin, Fusarin, Diaceotoxyscirpenol (DAS), Beauvericin, Enniatin, Fusaroproliferin, Fusarenol, Ochratoxine, Patulin, Mutterkornalkaloide und Aflatoxine, die beispielsweise von den folgenden Pilzen verursacht werden können: Fusarium spec., wie Fusarium acuminatum, F. avenaceum, F. crookwellense, F. culmorum, F. graminearum (Gibberella zeae), F. equiseti, F. fujikoroi, F. musarum, F. oxysporum, F. proliferatum, F. poae, F. pseudograminearum, F. sambucinum, F. scirpi, F. semitectum, F. solani, F. sporotrichoides, F. langsethiae, F. subglutinans, F. tricinctum, F. verticillioides u.a. sowie auch von Aspergillus spec., Penicillium spec., Claviceps purpurea, Stachybotrys spec. u.a.

Die erfindungsgemäßen Verbindungen können gegebenenfalls in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Zwischen- oder Vorprodukte für die Synthese weiterer Wirkstoffe einsetzen.

Die erfindungsgemäßen Wirkstoffe greifen in den Metabolismus der Pflanzen ein und können deshalb auch als Wachstumsregulatoren eingesetzt werden.

Pflanzenwachstumsregulatoren können verschiedenartige Wirkungen auf Pflanzen ausüben. Die Wirkungen der Stoffe hängen im Wesentlichen von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation ab. In jedem Fall sollen Wachstumsregulatoren die Kulturpflanzen in bestimmter gewünschter Weise beeinflussen.

Pflanzenwuchsregulierende Stoffe können zum Beispiel zur Hemmung des vegetativen Wachstums der Pflanzen eingesetzt werden. Eine derartige Wuchshemmung ist unter anderem bei Gräsern von wirtschaftlichem Interesse, denn dadurch kann die Häufigkeit der Grasschnitte in Ziergärten, Park- und Sportanlagen, an Straßenrändern, auf Flughäfen oder in Obstanlagen reduziert werden. Von Bedeutung ist auch die Hemmung des Wuchses von krautigen und holzigen Pflanzen an Straßenrändern und in der Nähe von Pipelines oder Überlandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide. Hierdurch wird die Gefahr des Umknickens ("Lagerns") der Pflanzen vor der Ernte verringert oder vollkommen beseitigt. Außerdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es, höhere Düngermengen auszubringen, um den Ertrag zu steigern, ohne dass die Gefahr besteht, dass das Getreide lagert.

Eine Hemmung des vegetativen Wachstums ermöglicht bei vielen Kulturpflanzen eine dichtere Anpflanzung, so dass Mehrerträger bezogen auf die Bodenfläche erzielt werden können. Ein Vorteil der so erzielten kleineren Pflanzen ist auch, dass die Kultur leichter bearbeitet und geerntet werden kann.

Eine Hemmung des vegetativen Wachstums der Pflanzen kann auch dadurch zu Ertragssteigerungen führen, dass die Nährstoffe und Assimilate in stärkerem Masse der Blüten- und Fruchtbildung zugute kommen als den vegetativen Pflanzenteilen.

Mit Wachstumsregulatoren lässt sich häufig auch eine Förderung des vegetativen Wachstums erzielen. Dies ist von großem Nutzen, wenn die vegetativen Pflanzenteile geerntet werden. Eine Förderung des vegetativen Wachstums kann aber auch gleichzeitig zu einer Förderung des generativen Wachstums führen, dadurch dass mehr Assimilate gebildet werden, so dass mehr oder größere Früchte entstehen.

Ertragssteigerungen können in manchen Fällen durch einen Eingriff in den pflanzlichen Stoffwechsel erreicht werden, ohne dass sich Änderungen des vegetativen Wachstums bemerkbar machen. Ferner kann mit Wachstumsregulatoren eine Veränderung der Zusammensetzung der Pflanzen erreicht werden, was wiederum zu einer Qualitätsverbesserung der Ernteprodukte führen kann. So ist es beispielsweise möglich, den Gehalt an Zucker in Zuckerrüben, Zuckerrohr, Ananas sowie in Zitrusfrüchten zu erhöhen oder den Proteingehalt in Soja oder Getreide zu steigern. Auch ist es beispielsweise möglich, den Abbau erwünschter Inhaltsstoffe, wie z. B. Zucker in Zuckerrüben oder Zuckerrohr, mit Wachstumsregulatoren vor oder nach der Ernte zu hemmen. Außerdem lässt sich die Produktion oder der Abfluss von sekundären Pflanzeninhaltsstoffen positiv beeinflussen. Als Beispiel sei die Stimulierung des Latexflusses bei Gummibäumen genannt.

Unter dem Einfluss von Wachstumsregulatoren kann es zur Ausbildung parthenokarper Früchte kommen. Ferner kann das Geschlecht der Blüten beeinflusst werden. Auch kann eine Sterilität des Pollens erzeugt werden, was bei der Züchtung und Herstellung von Hybridsaatgut eine große Bedeutung hat.

Durch den Einsatz von Wachstumsregulatoren lässt sich die Verzweigung der Pflanzen steuern. Einerseits kann durch Brechen der Apikaldominanz die Entwicklung von Seitentrieben gefördert werden, was besonders im Zierpflanzenbau auch in Verbindung mit einer Wuchshemmung sehr erwünscht sein kann. Andererseits ist es aber auch möglich, das Wachstum der Seitentriebe zu hemmen. Für diese Wirkung besteht z.B. großes Interesse im Tabakanbau oder bei der Anpflanzung von Tomaten.

Unter dem Einfluss von Wachstumsregulatoren kann der Blattbestand der Pflanzen so gesteuert werden, dass ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso lässt sich mit Wachstumsregulatoren der Fruchtfall steuern. Einerseits kann ein vorzeitiger Fruchtfall verhindert werden. Andererseits kann aber auch der Fruchtfall oder sogar das Abfallen der Blüten bis zu einem gewünschten Masse gefördert werden ("Ausdünnung"), um die Alternanz zu brechen. Unter Alternanz versteht man die Eigenart einiger Obst-Arten, endogen bedingt von Jahr zu Jahr sehr unterschiedliche Erträge zu bringen. Schließlich ist es möglich, mit Wachstumsregulatoren zum Zeitpunkt der Ernte die zum Ablösen der Früchte erforderlichen Kräfte zu reduzieren, um eine mechanische Beerntung zu ermöglichen oder eine manuelle Beerntung zu erleichtern.

Mit Wachstumsregulatoren lässt sich ferner eine Beschleunigung oder auch Verzögerung der Reife des Erntegutes vor oder nach der Ernte erreichen. Dieses ist von besonderem Vorteil, weil sich dadurch eine optimale Anpassung an die Bedürfnisse des Marktes herbeiführen lässt. Weiterhin können Wachstumsregulatoren in manchen Fällen die Fruchtausfärbung verbessern. Darüber hinaus kann mit Wachstumsregulatoren auch eine zeitliche Konzentrierung der Reife erzielt werden. Damit werden die Voraussetzungen dafür geschaffen, dass z.B. bei Tabak, Tomaten oder Kaffee eine vollständige mechanische oder manuelle Beerntung in einem Arbeitsgang vorgenommen werden kann.

Durch Anwendung von Wachstumsregulatoren kann ferner die Samen- oder Knospenruhe der Pflanzen beeinflusst werden, so dass die Pflanzen, wie z.B. Ananas oder Zierpflanzen in Gärtnereien, zu einem Zeitpunkt keimen, austreiben oder blühen, an dem sie normalerweise hierzu keine Bereitschaft zeigen. Eine Verzögerung des Austriebes von Knospen oder der Keimung von Samen mit Hilfe von Wachstumsregulatoren kann in frostgefährdeten Gebieten erwünscht sein, um Schädigungen durch Spätfröste zu vermeiden.

Schließlich kann mit Wachstumsregulatoren eine Resistenz der Pflanzen gegen Frost, Trockenheit oder hohen Salzgehalt des Bodens induziert werden. Hierdurch wird die Kultivierung von Pflanzen in Gebieten möglich, die hierzu normalerweise ungeeignet sind.

Die aufgeführten Pflanzen können besonders vorteilhaft erfindungsgemäß mit den Verbindungen der allgemeinen Formel (I) bzw. den erfindungsgemäßen Mitteln behandelt werden. Die bei den Wirkstoffen bzw. Mitteln oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mitteln.

Die Erfindung wird durch die folgenden Beispiele veranschaulicht. Die Erfindung ist jedoch nicht auf die Beispiele limitiert.

### Beispiele

**Allgemeines:** Wenn nicht anders angegeben, werden alle chromatografischen Reinigungs- bzw. Trennungsschritte an Kieselgel und mit einem Lösungsmittelgradienten von 0:100 Essigsäureethylester/Cyclohexan zu 100:0 Essigsäure-ethylester/Cyclohexan durchgeführt.

### Herstellung von Verbindung I-1

### Schritt 1

### tert-Butyl-4-{4-[5-(2-hydroxy-4,5-dimethylphenyl)-5-methyl-4,5-dihydro-1,2-oxazol-3-yl]-1,3-thiazol-2-yl}piperidin-1-carboxylat

Zu einer Lösung von *tert*-Butyl-4-{4-[(hydroxyimino)methyl]-1,3-thiazol-2-yl}piperidin-1-carboxylat (400 mg) in Ethylacetat (6 mL) wurden N-Chlorsuccinimid (206 mg), 4,5-Dimethyl-2-(prop-1-en-2-yl)phenol (1320 mg, Reinheit: 50%), Kaliumhydrogencarbonat (643 mg) und anschließend ein Tropfen Wasser gegeben. Das Reaktionsgemisch wurde 40 Minuten lang unter Rückfluss gerührt. Die Reaktionsmischung wurde bei Raumtemperatur mit Ethylacetat und Wasser versetzt und mit Ethylacetat extrahiert. Die organischen Extrakte wurden über Natriumsulfat getrocknet und unter vermindertem Druck eingeengt. Der Rückstand wurde chromatographisch gereinigt. Man erhielt *tert*-Butyl-4-{4-[5-(2-hydroxy-4,5-dimethylphenyl)-5-methyl-4,5-dihydro-1,2-oxazol-3-yl]-1,3-thiazol-2-yl}piperidin-1-carboxylat (293 mg).
¹H NMR (DMSO-d₆): 9.34 (s, 1H), 7.94 (s, 1H), 7.12 (s, 1H), 6.63 (s, 1H), 4.05-3.95 (m, 2H), 3.52 (d, 1H), 3.48 (d, 1H), 3.25-3.15 (m, 1H), 2.88 (bs, 2H), 2.09 (s, 6H), 2.05-1.96 (m, 2H), 1.66 (s, 3H), 1.58-1.46 (m, 2H), 1.40 (s, 9H)

### Schritt 2

### tert-Butyl-4-(4-{5-[4,5-dimethyl-2-(prop-2-in-1-yloxy)phenyl]-5-methyl-4,5-dihydro-1,2-oxazol-3-yl}-1,3-thiazol-2-yl)piperidin-1-carboxylat (II-1)

Zu einer Lösung von *tert*-Butyl-4-{4-[5-(2-hydroxy-4,5-dimethylphenyl)-5-methyl-4,5-dihydro-1,2-oxazol-3-yl]-1,3-thiazol-2-yl}piperidin-1-carboxylat (150 mg) und Kaliumcarbonat (132 mg) in Aceton (3 mL) wurde bei Raumtemperatur 3-Bromprop-1-in (42 mg, 80%ig in Toluol) gegeben. Das Reaktionsgemisch wurde 6 Stunden lang bei 60 °C gerührt. Daraufhin wurde das Gemisch mit Wasser versetzt und mit Essigester extrahiert. Die vereinigten organischen Phasen wurden über Natriumsulfat getrocknet und eingeengt. Nach säulenchromatographischer Reinigung erhielt man *tert*-Butyl-4-(4-{5-[4,5-dimethyl-2-(prop-2-in-1-yloxy)phenyl]-5-methyl-4,5-dihydro-1,2-oxazol-3-yl}-1,3-thiazol-2-yl)piperidin-1-carboxylat (64 mg).
¹H NMR (DMSO-d₆): 7.92 (s, 1H), 7.22 (s, 1H), 6.95 (s, 1H), 4.87 (s, 2H), 4.05-3.95 (m, 2H), 3.55 (d, 1H), 3.46 (d, 1H), 3.30 (s, 1H), 3.27-3.17 (m, 1H), 2.88 (bs, 2H), 2.19 (s, 3H), 2.15 (s, 3H), 2.04-1.96 (m, 2H), 1.66 (s, 3H), 1.58-1.46 (m, 2H), 1.40 (s, 9H)

### Schritt 3

### 2-[3,5-Bis(difluormethyl)-1H-pyrazol-1-yl]-1-[4-(4-{5-[4,5-dimethyl-2-(prop-2-in-1-yloxy)phenyl]-5-methyl-4,5-dihydro-1,2-oxazol-3-yl}-1,3-thiazol-2-yl)piperidin-1-yl]ethanon (I-1)

Zu *tert*-Butyl-4-(4-{5-[4,5-dimethyl-2-(prop-2-in-1-yloxy)phenyl]-5-methyl-4,5-dihydro-1,2-oxazol-3-yl}-1,3-thiazol-2-yl)piperidin-1-carboxylat (64 mg) wurde bei Raumtemperatur eine 4-molare Lösung von Chlorwasserstoff (1.9 mL) in 1,4-Dioxan getropft. Das Lösungsmittel und überschüssiger Chlorwasserstoff wurden entfernt. Man erhielt 4-(4-{5-[4,5-Dimethyl-2-(prop-2-in-1-yloxy)phenyl]-5-methyl-4,5-dihydro-1,2-oxazol-3-yl}-1,3-thiazol-2-yl)piperidiniumchlorid.

Zu einer Lösung von [3,5-Bis-(difluormethyl)-1H-pyrazol-1-yl]essigsäure (30 mg) in Dichlormethan (20 mL) wurden bei 0 °C Oxalylchlorid (48 mg) und ein Tropfen N,N-Dimethylformamid gegeben. Das Reaktionsgemisch wurde bei Raumtemperatur für 10 Minuten gerührt. Das Lösungsmittel und das überschüssige Reagenz wurden unter vermindertem Druck entfernt. Der Rückstand wurde erneut in Dichlormethan gelöst und bei Raumtemperatur tropfenweise zu einer Lösung von 4-(4-{5-[4,5-Dimethyl-2-(prop-2-in-1-yloxy)phenyl]-5-methyl-4,5-dihydro-1,2-oxazol-3-yl}-1,3-thiazol-2-yl)piperidiniumchlorid und Triethylamin (38 mg) in Dichlormethan (5 mL) gegeben. Die Reaktionsmischung wurde bei Raumtemperatur gerührt. Daraufhin wurde sie mit konzentrierter Natriumhydrogencarbonatlösung versetzt, die wässrige Phase abgetrennt und mit Essigsäureethylester extrahiert. Die vereinigten organischen Phasen wurden über Natriumsulfat getrocknet und eingeengt. Nach säulenchromatographischer Reinigung erhielt man 2-[3,5-Bis(difluormethyl)-1H-pyrazol-1-yl]-1-[4-(4-{5-[4,5-dimethyl-2-(prop-2-in-1-yloxy)phenyl]-5-methyl-4,5-dihydro-1,2-oxazol-3-yl}-1,3-thiazol-2-yl)piperidin-1-yl]ethanon (53 mg).
¹H NMR (DMSO-d₆): δₚₚₘ : 7.94 (s, 1H), 7.23 (s, 1H), 7.18 (t, 1H), 7.03 (t, 1H), 6.96 (s, 1H), 6.91 (s, 1H), 5.43 (d, 1H), 5.35 (d, 1H), 4.87 (s, 2H), 4.38-4.30 (m, 1H), 4.00-3.92 (m, 1H), 3.72-3.63 (m, 1H), 3.60-3.44 (m, 2H), 3.40-3.20 (m, 2H), 2.86-2.78 (m, 1H), 2.19 (s, 3H), 2.15 (s, 3H), 2.13-2.00 (m, 2H), 1.82-1.70 (m, 1H), 1.67 (s, 3H), 1.60-1.58 (m, 1H)

### Herstellung von Verbindung I-2

### Schritt 1

### tert-Butyl-4-{4-[5-(2,6-difluor-3-hydroxyphenyl)-4,5-dihydro-1,2-oxazol-3-yl]-1,3-thiazol-2-yl}piperidin-1-carboxylat

Zu einer Lösung von *tert*-Butyl-4-{4-[(hydroxyimino)methyl]-1,3-thiazol-2-yl}piperidin-1-carboxylat (1.278 g) in Ethylacetat (80 mL) wurde N-Chlorsuccinimid (658 mg) gegeben. Das Reaktionsgemisch wurde 30 Minuten lang unter Rückfluss gerührt. Zu dem Reaktionsgemisch wurden bei Raumtemperatur 2,4-Difluor-3-vinylphenol (705 mg) und Kaliumhydrogencarbonat (822 mg) gegeben und anschließend ein Tropfen Wasser. Nach Rühren über Nacht bei Raumtemperatur wurde die Reaktionsmischung mit Ethylacetat und Wasser versetzt und mit Ethylacetat extrahiert. Die organischen Extrakte wurden über Natriumsulfat getrocknet und unter vermindertem Druck eingeengt. Der Rückstand wurde chromatographisch gereinigt. Man erhielt tert-Butyl-4-{4-[5-(2,6-difluor-3-hydroxyphenyl)-4,5-dihydro-1,2-oxazol-3-yl]-1,3-thiazol-2-yl}piperidin-1-carboxylat (680 mg).
¹H NMR (DMSO-d₆): 9.92 (s, 1H), 8.01 (s, 1H), 7.02-6.90 (m, 2H), 5.96 (dd, 1H), 4.02 (d, 2H), 3.88 (dd, 1H), 3.51 (dd, 1H), 2.90 (bs, 1H), 2.10-2.00 (m, 2H), 1.65-1.50 (m, 2H), 1.42 (s, 9H)

### Schritt 2

### 2-[3,5-Bis(difluormethyl)-1H-pyrazol-1-yl]-1-(4-{4-[5-(2,6-difluor-3-hydroxyphenyl)-4,5-dihydro-1,2-oxazol-3-yl]-1,3-thiazol-2-yl}piperidin-1-yl)ethanon

Zu einer Lösung von *tert*-Butyl-4-{4-[5-(2,6-difluor-3-hydroxyphenyl)-4,5-dihydro-1,2-oxazol-3-yl]-1,3-thiazol-2-yl}piperidin-1-carboxylat (374 mg) in Dichlormethan wurde bei 0 °C eine 4-molare Lösung von Chlorwasserstoff (4.0 Äq.) in 1,4-Dioxan getropft. Das Reaktionsgemisch wurde bei 0 °C gerührt und dann langsam auf Raumtemperatur erwärmt. Nach Rühren über 5 Stunden wurden das Lösungsmittel und überschüssiger Chlorwasserstoff entfernt. Man erhielt 4-{4-[5-(2,6-Difluor-3-hydroxyphenyl)-4,5-dihydro-1,2-oxazol-3-yl]-1,3-thiazol-2-yl}piperidiniumchlorid.

Zu einer Lösung von [3,5-Bis-(difluormethyl)-1H-pyrazol-1-yl]essigsäure (160 mg) in Dichlormethan (20 mL) wurden bei 0 °C Oxalylchlorid (275 mg) und ein Tropfen N,N-Dimethylformamid gegeben. Das Reaktionsgemisch wurde bei Raumtemperatur für 2 Stunden gerührt. Das Lösungsmittel und das überschüssige Reagenz wurden unter vermindertem Druck entfernt. Der feste Rückstand wurde erneut in Dichlormethan gelöst und bei 0 °C tropfenweise zu einer Lösung von 4-{4-[5-(2,6-Difluor-3-hydroxyphenyl)-4,5-dihydro-1,2-oxazol-3-yl]-1,3-thiazol-2-yl}piperidiniumchlorid und Triethylamin (10 Äq.) in Dichlormethan (25 mL) gegeben. Die Reaktionsmischung wurde über Nacht bei Raumtemperatur gerührt. Daraufhin wurde sie mit konzentrierter Natriumhydrogencarbonatlösung versetzt, die wässrige Phase abgetrennt und mit Essigsäureethylester extrahiert. Die vereinigten organischen Phasen wurden über Natriumsulfat getrocknet und eingeengt. Nach säulenchromatographischer Reinigung erhielt man 2-[3,5-Bis(difluormethyl)-1H-pyrazol-1-yl]-1-(4-{4-[5-(2,6-difluor-3-hydroxyphenyl)-4,5-dihydro-1,2-oxazol-3-yl]-1,3-thiazol-2-yl}piperidin-1-yl)ethanon (177 mg).
¹H NMR (DMSO-d₆): 9.91 (s, 1H), 8.03 (s, 1H), 7.18 (t, 1H), 7.03 (t, 1H), 7.01-6.88 (m, 3H), 5.96 (dd, 1H), 5.38 (q, 2H), 4.35 (d, 1H), 4.02-3.83 (m, 2H), 3.51 (dd, 1H), 3.45-3.21 (m, 2H), 2.84 (t, 1H), 2.11 (t, 2H), 1.88-1.75 (m, 1H), 1.65-1.51 (m, 1H)

### Schritt 3

### 2-[3,5-Bis(difluormethyl)-1H-pyrazol-1-yl]-1-[4-(4-{5-[2,6-difluor-3-(prop-2-in-1-yloxy)phenyl]-4,5-dihydro-1,2-oxazol-3-yl}-1,3-thiazol-2-yl)piperidin-1-yl]ethanon (I-2)

Zu einer Lösung von 2-[3,5-Bis(difluormethyl)-1H-pyrazol-1-yl]-1-(4-{4-[5-(2,6-difluor-3-hydroxyphenyl)-4,5-dihydro-1,2-oxazol-3-yl]-1,3-thiazol-2-yl}piperidin-1-yl)ethanon (85 mg) und Kaliumcarbonat (31 mg) in N,N-Dimethylformamid (10 mL) wurden bei Raumtemperatur Kaliumiodid (13.5 mg) und 3-Bromprop-1-in (35 mg (80%ig in Toluol)) gegeben. Das Reaktionsgemisch wurde 4 Stunden lang bei 80 °C gerührt. Daraufhin wurde das Gemisch mit Wasser versetzt und mit Essigester extrahiert. Die vereinigten organischen Phasen wurden über Natriumsulfat getrocknet und eingeengt. Nach säulenchromatographischer Reinigung erhielt man 2-[3,5-Bis(difluormethyl)-1H-pyrazol-1-yl]-1-[4-(4-{5-[2,6-difluor-3-(prop-2-in-1-yloxy)phenyl]-4,5-dihydro-1,2-oxazol-3-yl}-1,3-thiazol-2-yl)piperidin-1-yl]ethanon (60 mg).
¹H NMR (DMSO-d₆): δₚₚₘ : 8.03 (s, 1H), 7.38-7.26 (m, 1H), 7.18 (t, 1H), 7.20- 7.10 (m, 1H), 7.03 (t, 1H), 6.91 (s, 1H), 5.99 (dd, 1H), 5.40 (q, 2H), 4.89 (d, 2H), 4.35 (d, 1H), 4.03-3.75 (m, 2H), 3.64 (t, 1H), 3.54 (dd, 1H), 3.47-3.20 (m, 2H), 2.84 (t, 1H), 2.12 (t, 2H), 1.88-1.74 (m, 1H), 1.65-1.50 (m, 1H)

### Herstellung von Verbindung I-4

### 1-[4-(4-{5-[3-(Allyloxy)-2,6-difluorphenyl]-4,5-dihydro-1,2-oxazol-3-yl}-1,3-thiazol-2-yl)piperidin-1-yl]-2-[3,5-bis(difluormethyl)-1H-pyrazol-1-yl]ethanon (I-4)

Zu einer Lösung von 2-[3,5-Bis(difluormethyl)-1H-pyrazol-1-yl]-1-(4-{4-[5-(2,6-difluor-3-hydroxyphenyl)-4,5-dihydro-1,2-oxazol-3-yl]-1,3-thiazol-2-yl}piperidin-1-yl)ethanon (85 mg) und Kaliumcarbonat (102 mg) in Aceton (5 mL) wurde bei Raumtemperatur Allylbromid (72 mg) gegeben. Das Reaktionsgemisch wurde 5 Stunden lang bei Rückfluss gerührt. Daraufhin wurde das Gemisch mit Wasser versetzt und mit Essigester extrahiert. Die vereinigten organischen Phasen wurden über Natriumsulfat getrocknet und eingeengt. Nach säulenchromatographischer Reinigung erhielt man -[4-(4-{5-[3-(Allyloxy)-2,6-difluorphenyl]-4,5-dihydro-1,2-oxazol-3-yl}-1,3-thiazol-2-yl)piperidin-1-yl]-2-[3,5-bis(difluormethyl)-1H-pyrazol-1-yl]ethanon (55 mg).
¹H NMR (DMSO-d₆): 8.03 (s, 1H), 7.29-7.20 (m, 1H), 7.18 (t, 1H), 7.13-7.03 (m, 1H), 7.03 (t, 1H), 6.91 (s, 1H), 6.08-5.95 (m, 2H), 5.50-5.24 (m, 4H), 4.63 (dt, 2H), 4.35 (d, 1H), 4.05-3.85 (m, 2H), 3.53 (dd, 1H), 3.45-3.20 (m, 2H), 2.84 (t, 1H), 2.12 (t, 2H), 1.87-1.75 (m, 1H), 1.65-1.50 (m, 1H)

### Herstellung von Verbindung I-5

### Schritt 1

### tert-Butyl-4-{4-[5-(4-amino-2,6-difluorphenyl)-4,5-dihydro-1,2-oxazol-3-yl]-1,3-thiazol-2-yl}piperidin-1-carboxylat

Zu einer Lösung von *tert*-Butyl-4-{4-[(hydroxyimino)methyl]-1,3-thiazol-2-yl}piperidin-1-carboxylat (500 mg) in N,N-Dimethylformamid (2 mL) wurde N-Chlorsuccinimid (257 mg) gegeben. Das Reaktionsgemisch wurde bei 50 °C für 1 Stunde gerührt. Zu dem Reaktionsgemisch wurden bei Raumtemperatur 3,5-Difluor-4-vinylanilin (324 mg) und Triethylamin (0.67 mL) gegeben. Nach Rühren für 2 Stunden bei 50 °C wurde die Reaktionsmischung mit Ethylacetat und Wasser versetzt und mit Ethylacetat extrahiert. Die organischen Extrakte wurden über Natriumsulfat getrocknet und unter vermindertem Druck eingeengt. Der Rückstand wurde chromatographisch gereinigt. Man erhielt *tert*-Butyl-4-{4-[5-(4-amino-2,6-difluorphenyl)-4,5-dihydro-1,2-oxazol-3-yl]-1,3-thiazol-2-yl}piperidin-1-carboxylat (400 mg).
¹H NMR (DMSO-d₆): 7.95 (s, 1H), 6.21 (d, 2H), 5.89 (s, 2H), 5.79 (dd, 1H), 4.05-3.97 (m, 2H), 3.73 (dd, 1H), 3.39 (dd, 1H), 3.30-3.20 (m, 1H), 2.89 (bs, 2H), 2.08-2.00 (m, 2H), 1.62-1.49 (m, 2H), 1.41 (s, 9H)

### Schritt 2

### tert-Butyl-4-[4-(5-{2,6-difluor-4-[(methylsulfonyl)amino]phenyl}-4,5-dihydro-1,2-oxazol-3-yl)-1,3-thiazol-2-yl]piperidin-1-carboxylat (II-2)

Zu einer Lösung von *tert*-Butyl-4-{4-[5-(4-amino-2,6-difluorphenyl)-4,5-dihydro-1,2-oxazol-3-yl]-1,3-thiazol-2-yl}piperidin-1-carboxylat (400 mg) in Dichlormethan (10 mL) wurden Methansulfonylchlorid (109 mg) und Pyridin (82 mg) gegeben. Das Reaktionsgemisch wurde über Nacht bei Raumtemperatur gerührt. Zu dem Reaktionsgemisch wurden bei Raumtemperatur Pyridin (82 mg) und Methansulfonylchlorid (109 mg) wieder gegeben. Nach Rühren für 2 Stunden bei Raumtemperatur wurde die Reaktionsmischung mit 1N HCl Lösung versetzt und mit Dichlormethan extrahiert. Die organischen Extrakte wurden über Natriumsulfat getrocknet und unter vermindertem Druck eingeengt. Der Rückstand wurde chromatographisch gereinigt. Man erhielt *tert-*Butyl-4-[4-(5-{2,6-difluor-4-[(methylsulfonyl)amino]phenyl}-4,5-dihydro-1,2-oxazol-3-yl)-1,3-thiazol-2-yl]piperidin-1-carboxylat (380 mg).
¹H NMR (DMSO-d₆): 7.99 (s, 1H), 6.89 (d, 2H), 5.93 (dd, 1H), 4.05-3.97 (m, 2H), 3.75 (dd, 1H), 3.49 (dd, 1H), 3.30-3.20 (m, 1H), 3.14 (s, 3H), 2.89 (bs, 2H), 2.08-2.00 (m, 2H), 1.62-1.50 (m, 2H), 1.41 (s, 9H)

### Schritt 3

### 4-[4-(5-{2,6-Difluor-4-[(methylsulfonyl)amino]phenyl}-4,5-dihydro-1,2-oxazol-3-yl)-1,3-thiazol-2-yl]piperidiniumchlorid (III-1)

Zu *tert*-Butyl-4-[4-(5-{2,6-difluor-4-[(methylsulfonyl)amino]phenyl}-4,5-dihydro-1,2-oxazol-3-yl)-1,3-thiazol-2-yl]piperidin-1-carboxylat (380 mg) wurde bei Raumtemperatur eine 4-molare Lösung von Chlorwasserstoff (2.6 mL) in 1,4-Dioxan getropft. Das Lösungsmittel und überschüssiger Chlorwasserstoff wurden entfernt. Man erhielt 4-[4-(5-{2,6-Difluor-4-[(methylsulfonyl)amino]phenyl}-4,5-dihydro-1,2-oxazol-3-yl)-1,3-thiazol-2-yl]piperidiniumchlorid (380 mg).
¹H NMR (DMSO-d₆): 9.19 (bs, 1H), 8.94 (bs, 1H), 8.04 (s, 1H), 6.92 (d, 2H), 5.94 (dd, 1H), 3.90-3.70 (m, 2H), 3.55-3.30 (m, 4H), 3.13 (s, 3H), 3.08-2.97 (m, 2H), 2.25-2.17 (m, 2H), 2.02-1.90 (m, 2H)

### Schritt 4

### N-(4-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3,5-difluorphenyl)methansulfonamid (I-5)

[3,5-Bis-(difluormethyl)-1H-pyrazol-1-yl]essigsäure (180 mg) und Triethylamin (242 mg) wurden in Dichlormethan (10 mL) gelöst und für 10 min gerührt. 4-[4-(5-{2,6-Difluor-4-[(methylsulfonyl)amino]phenyl}-4,5-dihydro-1,2-oxazol-3-yl)-1,3-thiazol-2-yl]piperidiniumchlorid (380 mg) und Brom-tris-pyrrolidino-phosphoniumhexafluorophosphat (445 mg) wurden zugegeben und die Reaktionsmischung wurde für 2 Stunden bei Raumtemperatur gerührt. Daraufhin wurde sie mit konzentrierter Natriumhydrogencarbonatlösung versetzt, die wässrige Phase abgetrennt und mit Essigsäureethylester extrahiert. Die vereinigten organischen Phasen wurden über Natriumsulfat getrocknet und eingeengt. Nach säulenchromatographischer Reinigung erhielt man N-(4-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3,5-difluorphenyl)methansulfonamid (255 mg).
¹H NMR (DMSO-d₆): 8.02 (s, 1H), 7.18 (t, 1H), 7.02 (t, 1H), 6.93-6.86 (m, 4H), 5.93 (dd, 1H), 5.43 (d, 1H), 5.35 (d, 1H), 4.40-4.32 (m, 1H), 4.01-3.95 (m, 1H), 3.83 (dd, 1H), 3.50 (dd, 1H), 3.45-3.35 (m, 1H), 3.35-3.24 (m, 1H), 3.14 (s, 3H), 2.89-2.80 (m, 1H), 2.16-2.05 (m, 2H), 1.86-1.75 (m, 1H), 1.63-1.50 (m, 1H)

### Herstellung von Verbindung XIV-1

### [3,5-Bis(difluormethyl)-1H-pyrazol-1-yl]acetylfluorid (XIV-1)

Zu einer Lösung von [3,5-Bis(difluormethyl)-1H-pyrazol-1-yl]essigsäure (1,0 g) und Pyridin (0,35 g) in Dichlormethan (10 mL) wurde bei 0°C 2,4,6-Trifluor-1,3,5-triazin (0,60 g) gegeben. Nach Rühren über Nacht bei 20 °C wurde die Reaktionsmischung mit Cyclohexan (10 mL) versetzt und filtriert. Das Filtrat wurde unter vermindertem Druck eingeengt. Man erhielt [3,5-Bis(difluormethyl)-1H-pyrazol-1-yl]acetylfluorid (1,1 g).
¹H NMR (600 MHz, CDCl₃): δₚₚₘ : 6.80 (s, 1H), 6.79 (t, 1H, J = 53.6 Hz), 6.68 (t, 1H, J = 54.7 Hz), 5.28 (d, 2H, J = 4.3 Hz).
¹⁹F NMR (566 MHz, CDCl₃): δₚₚₘ : 32.7 (t, J = 4.5 Hz), -113,3 (d, J = 53.3 Hz), -113,5 (d, J = 54.6 Hz).

Folgende Verbindungen können mit einem oder mehreren der oben genannten Verfahren hergestellt werden:

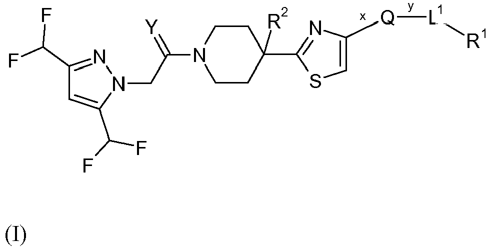

Das in Tablle 1 aufgelistetete Strukturelement Q-3 ist wie folgt definiert:

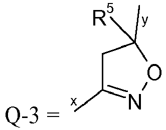

**Tabelle 1**

| Für alle in Tabelle 1 aufgeführten Verbindungen steht L¹ für eine direkte Bindung. | | | | | | |
|---|---|---|---|---|---|---|
| **Bsp.** | **Y** | **R²** | **Q** | **R⁵** | **R¹** | **LogP** |
| I-1 | O | H | Q- | CH₃ | 4,5-dimethyl-2-(prop-2-yn-1-yloxy)phenyl | 4,1^{[a]}; 4,04^{[b]} |
| I-2 | O | H | Q- | H | 2,6-difluoro-3-(prop-2-yn-1-yloxy)phenyl | 3,26^{[a]}; 3,18^{[b]} |
| I-3 | O | H | Q- | H | 2,6-difluoro-4-(prop-2-yn-1-yloxy)phenyl | 3,3^{[a]}; 3,26^{[b]} |
| I-4 | O | H | Q- | H | 3-(allyloxy)-2,6-difluorophenyl | 3,59^{[a]}; 3,51^{[b]} |
| I-5 | O | H | Q- | H | 2,6-difluoro-4-[(methylsulfonyl)amino]phenyl | 2,63^{[a]} |
| I-6 | O | H | Q- | H | 6-{[(2Z)-3-chloroprop-2-en-1-yl]oxy}-2,3-difluorophenyl | 3,79^{[a]}; 3,71^{[b]} |
| I-7 | O | H | Q- | H | 6-[(2-chloroprop-2-en-1-yl)oxy]-2,3-difluorophenyl | 3,68^{[a]}; 3,69^{[b]} |
| I-8 | O | H | Q- | H | 2,3-difluoro-6-(prop-2-yn-1-yloxy)phenyl | 3,3^{[a]}; 3,27^{[b]} |
| I-9 | O | H | Q- | H | 4-[(cyclopropylcarbonyl)oxy]-2,6-difluorophenyl | 3,53^{[a]} |
| I-10 | O | H | Q- | H | 3,5-difluoro-2-(prop-2-yn-1-yloxy)phenyl | 3,58^{[a]}; 3,51^{[b]} |
| I-11 | O | H | Q- | H | 4-(allyloxy)-2,6-difluorophenyl | 3,7^{[a]} |
| I-12 | O | H | Q- | H | 3,6-difluoro-2-(prop-2-yn-1-yloxy)phenyl | 3,31^{[a]}; 3,3^{[b]} |
| I-13 | O | H | Q- | H | 2,4-difluoro-6-(prop-2-yn-1-yloxy)phenyl | 3,32^{[a]} |
| I-14 | O | H | Q- | H | 2,6-difluoro-4-formylphenyl | 2,93^{[a]}; 2,87^{[b]} |
| I-15 | O | H | Q- | H | 2-(cyclopropylmethoxy)-4,6-difluorophenyl | 3,81^{[a]}; 3,76^{[b]} |
| I-16 | O | H | Q- | H | 2-[(cyclopropylcarbonyl)oxy]-4,6-difluorophenyl | 3,5^{[a]}; 3,42^{[b]} |
| I-17 | O | H | Q- | H | 6-(allyloxy)-2,3-difluorophenyl | 3,53^{[a]}; 3,56^{[b]} |
| I-18 | O | H | Q- | H | 2-(allyloxy)-4,6-dichlorophenyl | 4,29^{[a]}; 4,2^{[b]} |
| I-19 | O | H | Q- | H | 2-[(2-chloroprop-2-en-1-yl)oxy]-4,6-difluorophenyl | 3,79^{[a]}; 3,7^{[b]} |
| I-20 | O | H | Q- | H | 2-(allyloxy)-4,6-difluorophenyl | 3,67^{[a]}; 3,58^{[b]} |
| I-21 | O | H | Q- | H | 2,4-dichloro-6-(prop-2-yn-1-yloxy)phenyl | 3,94^{[a]}; 3,85^{[b]} |

Die Bestimmung der logP Werte erfolgte gemäß EEC Directive 79/831 Annex V.A8 durch HPLC (High Performance Liquid Chromatography) an reversed-phase Säulen (C 18), mit nachfolgenden Methoden:
^{[a]} Die Bestimmung mit der LC-MS im sauren Bereich erfolgt bei pH 2,7 mit 0,1 % wässriger Ameisensäure und Acetonitril (enthält 0,1% Ameisensäure) als Eluenten; linearer Gradient von 10% Acetonitril bis 95% Acetonitril
^{[b]} Die Bestimmung mit der LC-MS im neutralen Bereich erfolgt bei pH 7.8 mit 0,001 molarer wässriger Ammoniumhydrogencarbonat-Lösung und Acetonitril als Eluenten; linearer Gradient von 10 % Acetonitril bis 95 % Acetonitril.

Die Eichung erfolgt mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinander folgenden Alkanonen).

Die lambda-maX Werte wurden an Hand der UV-Spektren von 200 nm bis 400 nm in den Maxima der chromatographischen Signale ermittelt.

### NMR-Daten ausgewählter Beispiele

### NMR-Peak-Listenverfahren

Die 1H-NMR-Daten der Beispiele I-2 bis I-21 sind in Form von ¹H-NMR-Peaklisten notiert. Zu jedem Signalpeak sind der δ-Wert in ppm und die Signalintensität in Klammern aufgeführt:

| **Beispiel I-2, Lösungsmittel: DMSO-d₆, Spektrometer: 399,95 MHz** |
|---|
| 8.0321 (10.80); 7.3369 (0.68); 7.3244 (0.70); 7.3139 (2.93); 7.3007 (1.44); 7.2901 (0.83); 7.2775 (0.83); 7.1806 (3.49); 7.1669 (1.70); 7.1516 (0.99); 7.1269 (1.61); 7.1079 (0.75); 7.1032 (0.78); 7.0475 (1.68); 7.0309 (4.13); 6.9059 (3.38); 6.8950 (2.03); 6.0178 (1.09); 5.9964 (1.30); 5.9872 (1.32); 5.9660 (1.19); 5.7554 (16.00); 5.4601 (0.75); 5.4189 (2.59); 5.3772 (2.57); 5.3351 (0.73); 4.8979 (7.50); 4.8919 (7.68); 4.3728 (0.77); 4.3379 (0.85); 4.0558 (0.51); 4.0379 (1.27); 4.0201 (1.38); 4.0025 (0.71); 3.9879 (0.70); 3.9553 (0.82); 3.9404 (0.94); 3.9099 (0.97); 3.8963 (1.14); 3.8672 (0.97); 3.6460 (2.31); 3.6401 (5.32); 3.6342 (2.33); 3.5740 (1.23); 3.5530 (1.15); 3.5309 (0.98); 3.5097 (1.00); 3.4298 (0.59); 3.4104 (0.63); 3.4014 (1.14); 3.3913 (0.72); 3.3821 (0.52); 3.3724 (0.63); 3.3642 (0.52); 3.3217 (441.54); 3.2982 (2.78); 3.2716 (1.08); 3.2428 (0.61); 2.8745 (0.55); 2.8477 (0.91); 2.8146 (0.55); 2.6748 (0.82); 2.6700 (1.07); 2.6655 (0.76); 2.5405 (0.58); 2.5236 (1.67); 2.5190 (2.60); 2.5055 (115.26); 2.5013 (157.77); 2.4975 (107.29); 2.3326 (0.84); 2.3278 (1.12); 2.3233 (0.82); 2.3191 (0.43); 2.1488 (0.67); 2.1166 (1.32); 2.0734 (1.55); 1.9884 (6.00); 1.8249 (0.63); 1.8037 (0.58); 1.5985 (0.66); 1.5885 (0.60); 1.5672 (0.59); 1.5566 (0.60); 1.2350 (0.58); 1.1922 (1.60); 1.1744 (3.24); 1.1567 (1.56); 0.0079 (0.44); -0.0002 (14.13); -0.0084 (0.43) |

| **Beispiel I-3, Lösungsmittel: DMSO-d₆, Spektrometer: 399,95 MHz** |
|---|
| 8.0257 (0.56); 8.0121 (14.18); 7.3097 (2.42); 7.1764 (5.60); 7.1594 (2.69); 7.0432 (2.77); 7.0234 (6.20); 6.9003 (5.53); 6.8875 (3.27); 6.8811 (0.99); 6.8646 (6.62); 6.8384 (6.44); 5.9498 (1.71); 5.9284 (2.08); 5.9200 (1.97); 5.8983 (1.73); 5.4549 (1.09); 5.4121 (4.09); 5.3707 (4.02); 5.3284 (1.08); 4.8880 (11.02); 4.8821 (10.88); 4.3687 (1.26); 4.3370 (1.29); 3.9899 (1.20); 3.9563 (1.33); 3.8847 (1.37); 3.8543 (1.59); 3.8417 (1.88); 3.8114 (1.63); 3.6502 (3.01); 3.6443 (6.39); 3.6384 (2.86); 3.5314 (2.07); 3.5099 (2.15); 3.4880 (1.76); 3.4667 (1.80); 3.4363 (0.86); 3.4267 (1.28); 3.4173 (1.10); 3.4074 (1.56); 3.3983 (2.36); 3.3891 (1.73); 3.3782 (1.53); 3.3699 (2.00); 3.3592 (2.01); 3.3112 (736.95); 3.2456 (1.13); 2.8770 (0.87); 2.8500 (1.54); 2.8200 (0.87); 2.6741 (0.41); 2.6696 (0.54); 2.6654 (0.38); 2.5395 (0.76); 2.5094 (30.86); 2.5050 (57.09); 2.5006 (74.10); 2.4962 (51.38); 2.4918 (24.73); 2.3315 (0.34); 2.3272 (0.49); 2.3229 (0.36); 2.1495 (1.13); 2.1167 (2.29); 2.0808 (1.54); 2.0691 (16.00); 1.8528 (0.45); 1.8236 (1.01); 1.8000 (0.93); 1.7702 (0.38); 1.7632 (0.34); 1.6214 (0.47); 1.5997 (0.97); 1.5913 (1.01); 1.5709 (0.96); 1.5611 (0.92); 1.5416 (0.37); 0.0078 (0.32); -0.0002 (6.83) |

| **Beispiel I-4, Lösungsmittel: DMSO-d₆, Spektrometer: 399,95 MHz** |
|---|
| 8.0312 (10.33); 7.3143 (1.44); 7.2692 (0.57); 7.2561 (0.63); 7.2458 (1.27); 7.2329 (1.25); 7.2224 (0.78); 7.2094 (0.71); 7.1810 (3.32); 7.1672 (1.60); 7.1051 (0.87); 7.1005 (0.94); 7.0802 (1.40); 7.0766 (1.41); 7.0572 (0.73); 7.0524 (0.84); 7.0479 (1.67); 7.0312 (3.91); 6.9064 (3.12); 6.8954 (1.86); 6.0697 (0.49); 6.0563 (1.11); 6.0433 (1.03); 6.0301 (1.28); 6.0266 (0.69); 6.0133 (2.08); 6.0000 (1.33); 5.9942 (1.33); 5.9867 (2.19); 5.9736 (0.69); 5.9638 (1.12); 5.7558 (1.84); 5.4616 (0.69); 5.4271 (0.99); 5.4229 (3.18); 5.4189 (4.51); 5.3797 (3.63); 5.3758 (3.65); 5.3350 (0.69); 5.2961 (0.77); 5.2927 (1.94); 5.2887 (1.84); 5.2852 (0.76); 5.2699 (0.71); 5.2664 (1.77); 5.2623 (1.75); 5.2589 (0.70); 4.6453 (2.25); 4.6418 (3.99); 4.6381 (2.46); 4.6320 (2.37); 4.6284 (3.90); 4.6248 (2.29); 4.3719 (0.65); 4.3384 (0.68); 4.0558 (1.12); 4.0380 (3.54); 4.0202 (3.59); 4.0024 (1.44); 3.9916 (0.61); 3.9562 (0.68); 3.9382 (0.84); 3.9073 (0.86); 3.8947 (1.02); 3.8642 (0.88); 3.5690 (1.09); 3.5475 (1.13); 3.5255 (0.88); 3.5047 (0.91); 3.4294 (0.49); 3.4199 (0.33); 3.4101 (0.57); 3.4006 (1.03); 3.3915 (0.59); 3.3812 (0.37); 3.3717 (0.54); 3.3202 (94.97); 3.2965 (1.30); 3.2724 (0.86); 3.2432 (0.46); 2.8725 (0.46); 2.8451 (0.80); 2.8157 (0.47); 2.6704 (0.37); 2.5236 (0.61); 2.5055 (38.05); 2.5014 (52.03); 2.4980 (35.04); 2.3283 (0.36); 2.1482 (0.62); 2.1156 (1.18); 2.0738 (1.25); 1.9885 (16.00); 1.8250 (0.57); 1.8014 (0.47); 1.5985 (0.51); 1.5902 (0.56); 1.5692 (0.49); 1.5590 (0.49); 1.3975 (0.53); 1.1923 (4.37); 1.1745 (8.84); 1.1567 (4.26); -0.0002 (7.40) |

| **Beispiel I-5, Lösungsmittel: DMSO-d₆, Spektrometer: 399,95 MHz** |
|---|
| 10.3937 (2.73); 8.0149 (6.46); 7.3095 (1.10); 7.1762 (2.52); 7.1592 (1.22); 7.0429 (1.25); 7.0232 (2.80); 7.0162 (0.51); 6.9086 (3.56); 6.9007 (3.11); 6.8869 (2.75); 6.8831 (3.38); 5.9564 (0.78); 5.9351 (0.93); 5.9266 (0.88); 5.9049 (0.77); 5.7461 (8.38); 5.4548 (0.51); 5.4119 (1.88); 5.3707 (1.85); 5.3286 (0.49); 5.2173 (0.66); 4.3698 (0.58); 4.3367 (0.63); 4.0394 (0.49); 4.0215 (0.51); 4.0036 (0.40); 3.9908 (0.58); 3.9571 (0.63); 3.8970 (0.62); 3.8666 (0.73); 3.8538 (0.83); 3.8235 (0.73); 3.6198 (0.46); 3.5323 (0.94); 3.5226 (0.46); 3.5106 (1.09); 3.4889 (1.05); 3.4768 (0.42); 3.4748 (0.43); 3.4670 (0.89); 3.4256 (0.57); 3.4153 (0.49); 3.4058 (0.71); 3.3971 (1.06); 3.3875 (0.76); 3.3777 (0.67); 3.3683 (0.86); 3.3590 (0.78); 3.3090 (218.66); 3.2819 (1.10); 3.2761 (1.07); 3.2468 (0.52); 3.1407 (16.00); 2.8775 (0.41); 2.8494 (0.74); 2.8194 (0.41); 2.5396 (0.62); 2.5226 (1.59); 2.5093 (17.23); 2.5050 (31.09); 2.5006 (39.79); 2.4961 (27.48); 2.4918 (13.07); 2.1486 (0.52); 2.1151 (1.07); 2.0790 (0.63); 2.0693 (0.52); 1.9868 (1.93); 1.8278 (0.50); 1.8134 (0.48); 1.7954 (0.68); 1.5986 (0.46); 1.5916 (0.47); 1.5698 (0.45); 1.5604 (0.43); 1.1928 (0.54); 1.1750 (1.06); 1.1572 (0.53); -0.0002 (0.95) |

| **Beispiel I-6, Lösungsmittel: DMSO-d₆, Spektrometer: 399,95 MHz** |
|---|
| 7.9865 (12.73); 7.4770 (0.88); 7.4536 (2.07); 7.4284 (2.11); 7.4049 (0.93); 7.3138 (1.89); 7.1805 (4.51); 7.1668 (2.14); 7.0473 (2.15); 7.0308 (5.13); 6.9673 (1.06); 6.9630 (1.28); 6.9583 (1.21); 6.9539 (1.18); 6.9434 (1.04); 6.9395 (1.23); 6.9348 (1.17); 6.9312 (1.07); 6.9067 (4.30); 6.8950 (2.49); 6.4336 (1.25); 6.4298 (2.53); 6.4261 (1.30); 6.4156 (1.38); 6.4118 (2.74); 6.4081 (1.39); 6.0897 (1.48); 6.0692 (1.75); 6.0590 (1.68); 6.0383 (1.56); 6.0267 (1.22); 6.0116 (2.85); 5.9938 (2.68); 5.9788 (1.20); 5.7560 (16.00); 5.4656 (1.01); 5.4230 (3.22); 5.3782 (3.16); 5.3354 (1.00); 4.7347 (4.07); 4.7316 (4.12); 4.7169 (4.02); 4.3738 (0.89); 4.3407 (0.94); 4.0378 (0.69); 4.0200 (0.72); 4.0020 (0.67); 3.9922 (0.83); 3.9580 (0.92); 3.8464 (1.14); 3.8155 (1.36); 3.8037 (1.67); 3.7729 (1.44); 3.5679 (0.36); 3.5502 (1.68); 3.5296 (1.71); 3.5072 (1.32); 3.4868 (1.33); 3.4275 (0.62); 3.4180 (0.45); 3.4081 (0.77); 3.3988 (1.40); 3.3895 (0.78); 3.3792 (0.48); 3.3700 (0.73); 3.3609 (0.37); 3.3213 (83.38); 3.2737 (1.22); 3.2451 (0.69); 2.8745 (0.62); 2.8437 (1.12); 2.8171 (0.63); 2.6751 (0.50); 2.6706 (0.74); 2.6660 (0.51); 2.5240 (1.60); 2.5192 (2.57); 2.5106 (41.11); 2.5060 (85.13); 2.5014 (115.73); 2.4968 (85.95); 2.4923 (41.91); 2.3328 (0.61); 2.3282 (0.83); 2.3236 (0.62); 2.3192 (0.32); 2.1487 (0.80); 2.1153 (1.65); 2.0803 (0.94); 1.9886 (3.13); 1.8525 (0.36); 1.8236 (0.73); 1.7978 (0.66); 1.6205 (0.39); 1.6002 (0.74); 1.5907 (0.81); 1.5690 (0.76); 1.5597 (0.72); 1.3357 (0.59); 1.2495 (0.76); 1.1926 (0.85); 1.1747 (1.66); 1.1569 (0.83); 0.0080 (0.64); -0.0002 (22.05); -0.0085 (0.77) |

| **Beispiel I-7, Lösungsmittel: DMSO-d₆, Spektrometer: 399,95 MHz** |
|---|
| 8.3197 (0.65); 7.9856 (16.00); 7.4879 (1.28); 7.4644 (3.23); 7.4397 (3.13); 7.4160 (1.28); 7.3159 (2.66); 7.1826 (6.57); 7.1704 (3.06); 7.0494 (3.14); 7.0344 (6.91); 6.9987 (2.18); 6.9945 (2.47); 6.9900 (2.30); 6.9858 (2.03); 6.9749 (2.08); 6.9710 (2.29); 6.9668 (2.05); 6.9098 (7.03); 6.8986 (3.50); 6.1198 (2.17); 6.0971 (2.92); 6.0893 (2.52); 6.0669 (2.22); 5.7601 (15.24); 5.7022 (6.95); 5.6982 (6.18); 5.4683 (1.85); 5.4435 (8.54); 5.4390 (7.51); 5.4259 (5.36); 5.3797 (5.13); 5.3376 (1.75); 4.7945 (0.61); 4.7599 (13.49); 4.7241 (0.57); 4.5089 (0.84); 4.3691 (1.76); 4.3361 (1.83); 4.0372 (0.41); 4.0193 (0.46); 3.9893 (1.65); 3.9549 (1.79); 3.8680 (1.68); 3.8370 (2.07); 3.8253 (2.53); 3.7948 (2.21); 3.6188 (2.59); 3.5964 (2.61); 3.5759 (2.01); 3.5680 (1.20); 3.5537 (1.89); 3.4233 (1.18); 3.4140 (0.90); 3.4038 (1.53); 3.3950 (2.36); 3.3857 (1.44); 3.3749 (1.05); 3.3665 (1.44); 3.3563 (1.20); 3.3373 (20.72); 3.3328 (81.37); 3.3044 (1.38); 3.2731 (2.25); 3.2435 (1.28); 2.8904 (0.72); 2.8787 (1.07); 2.8742 (1.11); 2.8464 (2.18); 2.8161 (1.20); 2.7311 (0.42); 2.6759 (1.60); 2.6714 (1.91); 2.6669 (1.28); 2.5525 (2.03); 2.5110 (150.43); 2.5068 (249.11); 2.5023 (291.51); 2.4978 (204.83); 2.4936 (95.93); 2.3336 (1.86); 2.3291 (2.17); 2.3246 (1.56); 2.1444 (1.65); 2.1092 (3.36); 2.0752 (1.93); 1.9895 (1.78); 1.8551 (0.64); 1.8473 (0.72); 1.8253 (1.47); 1.8186 (1.49); 1.7956 (1.45); 1.7886 (1.29); 1.7657 (0.59); 1.6238 (0.64); 1.6127 (0.73); 1.5921 (1.48); 1.5832 (1.50); 1.5615 (1.49); 1.5530 (1.34); 1.5312 (0.61); 1.5218 (0.48); 1.3359 (2.56); 1.2980 (0.76); 1.2724 (0.37); 1.2582 (1.31); 1.2492 (3.17); 1.2351 (1.08); 1.1924 (0.61); 1.1746 (1.01); 1.1568 (0.58); 1.1377 (4.64); -0.0002 (36.52); -0.0085 (1.51) |

| **Beispiel I-8, Lösungsmittel: DMSO-d₆, Spektrometer: 399,95 MHz** |
|---|
| 7.9923 (11.58); 7.5087 (0.95); 7.4851 (2.36); 7.4605 (2.36); 7.4368 (0.95); 7.3140 (2.05); 7.1807 (4.71); 7.1670 (2.23); 7.0475 (2.29); 7.0310 (5.16); 7.0094 (1.55); 7.0048 (1.49); 7.0007 (1.43); 6.9860 (1.44); 6.9814 (1.34); 6.9059 (4.88); 6.8952 (2.64); 6.0729 (1.55); 6.0513 (1.86); 6.0426 (1.81); 6.0207 (1.62); 5.4625 (1.15); 5.4198 (3.83); 5.3779 (3.79); 5.3355 (1.14); 4.8879 (0.34); 4.8822 (0.35); 4.8478 (5.13); 4.8424 (8.89); 4.8371 (5.16); 4.8027 (0.36); 4.7968 (0.36); 4.3709 (1.11); 4.3372 (1.16); 3.9904 (1.04); 3.9561 (1.14); 3.9024 (16.00); 3.8624 (1.24); 3.8316 (1.41); 3.8195 (1.76); 3.7890 (1.54); 3.5683 (1.93); 3.5598 (2.71); 3.5539 (5.68); 3.5479 (4.11); 3.5256 (1.47); 3.5039 (1.45); 3.4343 (0.46); 3.4255 (0.76); 3.4159 (0.55); 3.4058 (0.92); 3.3968 (1.60); 3.3877 (0.94); 3.3774 (0.60); 3.3683 (0.86); 3.3596 (0.46); 3.3186 (55.10); 3.2727 (1.45); 3.2440 (0.80); 3.1746 (0.48); 3.1613 (0.45); 2.8903 (0.54); 2.8737 (0.78); 2.8443 (1.41); 2.8166 (0.78); 2.7310 (0.35); 2.6746 (1.14); 2.6702 (1.58); 2.6658 (1.17); 2.5233 (4.73); 2.5098 (90.48); 2.5056 (178.21); 2.5011 (234.80); 2.4967 (174.33); 2.3322 (1.22); 2.3279 (1.65); 2.3236 (1.24); 2.1526 (1.00); 2.1168 (2.04); 2.0816 (1.16); 1.8585 (0.35); 1.8532 (0.40); 1.8230 (0.91); 1.8004 (0.84); 1.7934 (0.81); 1.7711 (0.33); 1.6310 (0.35); 1.6204 (0.44); 1.5994 (0.89); 1.5901 (0.95); 1.5693 (0.91); 1.5596 (0.86); 1.5389 (0.36); 1.3353 (0.41); 1.2585 (0.38); 1.2494 (0.45); 0.0078 (1.46); -0.0002 (41.84); -0.0082 (1.76) |

| **Beispiel I-9, Lösungsmittel: DMSO-d₆, Spektrometer: 399,95 MHz** |
|---|
| 8.0348 (8.87); 7.3148 (1.29); 7.1815 (3.16); 7.1683 (2.30); 7.1628 (4.01); 7.1401 (3.74); 7.0484 (1.47); 7.0323 (3.61); 6.9072 (2.88); 6.8965 (1.74); 6.0081 (0.88); 5.9872 (1.05); 5.9779 (0.99); 5.9568 (0.90); 5.4627 (0.64); 5.4200 (2.17); 5.3784 (2.16); 5.3358 (0.63); 4.3701 (0.59); 4.3378 (0.63); 4.0554 (1.15); 4.0376 (3.55); 4.0199 (3.60); 4.0020 (1.43); 3.9900 (0.57); 3.9565 (0.64); 3.9330 (0.73); 3.9024 (0.78); 3.8895 (0.92); 3.8592 (0.79); 3.5813 (0.98); 3.5601 (1.01); 3.5377 (0.80); 3.5168 (0.80); 3.4290 (0.45); 3.4199 (0.33); 3.4099 (0.53); 3.4002 (0.91); 3.3915 (0.53); 3.3806 (0.35); 3.3715 (0.50); 3.3228 (57.43); 3.3007 (0.52); 3.2708 (0.80); 3.2420 (0.47); 2.8710 (0.44); 2.8399 (0.76); 2.8133 (0.45); 2.6749 (0.37); 2.6704 (0.55); 2.6658 (0.39); 2.5646 (2.25); 2.5238 (1.62); 2.5191 (2.42); 2.5104 (30.49); 2.5059 (62.12); 2.5013 (81.90); 2.4967 (57.90); 2.4921 (27.04); 2.3328 (0.38); 2.3280 (0.53); 2.3236 (0.38); 2.1485 (0.60); 2.1144 (1.09); 2.0794 (0.63); 1.9887 (16.00); 1.9330 (0.43); 1.9211 (0.85); 1.9135 (1.00); 1.9086 (2.86); 1.9018 (1.61); 1.8967 (0.73); 1.8899 (0.90); 1.8823 (0.95); 1.8706 (0.55); 1.8253 (0.56); 1.8002 (0.44); 1.7957 (0.44); 1.5956 (0.47); 1.5870 (0.52); 1.5656 (0.51); 1.5570 (0.46); 1.4058 (0.78); 1.3974 (5.11); 1.1922 (4.31); 1.1744 (8.70); 1.1566 (4.21); 1.1187 (0.45); 1.1048 (1.62); 1.0972 (3.04); 1.0912 (1.62); 1.0848 (1.50); 1.0770 (2.88); 1.0711 (1.55); 1.0654 (1.93); 1.0594 (2.95); 1.0520 (2.63); 1.0484 (3.15); 1.0404 (1.61); 1.0266 (0.36); 0.0080 (0.85); -0.0002 (28.40); -0.0086 (0.84) |

| **Beispiel I-10, Lösungsmittel: DMSO-d₆, Spektrometer: 399,95 MHz** |
|---|
| 8.0353 (13.66); 7.3972 (1.40); 7.3896 (1.42); 7.3756 (1.54); 7.3683 (2.73); 7.3609 (1.53); 7.3470 (1.47); 7.3393 (1.44); 7.3116 (2.43); 7.1783 (5.57); 7.1667 (2.71); 7.0451 (2.71); 7.0306 (6.11); 7.0162 (1.67); 7.0119 (2.00); 6.9936 (1.71); 6.9894 (2.02); 6.9060 (5.82); 6.8949 (3.16); 5.9853 (1.96); 5.9667 (2.25); 5.9576 (2.25); 5.9386 (2.03); 5.7558 (16.00); 5.4588 (1.35); 5.4163 (4.61); 5.3744 (4.56); 5.3320 (1.37); 4.8270 (11.86); 4.8210 (12.23); 4.3634 (1.35); 4.3305 (1.41); 4.0559 (0.33); 4.0381 (1.05); 4.0203 (1.08); 4.0024 (0.49); 3.9789 (3.26); 3.9506 (3.67); 3.9355 (3.34); 3.9074 (2.39); 3.6710 (2.94); 3.6650 (6.17); 3.6591 (3.10); 3.4209 (0.46); 3.4119 (1.00); 3.4034 (3.18); 3.3928 (1.31); 3.3844 (4.34); 3.3746 (1.26); 3.3601 (2.86); 3.3415 (2.65); 3.3199 (50.52); 3.2951 (1.12); 3.2646 (1.78); 3.2360 (0.96); 2.8658 (0.92); 2.8357 (1.68); 2.8080 (0.94); 2.6753 (0.56); 2.6708 (0.81); 2.6663 (0.59); 2.5239 (1.87); 2.5061 (95.77); 2.5017 (127.50); 2.4973 (96.60); 2.3328 (0.71); 2.3284 (0.93); 2.3244 (0.73); 2.1327 (1.21); 2.0977 (2.49); 2.0642 (1.40); 1.9888 (4.60); 1.8479 (0.44); 1.8397 (0.53); 1.8172 (1.06); 1.8094 (1.17); 1.7867 (1.07); 1.7793 (1.01); 1.7572 (0.43); 1.7477 (0.36); 1.6139 (0.44); 1.6032 (0.54); 1.5828 (1.08); 1.5730 (1.18); 1.5519 (1.10); 1.5432 (1.07); 1.5222 (0.45); 1.5115 (0.37); 1.3360 (0.93); 1.2589 (0.38); 1.2497 (1.07); 1.1927 (1.23); 1.1749 (2.39); 1.1571 (1.19); 0.0079 (1.10); -0.0002 (34.59); -0.0083 (1.46) |

| **Beispiel I-11, Lösungsmittel: DMSO-d₆, Spektrometer: 399,95 MHz** |
|---|
| 8.3939 (0.50); 8.0195 (16.00); 7.9523 (0.43); 7.4270 (0.41); 7.3150 (2.46); 7.1818 (5.77); 7.1688 (2.91); 7.0763 (0.46); 7.0486 (2.82); 7.0327 (6.70); 6.9974 (0.36); 6.9076 (5.75); 6.8969 (3.38); 6.8441 (0.70); 6.8286 (7.12); 6.8019 (7.15); 6.7866 (0.69); 6.0632 (0.73); 6.0499 (1.64); 6.0368 (1.58); 6.0236 (1.93); 6.0203 (1.13); 6.0103 (1.07); 6.0068 (2.10); 5.9936 (1.84); 5.9804 (2.08); 5.9672 (0.99); 5.9408 (1.78); 5.9193 (2.15); 5.9108 (2.09); 5.8891 (1.86); 5.7992 (1.11); 5.7576 (2.26); 5.7219 (0.52); 5.4631 (1.31); 5.4334 (1.52); 5.4295 (3.96); 5.4252 (5.07); 5.4210 (5.59); 5.3901 (1.56); 5.3863 (3.83); 5.3819 (5.52); 5.3785 (5.54); 5.3360 (1.31); 5.3035 (3.45); 5.2998 (3.48); 5.2772 (3.15); 5.2735 (3.25); 4.6353 (4.28); 4.6320 (7.26); 4.6285 (4.87); 4.6222 (4.70); 4.6187 (7.26); 4.6154 (4.55); 4.3715 (1.23); 4.3382 (1.30); 3.9892 (1.12); 3.9556 (1.26); 3.8790 (1.39); 3.8485 (1.60); 3.8358 (1.90); 3.8055 (1.66); 3.5154 (2.01); 3.4938 (2.07); 3.4721 (1.71); 3.4507 (1.68); 3.4357 (0.56); 3.4259 (0.94); 3.4170 (0.65); 3.4070 (1.06); 3.3974 (1.87); 3.3882 (1.10); 3.3784 (0.70); 3.3689 (0.99); 3.3600 (0.56); 3.3216 (94.65); 3.3001 (1.16); 3.2701 (1.67); 3.2420 (0.96); 2.9375 (0.93); 2.8901 (3.73); 2.8699 (0.86); 2.8396 (1.55); 2.8124 (0.91); 2.7305 (2.82); 2.6806 (2.46); 2.6749 (1.31); 2.6703 (1.75); 2.6658 (1.24); 2.6613 (0.53); 2.5236 (4.85); 2.5188 (8.09); 2.5102 (103.54); 2.5058 (208.03); 2.5012 (273.59); 2.4966 (198.03); 2.4922 (95.51); 2.3368 (0.75); 2.3325 (1.46); 2.3280 (1.96); 2.3234 (1.45); 2.3189 (0.76); 2.1470 (1.16); 2.1136 (2.34); 2.0789 (1.38); 1.8599 (0.46); 1.8503 (0.52); 1.8291 (1.01); 1.8214 (1.09); 1.7996 (0.98); 1.7908 (0.96); 1.7681 (0.43); 1.7594 (0.37); 1.6241 (0.42); 1.6153 (0.51); 1.5939 (1.01); 1.5854 (1.07); 1.5641 (1.01); 1.5546 (0.98); 1.5333 (0.44); 1.5239 (0.37); 1.2980 (0.56); 1.2584 (0.78); 1.2440 (0.42); 1.2339 (0.76); 0.1460 (0.65); 0.0079 (5.35); -0.0002 (159.14); -0.0084 (5.61); -0.0257 (0.38); -0.1497 (0.74) |

| **Beispiel I-12, Lösungsmittel: DMSO-d₆, Spektrometer: 399,95 MHz** |
|---|
| 8.3156 (0.33); 8.0077 (16.00); 7.4387 (1.52); 7.4260 (1.68); 7.4151 (2.11); 7.4120 (2.17); 7.4026 (2.14); 7.3994 (2.09); 7.3885 (1.82); 7.3758 (1.70); 7.3139 (2.92); 7.1807 (6.72); 7.1670 (3.26); 7.1336 (1.78); 7.1240 (1.88); 7.1094 (3.00); 7.0999 (2.97); 7.0856 (1.57); 7.0760 (1.49); 7.0475 (3.26); 7.0310 (7.20); 6.9065 (7.33); 6.8952 (3.87); 6.0606 (2.42); 6.0386 (2.92); 6.0303 (2.91); 6.0080 (2.54); 5.7560 (14.97); 5.4622 (1.70); 5.4196 (5.76); 5.3789 (5.65); 5.3363 (1.70); 4.8734 (1.67); 4.8675 (1.74); 4.8338 (6.04); 4.8278 (6.33); 4.8069 (5.95); 4.8010 (6.17); 4.7673 (1.62); 4.7614 (1.70); 4.3729 (1.75); 4.3403 (1.83); 4.0557 (0.46); 4.0377 (1.38); 4.0200 (1.45); 4.0018 (1.23); 3.9905 (1.65); 3.9562 (1.82); 3.8923 (1.61); 3.8616 (1.83); 3.8493 (2.23); 3.8190 (1.99); 3.6461 (3.82); 3.6402 (7.80); 3.6344 (3.96); 3.5426 (2.60); 3.5205 (2.62); 3.4994 (2.11); 3.4773 (2.08); 3.4345 (0.61); 3.4251 (1.17); 3.4160 (0.87); 3.4059 (1.40); 3.3967 (2.42); 3.3875 (1.48); 3.3776 (0.95); 3.3680 (1.33); 3.3590 (0.78); 3.3215 (59.77); 3.2719 (2.28); 3.2428 (1.22); 2.8720 (1.21); 2.8417 (2.19); 2.8144 (1.19); 2.6748 (0.82); 2.6705 (1.14); 2.6663 (0.88); 2.5058 (134.11); 2.5015 (175.63); 2.4972 (134.47); 2.3325 (0.92); 2.3283 (1.21); 2.3240 (0.97); 2.1491 (1.57); 2.1174 (3.23); 2.0818 (1.82); 1.9886 (5.76); 1.8629 (0.53); 1.8532 (0.66); 1.8314 (1.37); 1.8234 (1.52); 1.8009 (1.38); 1.7939 (1.31); 1.7716 (0.56); 1.7624 (0.47); 1.6308 (0.54); 1.6211 (0.70); 1.6006 (1.39); 1.5915 (1.53); 1.5696 (1.41); 1.5605 (1.36); 1.5393 (0.57); 1.5295 (0.47); 1.3358 (1.19); 1.2587 (0.37); 1.2496 (1.37); 1.2350 (0.45); 1.1925 (1.51); 1.1747 (2.97); 1.1570 (1.49); 0.0076 (1.19); -0.0002 (27.94) |

| **Beispiel I-13, Lösungsmittel: DMSO-d₆, Spektrometer: 399,95 MHz** |
|---|
| 8.0230 (0.42); 7.9766 (16.00); 7.3166 (2.62); 7.1833 (6.21); 7.1710 (3.10); 7.0502 (2.99); 7.0350 (7.08); 6.9724 (2.47); 6.9639 (2.17); 6.9575 (1.34); 6.9533 (1.89); 6.9447 (3.17); 6.9407 (3.16); 6.9372 (2.49); 6.9312 (1.60); 6.9102 (7.04); 6.8992 (3.60); 6.0215 (1.98); 5.9995 (2.40); 5.9910 (2.30); 5.9689 (2.05); 5.7596 (15.20); 5.4664 (1.51); 5.4237 (4.78); 5.3813 (4.71); 5.3387 (1.51); 4.9285 (0.41); 4.9226 (0.44); 4.8885 (6.19); 4.8833 (10.07); 4.8781 (6.12); 4.8440 (0.57); 4.8380 (0.44); 4.7531 (0.84); 4.3724 (1.35); 4.3391 (1.46); 4.0553 (0.53); 4.0374 (1.61); 4.0196 (1.67); 4.0017 (1.10); 3.9905 (1.27); 3.9562 (1.41); 3.8062 (1.29); 3.7745 (1.53); 3.7621 (1.86); 3.7319 (1.69); 3.5810 (3.07); 3.5751 (6.98); 3.5692 (3.06); 3.5102 (2.15); 3.4879 (2.20); 3.4672 (1.67); 3.4454 (1.70); 3.4305 (0.52); 3.4212 (0.93); 3.4116 (0.68); 3.4016 (1.14); 3.3926 (1.94); 3.3832 (1.15); 3.3731 (0.76); 3.3638 (1.08); 3.3548 (0.70); 3.3343 (63.61); 3.3003 (1.10); 3.2705 (1.82); 3.2414 (1.04); 2.8702 (0.96); 2.8398 (1.75); 2.8129 (1.01); 2.6761 (0.45); 2.6715 (0.67); 2.6671 (0.49); 2.5250 (1.73); 2.5202 (2.87); 2.5115 (39.45); 2.5071 (80.45); 2.5025 (106.22); 2.4980 (78.38); 2.4936 (39.34); 2.3338 (0.58); 2.3292 (0.78); 2.3247 (0.60); 2.1497 (1.21); 2.1145 (2.50); 2.0789 (1.45); 1.9896 (7.28); 1.8580 (0.45); 1.8507 (0.53); 1.8215 (1.15); 1.7982 (1.07); 1.7910 (1.03); 1.7700 (0.44); 1.7606 (0.37); 1.6249 (0.44); 1.6157 (0.54); 1.5948 (1.09); 1.5856 (1.20); 1.5643 (1.14); 1.5556 (1.09); 1.5346 (0.49); 1.5240 (0.38); 1.3973 (0.86); 1.3364 (0.68); 1.2586 (0.37); 1.2495 (0.89); 1.2347 (0.54); 1.1924 (1.98); 1.1747 (3.96); 1.1568 (1.97); -0.0002 (5.11) |

| **Beispiel I-14, Lösungsmittel: DMSO-d₆, Spektrometer: 399,95 MHz** |
|---|
| 9.9796 (4.86); 9.9008 (0.54); 8.6585 (0.57); 8.3154 (0.32); 8.0576 (10.91); 7.7244 (0.84); 7.7147 (5.87); 7.6945 (5.87); 7.6852 (0.93); 7.3144 (1.98); 7.1811 (4.70); 7.1668 (2.22); 7.0479 (2.24); 7.0307 (5.04); 6.9066 (4.74); 6.8949 (2.57); 6.0913 (1.41); 6.0706 (1.66); 6.0611 (1.62); 6.0401 (1.48); 5.7560 (16.00); 5.4629 (1.02); 5.4204 (3.52); 5.3792 (3.61); 5.3368 (1.08); 4.3743 (1.05); 4.3415 (1.09); 4.0557 (0.33); 4.0378 (1.00); 4.0199 (1.07); 3.9949 (2.01); 3.9647 (2.07); 3.9523 (2.36); 3.9215 (1.40); 3.6330 (1.57); 3.6121 (1.61); 3.5893 (1.31); 3.5684 (1.35); 3.4431 (0.37); 3.4337 (0.70); 3.4239 (0.52); 3.4143 (0.85); 3.4048 (1.46); 3.3953 (0.87); 3.3857 (0.56); 3.3763 (0.76); 3.3669 (0.42); 3.3219 (89.18); 3.2726 (1.41); 3.2440 (0.82); 2.8731 (0.73); 2.8420 (1.33); 2.8151 (0.76); 2.6751 (0.66); 2.6707 (0.94); 2.6662 (0.70); 2.5239 (2.72); 2.5104 (55.80); 2.5061 (112.26); 2.5017 (148.22); 2.4972 (109.34); 2.4928 (55.27); 2.3328 (0.76); 2.3284 (1.03); 2.3238 (0.79); 2.1512 (0.95); 2.1178 (1.95); 2.0830 (1.12); 1.9887 (4.39); 1.8568 (0.39); 1.8269 (0.85); 1.8049 (0.79); 1.6219 (0.41); 1.6006 (0.85); 1.5913 (0.88); 1.5703 (0.82); 1.5613 (0.78); 1.5409 (0.35); 1.3358 (1.70); 1.2983 (0.37); 1.2587 (0.54); 1.2496 (2.04); 1.2348 (0.55); 1.1926 (1.17); 1.1748 (2.28); 1.1570 (1.14); 0.0079 (0.70); -0.0002 (19.92); -0.0081 (0.85) |

| **Beispiel I-15, Lösungsmittel: DMSO-d₆, Spektrometer: 399,95 MHz** |
|---|
| 7.9638 (16.00); 7.3135 (2.67); 7.1803 (6.33); 7.1676 (3.03); 7.0472 (3.06); 7.0317 (6.94); 6.9073 (6.53); 6.8959 (3.48); 6.8640 (1.24); 6.8580 (1.73); 6.8357 (7.52); 6.8101 (6.76); 6.0360 (2.24); 6.0152 (2.76); 6.0049 (2.58); 5.9840 (2.36); 5.4671 (1.70); 5.4252 (4.81); 5.3719 (4.71); 5.3290 (1.81); 4.3629 (1.49); 4.3316 (1.53); 4.0557 (0.64); 4.0378 (1.80); 4.0200 (1.88); 4.0024 (0.85); 3.9846 (1.39); 3.9500 (1.57); 3.9166 (2.34); 3.8997 (2.54); 3.8909 (3.26); 3.8741 (3.15); 3.7851 (1.71); 3.7600 (2.98); 3.7539 (2.55); 3.7418 (5.55); 3.7349 (2.71); 3.7145 (3.16); 3.5839 (2.65); 3.5629 (2.77); 3.5416 (1.93); 3.5208 (1.83); 3.4275 (0.70); 3.4181 (1.18); 3.4078 (0.90); 3.3984 (1.36); 3.3891 (2.37); 3.3808 (1.53); 3.3601 (2.29); 3.3507 (2.26); 3.3268 (446.42); 3.2902 (0.94); 3.2701 (2.16); 3.2422 (1.04); 2.8712 (1.05); 2.8397 (1.87); 2.8124 (1.08); 2.6752 (1.31); 2.6708 (1.75); 2.6662 (1.33); 2.5239 (5.68); 2.5105 (105.72); 2.5062 (210.23); 2.5017 (276.22); 2.4972 (205.17); 2.4929 (104.36); 2.3330 (1.29); 2.3285 (1.76); 2.3239 (1.28); 2.1225 (1.35); 2.0915 (2.77); 2.0560 (1.54); 2.0275 (0.42); 2.0086 (0.62); 1.9889 (8.29); 1.8299 (0.55); 1.8098 (1.17); 1.8016 (1.25); 1.7801 (1.15); 1.7723 (1.09); 1.7497 (0.44); 1.5959 (0.54); 1.5689 (1.19); 1.5437 (1.12); 1.5124 (0.46); 1.3977 (1.17); 1.3356 (1.03); 1.2943 (0.38); 1.2493 (2.29); 1.2363 (2.34); 1.1924 (2.17); 1.1746 (4.15); 1.1568 (2.07); 1.0059 (0.60); 0.9922 (1.19); 0.9876 (1.08); 0.9753 (1.81); 0.9571 (1.49); 0.9421 (0.72); 0.8542 (0.74); 0.8369 (0.33); 0.3767 (0.61); 0.3674 (0.77); 0.3551 (1.80); 0.3466 (1.68); 0.3368 (2.37); 0.3216 (1.21); 0.3159 (0.91); 0.2094 (0.37); 0.1899 (1.04); 0.1722 (2.53); 0.1621 (5.70); 0.1550 (4.79); 0.1489 (4.84); 0.1364 (3.36); 0.1256 (2.35); 0.1167 (1.24); 0.1082 (0.77); 0.0001 (2.70) |

| **Beispiel I-16, Lösungsmittel: DMSO-d₆, Spektrometer: 399,95 MHz** |
|---|
| 8.0268 (14.48); 7.3244 (1.39); 7.3178 (1.71); 7.3125 (2.87); 7.3014 (1.80); 7.2963 (2.50); 7.2911 (1.80); 7.2746 (1.43); 7.2682 (1.44); 7.1792 (5.89); 7.1660 (2.94); 7.1548 (1.89); 7.1501 (2.49); 7.1451 (1.79); 7.1312 (1.92); 7.1266 (2.48); 7.0460 (2.81); 7.0300 (6.49); 6.9059 (6.08); 6.8942 (3.34); 5.8657 (2.07); 5.8437 (2.49); 5.8351 (2.47); 5.8129 (2.16); 5.7556 (16.00); 5.4644 (1.49); 5.4218 (4.55); 5.3742 (4.52); 5.3317 (1.50); 4.3690 (1.38); 4.3358 (1.47); 4.0555 (0.76); 4.0377 (2.35); 4.0199 (2.42); 4.0020 (1.15); 3.9874 (1.29); 3.9525 (1.45); 3.8976 (1.65); 3.8667 (1.92); 3.8542 (2.20); 3.8235 (1.89); 3.4337 (2.66); 3.4236 (1.21); 3.4118 (2.82); 3.4048 (1.49); 3.3907 (2.94); 3.3679 (2.77); 3.3577 (0.78); 3.3217 (65.27); 3.2691 (1.83); 3.2411 (0.99); 3.1182 (0.41); 2.8695 (0.96); 2.8392 (1.76); 2.8121 (1.03); 2.6749 (0.55); 2.6704 (0.80); 2.6660 (0.60); 2.5237 (2.14); 2.5099 (45.65); 2.5058 (91.16); 2.5014 (120.94); 2.4969 (90.89); 2.4926 (47.44); 2.3326 (0.61); 2.3282 (0.85); 2.3237 (0.65); 2.1379 (1.22); 2.1042 (2.59); 2.0695 (1.44); 1.9885 (10.15); 1.8510 (0.44); 1.8412 (0.52); 1.8204 (1.09); 1.8118 (1.19); 1.7899 (1.09); 1.7821 (1.04); 1.7608 (0.45); 1.7493 (0.40); 1.7424 (0.76); 1.7308 (1.41); 1.7226 (1.67); 1.7113 (2.97); 1.6999 (1.82); 1.6914 (1.68); 1.6799 (0.84); 1.6159 (0.42); 1.6054 (0.51); 1.5841 (1.07); 1.5750 (1.17); 1.5539 (1.08); 1.5451 (1.06); 1.5235 (0.44); 1.5176 (0.40); 1.3357 (1.04); 1.2495 (1.26); 1.1925 (2.72); 1.1748 (5.43); 1.1569 (2.68); 1.0448 (0.33); 1.0350 (0.92); 1.0210 (1.53); 1.0161 (1.70); 1.0112 (1.94); 1.0000 (3.16); 0.9909 (2.45); 0.9814 (3.03); 0.9739 (1.47); 0.9618 (1.29); 0.9528 (0.84); 0.9322 (0.97); 0.9222 (1.68); 0.9119 (1.46); 0.9067 (1.74); 0.8957 (1.71); 0.8891 (1.79); 0.8813 (1.66); 0.8701 (2.61); 0.8613 (1.65); 0.8494 (1.79); 0.8410 (1.16); 0.8295 (0.77); 0.8202 (0.45); 0.8154 (0.40); 0.8081 (0.67); 0.7881 (0.67); 0.7832 (0.64); 0.7772 (0.69); 0.7702 (0.59); 0.7658 (0.75); 0.7586 (0.33); 0.6934 (0.62); - 0.0002 (6.53); -0.0084 (0.34) |

| **Beispiel I-17, Lösungsmittel: DMSO-d₆, Spektrometer: 399,95 MHz** |
|---|
| 8.3192 (0.59); 7.9858 (16.00); 7.4606 (1.56); 7.4367 (4.09); 7.4123 (4.19); 7.3884 (1.63); 7.3160 (3.19); 7.1828 (7.31); 7.1702 (3.69); 7.0497 (3.58); 7.0341 (7.61); 6.9086 (10.84); 6.8986 (7.19); 6.1083 (2.81); 6.0869 (3.42); 6.0780 (3.39); 6.0564 (2.96); 5.9233 (0.75); 5.9097 (1.59); 5.8968 (1.64); 5.8831 (2.09); 5.8674 (2.27); 5.8536 (1.91); 5.8404 (1.99); 5.8272 (1.03); 5.7596 (12.92); 5.4694 (2.35); 5.4267 (6.85); 5.3797 (6.78); 5.3372 (6.61); 5.2915 (3.99); 5.1277 (4.64); 5.1013 (4.37); 4.6098 (0.83); 4.5972 (0.95); 4.5772 (4.62); 4.5648 (8.51); 4.5519 (4.63); 4.5334 (0.97); 4.5192 (0.86); 4.5083 (1.39); 4.3716 (2.33); 4.3401 (2.43); 4.0371 (0.32); 4.0197 (0.41); 3.9901 (2.15); 3.9563 (2.36); 3.8503 (2.27); 3.8194 (2.67); 3.8076 (3.44); 3.7769 (2.96); 3.5698 (3.79); 3.5492 (3.34); 3.5282 (2.56); 3.5067 (2.49); 3.4287 (1.43); 3.4197 (1.13); 3.4092 (1.81); 3.4000 (2.96); 3.3917 (1.89); 3.3809 (1.26); 3.3719 (1.72); 3.3322 (80.29); 3.3046 (2.09); 3.2739 (3.01); 3.2448 (1.60); 2.8900 (0.73); 2.8743 (1.52); 2.8441 (2.85); 2.8156 (1.54); 2.7311 (0.42); 2.6715 (2.00); 2.5525 (2.69); 2.5025 (299.09); 2.3292 (2.27); 2.1459 (2.12); 2.1112 (4.39); 2.0771 (2.51); 1.9895 (1.29); 1.8590 (0.74); 1.8508 (0.89); 1.8287 (1.83); 1.8219 (1.98); 1.7996 (1.83); 1.7923 (1.74); 1.7692 (0.75); 1.7606 (0.64); 1.6251 (0.72); 1.6155 (0.90); 1.5952 (1.81); 1.5861 (2.01); 1.5642 (1.86); 1.5557 (1.81); 1.5349 (0.77); 1.5251 (0.63); 1.3359 (1.62); 1.2981 (0.56); 1.2585 (0.77); 1.2494 (1.77); 1.2341 (0.61); 1.1930 (0.44); 1.1746 (0.70); 1.1571 (0.45); 1.1378 (6.70); -0.0002 (28.42) |

| **Beispiel I-18, Lösungsmittel: DMSO-d₆, Spektrometer: 399,95 MHz** |
|---|
| 7.9519 (12.77); 7.3153 (2.05); 7.2595 (5.62); 7.2546 (6.60); 7.2018 (5.65); 7.1970 (5.00); 7.1820 (4.90); 7.1688 (2.31); 7.0489 (2.36); 7.0328 (5.52); 6.9086 (4.64); 6.8970 (2.70); 6.2163 (1.88); 6.1931 (2.40); 6.1853 (2.22); 6.1620 (1.95); 5.7985 (0.50); 5.7847 (1.07); 5.7717 (1.08); 5.7580 (16.00); 5.7416 (1.38); 5.7284 (1.10); 5.7152 (1.25); 5.7017 (0.61); 5.4693 (1.24); 5.4267 (3.60); 5.3773 (3.53); 5.3349 (1.23); 5.2631 (2.60); 5.2593 (2.67); 5.2198 (2.20); 5.2160 (2.25); 5.0181 (2.44); 5.0149 (2.45); 4.9917 (2.22); 4.9885 (2.27); 4.6258 (0.77); 4.6123 (0.78); 4.5940 (2.10); 4.5807 (2.03); 4.5625 (2.07); 4.5483 (2.01); 4.5308 (0.78); 4.5165 (0.72); 4.3691 (1.10); 4.3368 (1.13); 4.0553 (0.40); 4.0375 (1.18); 4.0197 (1.22); 4.0017 (0.83); 3.9905 (1.01); 3.9562 (1.11); 3.7671 (1.49); 3.7359 (1.90); 3.7249 (2.54); 3.6939 (2.04); 3.5559 (2.19); 3.5327 (2.20); 3.5138 (1.59); 3.4907 (1.57); 3.4354 (0.40); 3.4259 (0.76); 3.4169 (0.57); 3.4066 (0.93); 3.3976 (1.58); 3.3884 (0.94); 3.3782 (0.62); 3.3690 (0.88); 3.3593 (0.59); 3.3279 (75.26); 3.3043 (2.20); 3.2757 (1.43); 3.2467 (0.75); 2.8765 (0.78); 2.8461 (1.36); 2.8185 (0.76); 2.6757 (0.34); 2.6709 (0.47); 2.6665 (0.34); 2.5410 (0.37); 2.5108 (26.24); 2.5064 (52.28); 2.5019 (69.42); 2.4974 (50.91); 2.4929 (24.96); 2.3332 (0.33); 2.3287 (0.47); 2.3240 (0.33); 2.1442 (0.95); 2.1092 (2.00); 2.0757 (1.10); 1.9890 (5.16); 1.8563 (0.34); 1.8468 (0.41); 1.8255 (0.88); 1.8173 (0.94); 1.7945 (0.87); 1.7859 (0.81); 1.7655 (0.34); 1.6238 (0.34); 1.6133 (0.42); 1.5924 (0.87); 1.5834 (0.96); 1.5624 (0.88); 1.5534 (0.85); 1.5322 (0.35); 1.3358 (0.63); 1.2585 (0.41); 1.2491 (0.82); 1.2352 (0.45); 1.1924 (1.41); 1.1746 (2.79); 1.1568 (1.36); 0.0844 (0.53); 0.0707 (0.38); 0.0079 (2.33); -0.0002 (57.94); -0.0085 (2.34) |

| **Beispiel I-19, Lösungsmittel: DMSO-d₆, Spektrometer: 399,95 MHz** |
|---|
| 8.3189 (0.39); 7.9626 (12.22); 7.3152 (1.99); 7.1819 (4.72); 7.1696 (2.36); 7.0488 (2.27); 7.0335 (5.32); 7.0053 (1.86); 6.9775 (1.90); 6.9603 (1.25); 6.9544 (1.02); 6.9370 (1.56); 6.9326 (2.02); 6.9275 (1.31); 6.9094 (5.97); 6.8978 (2.82); 6.0590 (1.58); 6.0363 (1.96); 6.0286 (1.87); 6.0056 (1.69); 5.7591 (16.00); 5.7252 (4.35); 5.7230 (4.39); 5.7212 (4.45); 5.4675 (1.26); 5.4403 (5.36); 5.4358 (5.54); 5.4251 (3.89); 5.3782 (3.64); 5.3357 (1.24); 4.7896 (11.21); 4.3666 (1.10); 4.3338 (1.17); 4.0373 (0.84); 4.0194 (0.86); 4.0014 (0.57); 3.9869 (1.02); 3.9524 (1.13); 3.8054 (1.09); 3.7750 (1.31); 3.7632 (1.69); 3.7325 (1.50); 3.5684 (1.81); 3.5457 (1.82); 3.5258 (1.32); 3.5032 (1.30); 3.4276 (0.37); 3.4183 (0.72); 3.4085 (0.54); 3.3991 (0.93); 3.3899 (1.54); 3.3806 (0.95); 3.3705 (0.68); 3.3613 (0.95); 3.3332 (104.96); 3.3063 (0.96); 3.3010 (1.00); 3.2709 (1.49); 3.2423 (0.82); 2.8725 (0.77); 2.8414 (1.40); 2.8145 (0.79); 2.6805 (0.32); 2.6760 (0.66); 2.6714 (0.92); 2.6668 (0.67); 2.5414 (0.34); 2.5247 (2.44); 2.5111 (52.04); 2.5069 (104.57); 2.5023 (139.02); 2.4978 (104.39); 2.4936 (53.31); 2.3337 (0.75); 2.3291 (1.01); 2.3245 (0.76); 2.1392 (0.95); 2.1053 (2.03); 2.0705 (1.18); 1.9894 (3.75); 1.8525 (0.36); 1.8439 (0.45); 1.8229 (0.89); 1.8145 (0.97); 1.7919 (0.92); 1.7839 (0.84); 1.7627 (0.38); 1.6201 (0.35); 1.6095 (0.44); 1.5888 (0.87); 1.5790 (0.97); 1.5581 (0.92); 1.5492 (0.88); 1.5287 (0.39); 1.3358 (0.67); 1.2493 (0.83); 1.1925 (1.01); 1.1747 (1.98); 1.1569 (0.97); 0.1459 (0.36); 0.0080 (2.93); -0.0002 (85.86); -0.0084 (3.77); -0.1497 (0.39) |

| **Beispiel I-20, Lösungsmittel: DMSO-d₆, Spektrometer: 399,95 MHz** |
|---|
| 8.0177 (0.50); 7.9663 (12.01); 7.3158 (2.02); 7.1825 (4.88); 7.1699 (2.48); 7.0494 (2.29); 7.0339 (5.47); 6.9095 (5.64); 6.8981 (3.92); 6.8859 (2.24); 6.8775 (2.09); 6.8739 (1.98); 6.8680 (1.34); 6.8508 (1.36); 6.8450 (1.00); 6.0534 (1.62); 6.0320 (1.93); 6.0228 (1.87); 6.0011 (1.71); 5.9005 (0.48); 5.8872 (1.02); 5.8740 (0.98); 5.8607 (1.23); 5.8441 (1.35); 5.8307 (1.20); 5.8175 (1.31); 5.8044 (0.64); 5.7593 (16.00); 5.4690 (1.24); 5.4264 (3.71); 5.3790 (3.71); 5.3378 (3.52); 5.3342 (3.48); 5.2948 (2.12); 5.2908 (2.18); 5.1247 (2.30); 5.1214 (2.37); 5.0983 (2.16); 5.0950 (2.23); 4.6331 (0.50); 4.6201 (0.49); 4.6007 (2.35); 4.5972 (1.88); 4.5882 (3.95); 4.5762 (2.34); 4.5570 (0.64); 4.5436 (0.56); 4.5087 (0.38); 4.3711 (1.11); 4.3379 (1.18); 4.0371 (0.66); 4.0194 (0.71); 4.0010 (0.64); 3.9893 (1.03); 3.9560 (1.13); 3.7900 (1.12); 3.7589 (1.33); 3.7471 (1.67); 3.7166 (1.45); 3.5152 (1.76); 3.4935 (1.80); 3.4726 (1.39); 3.4511 (1.36); 3.4338 (0.42); 3.4245 (0.72); 3.4153 (0.54); 3.4052 (0.91); 3.3960 (1.56); 3.3865 (0.98); 3.3770 (0.62); 3.3673 (0.87); 3.3576 (0.63); 3.3337 (91.42); 3.3088 (1.07); 3.3029 (1.05); 3.2718 (1.47); 3.2434 (0.84); 2.8724 (0.76); 2.8416 (1.38); 2.8148 (0.81); 2.6757 (0.62); 2.6712 (0.86); 2.6668 (0.64); 2.5525 (0.69); 2.5244 (2.32); 2.5110 (47.11); 2.5067 (94.65); 2.5022 (126.48); 2.4977 (95.53); 2.4934 (49.52); 2.3335 (0.70); 2.3290 (0.94); 2.3244 (0.71); 2.1427 (0.98); 2.1102 (2.06); 2.0752 (1.20); 1.9893 (2.96); 1.8579 (0.37); 1.8484 (0.48); 1.8267 (0.91); 1.8184 (0.98); 1.7966 (0.91); 1.7883 (0.85); 1.7671 (0.39); 1.6230 (0.38); 1.6129 (0.48); 1.5917 (0.91); 1.5829 (1.01); 1.5613 (0.94); 1.5518 (0.89); 1.5317 (0.41); 1.5211 (0.33); 1.3356 (1.57); 1.2979 (0.55); 1.2583 (0.82); 1.2491 (1.97); 1.2343 (0.97); 1.1924 (0.83); 1.1747 (1.56); 1.1569 (0.81); 1.1378 (2.06); 0.0079 (2.38); -0.0002 (69.49); -0.0084 (3.56) |

| **Beispiel I-21, Lösungsmittel: DMSO-d₆, Spektrometer: 399,95 MHz** |
|---|
| 7.9640 (12.03); 7.3189 (5.52); 7.3141 (8.26); 7.2767 (5.84); 7.2720 (4.94); 7.1815 (4.70); 7.1691 (2.24); 7.0483 (2.25); 7.0331 (5.18); 6.9085 (4.76); 6.8973 (2.56); 6.1891 (1.86); 6.1649 (2.40); 6.1584 (2.24); 6.1340 (1.93); 5.7579 (16.00); 5.4636 (1.17); 5.4210 (3.78); 5.3793 (3.69); 5.3367 (1.14); 4.8995 (0.50); 4.8936 (0.51); 4.8596 (4.21); 4.8522 (5.90); 4.8451 (4.17); 4.8111 (0.49); 4.8052 (0.49); 4.3699 (1.11); 4.3371 (1.18); 4.0553 (0.59); 4.0374 (1.80); 4.0196 (1.85); 4.0018 (1.04); 3.9894 (1.03); 3.9552 (1.12); 3.7725 (1.46); 3.7415 (1.85); 3.7302 (2.45); 3.6992 (1.98); 3.5621 (2.19); 3.5378 (2.21); 3.5198 (1.61); 3.4956 (1.59); 3.4756 (2.42); 3.4698 (5.27); 3.4640 (2.36); 3.4266 (0.40); 3.4188 (0.78); 3.4090 (0.56); 3.3995 (0.93); 3.3900 (1.60); 3.3810 (0.95); 3.3706 (0.65); 3.3611 (0.91); 3.3524 (0.61); 3.3282 (59.14); 3.3043 (2.05); 3.2708 (1.45); 3.2418 (0.78); 2.8735 (0.81); 2.8426 (1.40); 2.8151 (0.78); 2.6756 (0.35); 2.6709 (0.47); 2.6666 (0.36); 2.5064 (50.45); 2.5019 (67.00); 2.4974 (49.25); 2.4931 (24.03); 2.3286 (0.44); 2.3241 (0.34); 2.1495 (0.99); 2.1164 (1.96); 2.0804 (1.10); 1.9890 (7.82); 1.8487 (0.39); 1.8255 (0.88); 1.8194 (0.91); 1.7963 (0.82); 1.6264 (0.34); 1.6162 (0.40); 1.5952 (0.85); 1.5862 (0.92); 1.5645 (0.85); 1.5568 (0.81); 1.5356 (0.33); 1.3357 (0.61); 1.2585 (0.35); 1.2492 (0.75); 1.1924 (2.17); 1.1746 (4.27); 1.1568 (2.09); 0.0844 (0.48); 0.0707 (0.35); 0.0080 (2.15); -0.0002 (54.96); -0.0085 (1.95) |

Die Intensität scharfer Signale korreliert mit der Höhe der Signale in einem gedruckten Beispiel eines NMR-Spektrums in cm und zeigt die wirklichen Verhältnisse der Signalintensitäten. Bei breiten Signalen können mehrere Peaks oder die Mitte des Signals und ihre relative Intensität im Vergleich zum intensivsten Signal im Spektrum gezeigt werden.

Die Listen der 1H-NMR-Peaks sind ähnlich den klassischen 1H-NMR-Ausdrucken und enthalten somit gewöhnlich alle Peaks, die bei einer klassischen NMR-Interpretation aufgeführt werden.

Darüber hinaus können sie wie klassische 1H-NMR-Ausdrucke Lösungsmittelsignale, Signale von Stereoisomeren der Zielverbindungen, die ebenfalls Gegenstand der Erfindung sind, und/oder Peaks von Verunreinigungen zeigen.

Bei der Angabe von Verbindungssignalen im delta-Bereich von Lösungsmitteln und/oder Wasser sind in unseren Listen von 1H-NMR-Peaks die gewöhnlichen Lösungsmittelpeaks, zum Beispiel Peaks von DMSO in DMSO-d₆ und der Peak von Wasser, gezeigt, die gewöhnlich im Durchschnitt eine hohe Intensität aufweisen.

Die Peaks von Stereoisomeren der Targetverbindungen und/oder Peaks von Verunreinigungen haben gewöhnlich im Durchschnitt eine geringere Intensität als die Peaks der Zielverbindungen (zum Beispiel mit einer Reinheit von >90%).

Solche Stereoisomere und/oder Verunreinigungen können typisch für das jeweilige Herstellungsverfahren sein. Ihre Peaks können somit dabei helfen, die Reproduktion unseres Herstellungsverfahrens anhand von "Nebenprodukt-Fingerabdrücken" zu erkennen.

Einem Experten, der die Peaks der Zielverbindungen mit bekannten Verfahren (MestreC, ACD-Simulation, aber auch mit empirisch ausgewerteten Erwartungswerten) berechnet, kann je nach Bedarf die Peaks der Zielverbindungen isolieren, wobei gegebenenfalls zusätzliche Intensitätsfilter eingesetzt werden. Diese Isolierung wäre ähnlich dem betreffenden Peak-Picking bei der klassischen 1H-NMR-Interpretation.

### Verwendungsbeispiele

### Beispiel A

### Phytophthora-Test (Tomate)/protektiv

| | | |
|---|---|---|
| Lösungsmittel: | 49 | Gewichtsteile N, N - Dimethylformamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Tomatenpflanzen mit der Wirkstoffzubereitung in der angegebenen Aufwandmenge besprüht. 1 Tag nach der Behandlung werden die Pflanzen mit einer Sporensuspension von ***Phytophthora infestans*** inokuliert und stehen dann 24h bei 100 rel. Feuchte und 22°C. Anschließend werden die Pflanzen in einer Klimazelle bei ca. 96% relativer Luftfeuchtigkeit und einer Temperatur von ca. 20°C aufgestellt.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

In diesem Test zeigen die nachfolgenden erfindungsgemäßen Verbindungen bei einer Konzentration an Wirkstoff von 100ppm einen Wirkungsgrad von 70% oder mehr.

| Bsp.-Nr. | Eff.% |
|---|---|
| I-1 | 98 |
| I-2 | 94 |
| I-3 | 98 |
| I-4 | 95 |
| I-5 | 95 |
| I-18 | 95 |
| I-21 | 95 |

### Beispiel B

### Plasmopara-Test (Rebe)/protektiv

| | | |
|---|---|---|
| Lösungsmittel : | 24,5 | Gewichtsteile Aceton |
| | 24,5 | Gewichtsteile Dimethylacetamid |
| Emulgator : | 1 | Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Sporensuspension von ***Plasmopara viticola*** inokuliert und verbleiben dann 1 Tag in einer Inkubationskabine bei ca. 20°C und 100% relativer Luftfeuchtigkeit. Anschließend werden die Pflanzen 4 Tage im Gewächshaus bei ca. 21°C und ca. 90% Luftfeuchtigkeit aufgestellt. Die Pflanzen werden dann angefeuchtet und 1 Tag in eine Inkubationskabine gestellt.

6 Tage nach der Inokulation erfolgt die Auswertung. Dabei bedeutet 0% ein Wirkungsgrad, der demjenigen der Kontrolle entspricht, während ein Wirkungsgrad von 100% bedeutet, daß kein Befall beobachtet wird.

In diesem Test zeigen die nachfolgenden erfindungsgemäßen Verbindungen bei einer Konzentration an Wirkstoff von 10ppm einen Wirkungsgrad von 70% oder mehr.

| Bsp.-Nr. | Eff.% |
|---|---|
| I-2 | 100 |
| I-3 | 100 |
| I-4 | 100 |
| I-5 | 96 |
| I-7 | 100 |
| I-8 | 100 |
| I-13 | 100 |
| I-17 | 100 |
| I-18 | 96 |
| I-19 | 100 |
| I-20 | 100 |
| I-21 | 100 |

## Patentansprüche

1. Verbindungen der Formel (I), 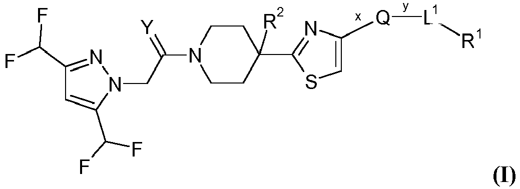 in welcher die Restedefinitionen folgende Bedeutungen haben:
Y steht für Sauerstoff oder Schwefel,
R² steht für Wassertoff oder Halogen
Q steht für 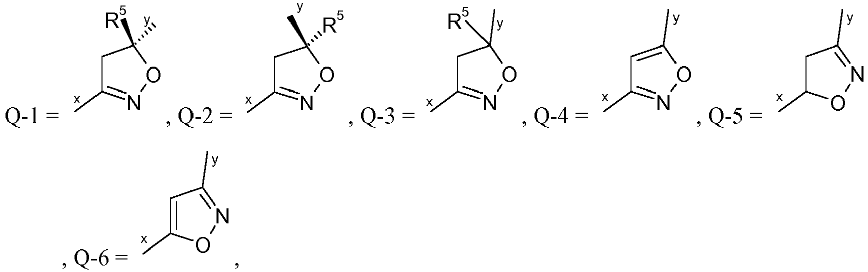
wobei die Bindung, die mit "x" identifiziert ist, direkt an das Thiazol gebunden ist, und wobei die Bindung, die mit "y" identifiziert ist, direkt an L¹ bzw. R¹ gebunden ist,
R⁵ steht für Wasserstoff, Cyano, C₁-C₃-Alkyl oder C₁-C₃-Halogenalkyl,
L¹ steht für eine direkte Bindung, -CH₂-, -(C=O)-, Schwefel oder Sauerstoff,
R¹ steht für Phenyl, das mindestens einen Substituenten Z⁴ enthält und zusätzlich zwei, drei oder vier weitere Substituenten, welche unabhängig ausgewählt sind aus Z⁴ und Z¹, enthält,
oder
R¹ steht für Naphthyl, Dihydronaphthalenyl, Tetrahydronaphthalenyl, Hexahydronaphthalenyl, Octahydronaphthalenyl oder Indenyl, das mindestens einen Substituenten Z⁵ enthält und zusätzlich zwei, drei oder vier weitere Substituenten, welche unabhängig ausgewählt sind aus Z⁵ und Z¹, enthält,
oder
R¹ steht für ein gegebenenfalls benzokondensiertes, substituiertes 5- oder 6-gliedriges Heteroaryl, das mindestens einen Substituenten Z⁶ emthält und zusätzlich zwei, drei oder vier weitere Substituenten, welche am Kohlenstoff unabhängig voneinander aus Z⁶ und Z¹ ausgewählt sind und am Stickstoff unabhängig voneinander ausgewählt sind aus Z⁶ und Z², enthält,
oder
R¹ steht für ein C₃-C₈-Cycloalkyl oder für ein C₅-C₈-Cycloalkenyl, das mindestens einen Substituenten Z⁷ enthält und zusätzlich zwei, drei oder vier weitere Substituenten, welche unabhängig voneinander ausgewählt sind aus Z⁷ und Z¹, enthält,
Z¹ steht für Halogen, Hydroxy, Nitro, Cyano, Alkyl, Alkenyl, Alkinyl, Halogenalkyl, Halogenalkenyl, Halogenalkinyl, Cycloalkyl, Halogencycloalkyl, Alkoxy, Alkoxyalkyl, Hydroxyalkyl, Halogenalkoxy, Alkylcarbonyl, Alkoxycarbonyl, Alkylcycloalkyl, Cycloalkoxyalkyl, Cycloalkylamino, Alkylthio, Halogenalkylthio, Cycloalkylthio, Cycloalkylalkyl, Alkylcarbonyloxy, Alkylcarbonylamino, Halogenalkylcarbonylamino, Alkylcarbonylthio, Alkylsulfinyl, Halogenalkylsulfinyl, Alkylsulfonyloxy, Halogenalkylsulfonyloxy, Alkylcycloalkylalkyl, -C(=O)NR³R⁴, -NR³R⁴, Tri(C₁-C₂-alkyl)silyl, oder -L³Z³,
Z² steht für Wasserstoff, Halogen, Hydroxy, Nitro, Cyano, Alkyl, Alkenyl, Alkinyl, Halogenalkyl, Halogenalkenyl, Halogenalkinyl, Cycloalkyl, Halogencycloalkyl, Alkoxy, Alkoxyalkyl, Hydroxyalkyl, Halogenalkoxy, Alkylcarbonyl, Alkoxycarbonyl, Alkylcycloalkyl, Cycloalkoxyalkyl, Cycloalkylamino, Alkylthio, Halogenalkylthio, Cycloalkylthio, Cycloalkylalkyl, Alkylcarbonyloxy, Alkylcarbonylamino, Halogenalkylcarbonylamino, Alkylcarbonylthio, Alkylsulfinyl, Halogenalkylsulfinyl, Alkylsulfonyloxy, Halogenalkylsulfonyloxy, Alkylcycloalkylalkyl, -C(=O)NR³R⁴, -NR³R⁴ oder Tri(C₁-C₂-alkyl)silyl,
Z³ steht für einen Phenylrest, Naphthalenylrest oder einen 5- oder 6-gliedrigen Heteroarylrest, der jeweils 0, 1, 2 oder 3 Substituenten enthalten kann, wobei die Substituenten unabhängig voneinander ausgewählt sind aus folgender Liste:
Substituenten am Kohlenstoff: Halogen, Cyano, Nitro, Hydroxy, Amino, -SH, Alkyl, Alkenyl, Alkinyl, Halogenalkyl, Halogenalkenyl, Halogenalkinyl, Cycloalkyl, Halogencycloalkyl, Alkoxyalkyl, Alkylcarbonyl, Halogenalkylcarbonyl, Alkoxycarbonyl, Alkoxy, Halogenalkoxy, Cycloalkoxy, Halogencycloalkoxy, Alkenyloxy, Alkinyloxy, Alkoxyalkoxy, Alkylamino, Dialkylamino, Alkylthio, Halogenalkylthio, Alkylsulfinyl, Halogenalkylsulfinyl, Alkylsulfonyl, Halogenalkylsulfonyl, Trisilylalkyl oder Phenyl,
Substituenten am Stickstoff: Wasserstoff, -C(=O)H, Alkyl, Alkenyl, Alkinyl, Halogenalkyl, Halogenalkenyl, Halogenalkinyl, Cycloalkyl, Halogencycloalkyl, Alkylcycloalkyl, Cycloalkylalkyl, Alkoxyalkyl, Alkylsulfonyl, Halogenalkylsulfonyl, Cycloalkylsulfonyl, Phenylsulfonyl, Alkylcarbonyl, Halogenalkylcarbonyl, Alkoxycarbonyl, Halogenalkoxycarbonyl, Cycloalkoxycarbonyl, -C(=O)NR³R⁴, Phenyl oder Benzyl,
Z⁴ steht für SH, C(=O)H, C₇-C₈-Cycloalkyl, C₇-C₈-Halogencycloalkyl, Cycloalkylcycloalkyl, Halogencycloalkylalkyl, Cycloalkenyl, Halogencycloalkenyl, C₅-C₆-Alkoxyalkyl, Alkoxyalkoxyalkyl, Alkylthioalkyl, Alkylsulfinylalkyl, Alkylsulfonylalkyl, Alkylaminoalkyl, Dialkylaminoalkyl, Halogenalkylaminoalkyl, Cycloalkylaminoalkyl, C₅-C₆-Alkylcarbonyl, Halogenalkylcarbonyl, Cycloalkylcarbonyl, Cycloalkoxycarbonyl, Cycloalkylalkoxycarbonyl, Cycloalkylaminocarbonyl, Halogenalkoxyalkyl, C₅-C₆-Hydroxyalkyl, C₅-C₆-Alkoxy, C₅-C₆-Halogenalkoxy, Cycloalkoxy, Halogencycloalkoxy, Cycloalkylalkoxy, Alkenyloxy, Halogenalkenyloxy, Alkinyloxy, Halogenalkinyloxy, Alkoxyalkoxy, Halogenalkylcarbonyloxy, Cycloalkylcarbonyloxy, Alkylcarbonylalkoxy, C₅-C₆-Alkylthio, C₅-C₆-Halogenalkylthio, C₅-C₆-Alkylsulfinyl, C₅-C₆-Halogenalkylsulfinyl, Alkylsulfonyl, C₁-C₄-Halogenalkylsulfonyl, Cycloalkylsulfonyl, Tri(C₃-C₄-alkyl)silyl, Alkylsulfonylamino oder Halogenalkylsulfonylamino,
Z⁵ steht für Tri(C₂-C₄-alkyl)silyl, Benzyl, Phenyl, SH, C₅-C₆-Alkoxy, C₅-C₆-Halogenalkoxy, C₂-C₆-Alkenyloxy, C₂-C₆-Alkinyloxy, C₅-C₆-Alkylthio oder C₅-C₆-Halogenalkylthio,
Z⁶ steht für
Substituenten am Kohlenstoff: SH, Cycloalkylcycloalkyl oder Tri(C₃-C₄-alkyl)silyl,
Substituenten am Stickstoff: Alkenyl, Alkinyl, Halogenalkyl, Halogenalkenyl, Halogenalkinyl, Cycloalkyl, Halogencycloalkyl, Alkylcycloalkyl, Cycloalkylalkyl, Cycloalkylcycloalkyl, Alkylsulfonyl, C(=O)H, Benzyl oder Phenyl,
Z⁷ steht für Cyano, Halogen, Hydroxy, Oxo, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio oder Phenyl,
L³ steht für eine direkte Bindung, -CH₂-, -C(=O)-, Schwefel, Sauerstoff, -C(=O)O-, -C(=O)NH-, -OC(=O)- oder -NHC(=O)-,
R³ und R⁴ stehen gleich oder verschieden und unabhängig voneinander für Wasserstoff, Alkyl, Alkenyl, Alkinyl, Halogenalkyl, Cycloalkyl, Benzyl oder Phenyl,
sowie Salze, Metallkomplexe und N-Oxide der Verbindungen der Formel **(I).**

2. Verbindungen der Formel (I) gemäß Anspruch 1, dadurch gekennzeichent, dass
Y für Sauerstoff oder Schwefel steht,
R² für Wasserstoff oder Halogen steht,
Q für Q-1, Q-2, Q-3, Q-4, Q-5 und Q-6, wobei die Bindung steht, die mit "x" identifiziert ist, direkt an das Thiazol gebunden ist, und wobei die Bindung, die mit "y" identifiziert ist, direkt an L¹ bzw. R¹ gebunden ist,
R⁵ für Wasserstoff, Cyano, Methyl, Ethyl, Trifluomethyl oder Difluormethyl steht,
L¹ für eine direkte Bindung oder Sauerstoff steht,
R¹ für Phenyl steht, das mindestens einen Substituenten Z⁴ enthält und zusätzlich zwei oder drei weitere Substituenten, welche unabhängig voneinander ausgewählt sind aus Z⁴ und Z¹, enthält, oder
R¹ für Naphthyl, Dihydronaphthalenyl, Tetrahydronaphthalenyl, Hexahydronaphthalenyl, Octahydronaphthalenyl oder Indenyl steht, das mindestens einen Substituenten Z⁵ enthält und zusätzlich zwei oder drei weitere Substituenten, welche unabhängig voneinander ausgewählt sind aus Z⁵ und Z¹, enthält, oder
R¹ für ein gegebenenfalls benzokondensiertes, substituiertes 5- oder 6-gliedriges Heteroaryl steht, das mindestens einen Substituenten Z⁶ enthält und zusätzlich zwei oder drei weitere Substituenten, welche am Kohlenstoff unabhängig voneinander aus Z⁶ und Z¹ ausgewählt sind und am Stickstoff unabhängig voneinander ausgewählt sind aus Z⁶ und Z², enthält, oder
R¹ für ein C₃-C₈-Cycloalkyl oder für ein C₅-C₈-Cycloalkenyl steht, das mindestens einen Substituenten Z⁷ enthält und zusätzlich zwei oder drei weitere Substituenten, welche unabhängig voneinander ausgewählt sind aus Z⁷ und Z¹, enthält, oder
Z² für Wasserstoff, Halogen, Cyano, Hydroxy, Nitro, -C(=O)NR³R⁴, -NR³R⁴, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₃-C₈-Halogencycloalkyl, C₁-C₆-Alkoxy-C₁-C₆-alkyl, C₁-C₆-Alkoxycarbonyl, C₁-C₄-Alkoxy, C₁-C₄-Halogenalkoxy, C₁-C₆-Alkylcarbonyloxy, C₁-C₄- Alkylthio, C₁-C₄-Halogenalkylthio, C₃-C₆-Cycloalkylthio oder Tri(C₁-C₂-alkyl)silyl steht,
Z³ für einen Phenylrest, Naphthalenyl oder einen 5- oder 6-gliedrigen Heteroarylrest steht, der bis zu zwei Substituenten enthalten kann, wobei die Substituenten unabhängig voneinander ausgewählt sind aus folgender Liste:
Substituenten am Kohlenstoff: Halogen, Cyano, Nitro, Hydroxy, Amino, -SH, C₁-C₄-Alkyl, C₂-C₄-Alkenyl, C₂-C₄-Alkinyl, C₁-C₄-Halogenalkyl, C₂-C₄-Halogenalkenyl, C₂-C₄-Halogenalkinyl, C₂-C₄-Alkoxyalkyl, C₁-C₆-Alkylcarbonyl, C₁-C₆-Halogenalkylcarbonyl, C₁-C₆-Alkoxycarbonyl, C₁-C₄-Alkoxy, C₁-C₄-Halogenalkoxy, C₂-C₆-Alkenyloxy, C₂-C₆-Alkinyloxy, C₁-C₄-Alkylthio, C₁-C₄-Halogenalkylthio, C₁-C₄-Alkylsulfonyl, C₁-C₄-Halogenalkylsulfonyl oder C₁-C₄-Alkylamino, Di(C₁-C₄-alkyl)amino,
Substituenten am Stickstoff: C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₁-C₄-Alkoxy-C₁-C₄-alkyl, Phenyl, Benzyl, C₁-C₄-Halogenalkylsulfonyl, C₁-C₆-Alkoxycarbonyl, C₁-C₆-Halogenalkoxycarbonyl, Phenylsulfonyl, C₁-C₄-Alkylsulfonyl, -C(=O)H, oder C₁-C₃-Alkylcarbonyl, und
Z⁴ für C(=O)H, C₇-C₈-Cycloalkyl, C₃-C₆-Cycloalkyl-C₃-C₆-cycloalkyl, C₃-C₆-Cycloalkenyl, C₄-C₆-Alkoxy-C₁-C₄-alkyl, C₃-C₆-Alkoxy-C₂-C₄-alkyl, C₁-C₃-Alkoxy-C₅-C₆-alkoxy-C₁-C₃-alkyl, C₁-C₄-Alkylthio-C₁-C₂-alkyl, C₁-C₄-Alkylsulfinyl-C₁-C₂-alkyl, C₁-C₄-Alkylsulfonyl-C₁-C₂-alkyl, C₁-C₄-Alkylamino-C₁-C₂-alkyl, C₁-C₂-Dialkylamino-C₁-C₂-alkyl, C₁-C₄-Halogenalkylamino-C₁-C₂-alkyl, C₃-C₆-Cycloalkylamino-C₁-C₂-alkyl, C₅-C₆-Alkylcarbonyl, C₁-C₄-Halogenalkylcarbonyl, C₃-C₆-Cycloalkylcarbonyl, C₃-C₆-Cycloalkoxycarbonyl, C₃-C₆-Cycloalkyl-C₁-C₂-alkoxycarbonyl, C₃-C₆-Cy-cloalkylaminocarbonyl, C₁-C₄-Halogenalkoxy-C₁-C₂-alkyl, C₅-C₆-Hydroxyalkyl, C₅-C₆-Alkoxy, C₅-C₆-Halogenalkoxy, C₃-C₆-Cycloalkoxy, C₃-C₆-Halogencycloalkoxy, C₃-C₆-Cycloalkylalkoxy, C₂-C₆-Alkenyloxy, C₂-C₆-Halogenalkenyloxy, C₂-C₆-Alkinyloxy, C₂-C₆-Halogenalkinyloxy, C₁-C₄-Alkoxy-C₁-C₄-alkoxy, C₁-C₄-Halogenalkylcarbonyloxy, C₃-C₆-Cycloalkylcarbonyloxy, C₁-C₄-Alkylcarbonyl-C₁-C₄-alkoxy, C₅-C₆-Alkylthio, C₅-C₆-Halogenalkylthio, C₅-C₆-Alkylsulfinyl, C₅-C₆-Halogenalkylsulfinyl, C₁-C₄-Alkyl-sulfonyl, C₁-C₄-Halogenalkylsulfonyl, C₃-C₆-Cycloalkylsulfonyl, Tri(C₃-C₄-alkyl)silyl, C₁-C₄-Alkylsulfonylamino oder C₁-C₄-Halogenalkylsulfonylamino steht,
Z⁵ für Benzyl, Phenyl, C₅-C₆-Alkoxy, C₅-C₆-Halogenalkoxy, C₂-C₄-Alkenyloxy, C₂-C₄-Alkinyloxy, C₅-C₆-Alkylthio oder C₅-C₆-Halogenalkylthio steht,
Z⁶ für
Substituenten am Kohlenstoff: C₃-C₆-Cycloalkylcyclopropyl, C₃-C₆-Cycloalkylcyclohexyl oder Tri(C₃-C₄-alkyl)silyl steht,
Substituenten am Stickstoff: C₂-C₆-Alkenyl, C₂-C₆-Alkinyl, C₁-C₆-Halogenalkyl, C₃-C₆-Cycloalkyl, C₁-C₄-Alkyl-C₃-C₆-cycloalkyl, C₃-C₆-Cycloalkylcyclopropyl, C₃-C₆-Cycloalkylcyclohexyl, C₃-C₆-Cycloalkyl-C₁-C₄-alkyl, C₁-C₄-Alkylsulfonyl, C(=O)H, Benzyl oder Phenyl steht,
L³ für eine direkte Bindung, -CH₂-, Schwefel, Sauerstoff steht,
R³ und R⁴ stehen gleich oder verschieden und unabhängig voneinander für Wasserstoff, C₁-C₄-Alkyl, C₂-C₄-Alkenyl, C₂-C₄-Alkinyl, C₁-C₄-Halogenalkyl, C₅-C₆-Cycloalkyl, Benzyl oder Phenyl.

3. Verbindungen der Formel (I) gemäß Anspruch 1, **dadurch gekennzeichnet, dass**
Y für Sauerstoff steht;
R² für Wasserstoff steht;
Q für Q-3 steht;
R⁵ für Wasserstoff steht;
L¹ für eine direkte Bindung steht;
R¹ für 4,5-Dimethyl-2-(prop-2-in-1-yloxy)phenyl steht oder,
R¹ für 2,6-Difluor-3-(prop-2-in-1-yloxy)phenyl steht oder,
R¹ für 2,6-Difluor-4-(prop-2-in-1-yloxy)phenyl steht oder,
R¹ für 2,6-Difluor-4-[(methylsulfonyl)amino]phenyl steht oder,
R¹ für 3-(Allyloxy)-2,6-difluorphenyl steht.

4. Verfahren zur Bekämpfung von pflanzenpathogenen Schadpilzen, **dadurch gekennzeichnet, dass** man Verbindungen der Formel (I) gemäß Anspruch 1 auf die pflanzenpathogenen Schadpilze und/oder deren Lebensraum ausbringt.

5. Mittel zur Bekämpfung von pflanzenpathogenen Schadpilzen, **gekennzeichnet durch** einen Gehalt an mindestens einer Verbindung der Formel (I) gemäß Anspruch 1, neben Streckmitteln und/oder oberflächenaktiven Stoffen.

6. Verwendung von Verbindungen der Formel (I), gemäß Anspruch 1 zur Bekämpfung von pflanzenpathogenen Schadpilzen in der Land-, Garten- und Forstwirtschaft, im Materialschutz sowie im Bereich Haushalt und Hygiene.

7. Verfahren zur Herstellung von Mitteln zur Bekämpfung von pflanzenpathogenen Schadpilzen, **dadurch gekennzeichnet, dass** Verbindungen der Formel (I) gemäß Anspruch 1 mit Streckmitteln und/oder oberflächenaktiven Stoffen vermischt werden.

8. Verwendung von Verbindungen der Formel (I) gemäß Anspruch 1 zur Behandlung von transgenen Pflanzen.

9. Verwendung von Verbindungen der Formel (I) gemäß Anspruch 1 zur Behandlung von Saatgut sowie von Saatgut transgener Pflanzen.

## Claims

1. Compounds of the formula **(I),** 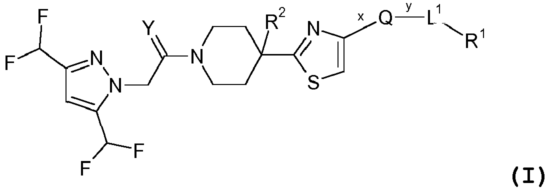 in which the definitions of radicals have the following meanings:
Y is oxygen or sulphur,
R² is hydrogen or halogen
Q is 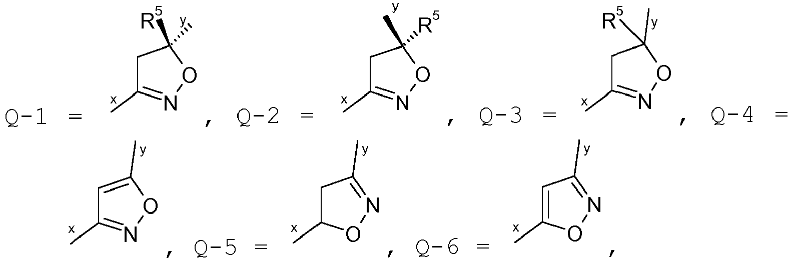
where the bond identified by "x" is bonded directly to the thiazole and the bond identified by "y" is bonded directly to L¹ or R¹,
R⁵ is hydrogen, cyano, C₁-C₃ alkyl or C₁-C₃ haloalkyl,
L¹ is a direct bond, -CH₂-, -(C=O)-, sulphur or oxygen,
R¹ is phenyl which contains at least one substituent Z⁴ and additionally two, three or four further substituents which independently are selected from Z⁴ and Z¹,
or
R¹ is naphthyl, dihydronaphthalenyl, tetrahydronaphthalenyl, hexahydronaphthalenyl, octahydronaphthalenyl or indenyl which contains at least one substituent Z⁵ and additionally contains two, three or four further further substituents which independently are selected from Z⁵ and Z¹,
or
R¹ is an optionally benzo-fused, substituted 5- or 6-membered heteroaryl which contains at least one substituent Z⁶ and additionally contains two, three or four further substituents which on the carbon are selected independently of one another from Z⁶ and Z¹ and on the nitrogen are selected independently of one another from Z⁶ and Z²,
or
R¹ is a C₃-C₈ cycloalkyl or is a C₅-C₈ cycloalkenyl which contains at least one substituent Z⁷ and additionally contains two, three or four further substituents which independently of one another are selected from Z⁷ and Z¹,
Z¹ is halogen, hydroxyl, nitro, cyano, alkyl, alkenyl, alkynyl, haloalkyl, haloalkenyl, haloalkynyl, cycloalkyl, halocycloalkyl, alkoxy, alkoxyalkyl, hydroxyalkyl, haloalkoxy, alkylcarbonyl, alkoxycarbonyl, alkylcycloalkyl, cycloalkoxyalkyl, cycloalkylamino, alkylthio, haloalkylthio, cycloalkylthio, cycloalkylalkyl, alkylcarbonyloxy, alkylcarbonylamino, haloalkyl-carbonylamino, alkylcarbonylthio, alkylsulphinyl, haloalkylsulphinyl, alkylsulphonyloxy, haloalkyl-sulphonyloxy, alkylcycloalkylalkyl, -C(=O)NR³R⁴,-NR³R⁴, tri(C₁-C₂ alkyl)silyl, or -L³Z³,
Z² is hydrogen, halogen, hydroxyl, nitro, cyano, alkyl, alkenyl, alkynyl, haloalkyl, haloalkenyl, haloalkynyl, cycloalkyl, halocycloalkyl, alkoxy, alkoxyalkyl, hydroxyalkyl, haloalkoxy, alkylcarbonyl, alkoxycarbonyl, alkylcycloalkyl, cycloalkoxyalkyl, cycloalkylamino, alkylthio, haloalkylthio, cycloalkylthio, cycloalkylalkyl, alkylcarbonyloxy, alkylcarbonylamino, haloalkylcarbonylamino, alkylcarbonylthio, alkylsulphinyl, haloalkylsulphinyl, alkylsulphonyloxy, haloalkylsulphonyloxy, alkylcycloalkylalkyl, -C(=O)NR³R⁴, -NR³R⁴ or tri(C₁-C₂ alkyl)silyl,
Z³ is a phenyl radical, naphthalenyl radical or a 5- or 6-membered heteroaryl radical which in each case may contain 0, 1, 2 or 3 substituents, the substituents being selected independently of one another from the following list:
Substituents on the carbon: halogen, cyano, nitro, hydroxyl, amino, -SH, alkyl, alkenyl, alkynyl, haloalkyl, haloalkenyl, haloalkynyl, cycloalkyl, halocycloalkyl, alkoxyalkyl, alkylcarbonyl, haloalkylcarbonyl, alkoxycarbonyl, alkoxy, haloalkoxy, cycloalkoxy, halocycloalkoxy, alkenyloxy, alkynyloxy, alkoxyalkoxy, alkylamino, dialkylamino, alkylthio, haloalkylthio, alkylsulphinyl, haloalkylsulphinyl, alkylsulphonyl, haloalkylsulphonyl, trisilylalkyl or phenyl,
Substituents on the nitrogen: hydrogen, -C(=O)H, alkyl, alkenyl, alkynyl, haloalkyl, haloalkenyl, haloalkynyl, cycloalkyl, halocycloalkyl, alkylcycloalkyl, cycloalkylalkyl, alkoxyalkyl, alkylsulphonyl, haloalkylsulphonyl, cycloalkylsulphonyl, phenylsulphonyl, alkylcarbonyl, haloalkylcarbonyl, alkoxycarbonyl, haloalkoxycarbonyl, cycloalkoxycarbonyl, -C(=O)NR³R⁴, phenyl or benzyl,
Z⁴ is SH, C(=O)H, C₇-C₈ cycloalkyl, C₇-C₈ halocycloalkyl, cycloalkylcycloalkyl, halocycloalkylalkyl, cycloalkenyl, halocycloalkenyl, C₅-C₆ alkoxyalkyl, alkoxyalkoxyalkyl, alkylthioalkyl, alkylsulphinylalkyl, alkylsulphonylalkyl, alkylaminoalkyl, dialkylaminoalkyl, haloalkylaminoalkyl, cycloalkylaminoalkyl, C₅-C₆ alkylcarbonyl, haloalkylcarbonyl, cycloalkylcarbonyl, cycloalkoxycarbonyl, cycloalkylalkoxycarbonyl, cycloalkylaminocarbonyl, haloalkoxyalkyl, C₅-C₆ hydroxyalkyl, C₅-C₆ alkoxy, C₅-C₆ haloalkoxy, cycloalkoxy, halocycloalkoxy, cycloalkylalkoxy, alkenyloxy, haloalkenyloxy, alkynyloxy, haloalkynyloxy, alkoxyalkoxy, haloalkylcarbonyloxy, cycloalkylcarbonyloxy, alkylcarbonylalkoxy, C₅-C₆ alkylthio, C₅-C₆ haloalkylthio, C₅-C₆ alkylsulphinyl, C₅-C₆ haloalkylsulphinyl, alkylsulphonyl, C₁-C₄ haloalkylsulphonyl, cycloalkylsulphonyl, tri(C₃-C₄ alkyl)silyl, alkylsulphonylamino or haloalkylsulphonylamino,
Z⁵ is tri(C₂-C₄ alkyl)silyl, benzyl, phenyl, SH, C₅-C₆ alkoxy, C₅-C₆ haloalkoxy, C₂-C₆ alkenyloxy, C₂-C₆ alkynyloxy, C₅-C₆ alkylthio or C₅-C₆ haloalkylthio,
Z⁶ is
Substituents on the carbon: SH, cycloalkylcycloalkyl or tri(C₃-C₄ alkyl)silyl,
Substituents on the nitrogen: alkenyl, alkynyl, haloalkyl, haloalkenyl, haloalkynyl, cycloalkyl, halocycloalkyl, alkylcycloalkyl, cycloalkylalkyl, cycloalkylcycloalkyl, alkylsulphonyl, C(=O)H, benzyl or phenyl,
Z⁷ is cyano, halogen, hydroxyl, oxo, C₁-C₄ alkyl, C₁-C₄ haloalkyl, C₁-C₄ alkoxy, C₁-C₄ haloalkoxy, C₁-C₄ alkylthio, C₁-C₄ haloalkylthio or phenyl,
L³ is a direct bond, -CH₂-, -C(=O)-, sulphur, oxygen, -C(=O)O-, -C(=O)NH-, -OC(=O)- or -NHC(=O)-,
R³ and R⁴, identically or differently and independently of one another, are hydrogen, alkyl, alkenyl, alkynyl, haloalkyl, cycloalkyl, benzyl or phenyl, and also salts, metal complexes and N-oxides of the compounds of the formula **(I).**

2. Compounds of the formula (I) according to Claim 1, **characterized in that**
Y is oxygen or sulphur,
R² is hydrogen or halogen,
Q is Q-1, Q-2, Q-3, Q-4, Q-5 and Q-6, where the bond identified by "x" is bonded directly to the thiazole and the bond identified by "y" is bonded directly to L¹ or R¹,
R⁵ is hydrogen, cyano, methyl, ethyl, trifluoromethyl or difluoromethyl,
L¹ is a direct bond or oxygen,
R¹ is phenyl which contains at least one substituent Z⁴ and additionally contains two or three further substituents which independently of one another are selected from Z⁴ and Z¹, or
R¹ is naphthyl, dihydronaphthalenyl, tetrahydronaphthalenyl, hexahydronaphthalenyl, octahydronaphthalenyl or indenyl which contains at least one substituent Z⁵ and additionally contains two or three further substituents which independently of one another are selected from Z⁵ and Z¹, or
R¹ is an optionally benzo-fused, substituted 5- or 6-membered heteroaryl which contains at least one substituent Z⁶ and additionally contains two or three further substituents which on the carbon are selected independently of one another from Z⁶ and Z¹ and on the nitrogen are selected independently of one another from Z⁶ and Z², or
R¹ is a C₃-C₈ cycloalkyl or is a C₅-C₈ cycloalkenyl which contains at least one substituent Z⁷ and additionally contains two or three further substituents which independently of one another are selected from Z⁷ and Z¹, or
Z² is hydrogen, halogen, cyano, hydroxyl, nitro, -C(=O)NR³R⁴, -NR³R⁴, C₁-C₆ alkyl, C₂-C₆ alkenyl, C₂-C₆ alkynyl, C₁-C₆ haloalkyl, C₂-C₆ haloalkenyl, C₂-C₆ haloalkynyl, C₃-C₆ cycloalkyl, C₃-C₈ halocycloalkyl, C₁-C₆ alkoxy-C₁-C₆ alkyl, C₁-C₆ alkoxycarbonyl, C₁-C₄ alkoxy, C₁-C₄ haloalkoxy, C₁-C₆ alkylcarbonyloxy, C₁-C₄ alkylthio, C₁-C₄ haloalkylthio, C₃-C₆ cycloalkylthio or tri(C₁-C₂ alkyl)silyl,
Z³ is a phenyl radical, naphthalenyl or a 5- or 6-membered heteroaryl radical which may contain up to two substituents, the substituents being selected independently of one another from the following list:
Substituents on the carbon: halogen, cyano, nitro, hydroxyl, amino, -SH, C₁-C₄ alkyl, C₂-C₄ alkenyl, C₂-C₄ alkynyl, C₁-C₄ haloalkyl, C₂-C₄ haloalkenyl, C₂-C₄ haloalkynyl, C₂-C₄ alkoxyalkyl, C₁-C₆ alkylcarbonyl, C₁-C₆ haloalkylcarbonyl, C₁-C₆ alkoxycarbonyl, C₁-C₄ alkoxy, C₁-C₄ haloalkoxy, C₂-C₆ alkenyloxy, C₂-C₆ alkynyloxy, C₁-C₄ alkylthio, C₁-C₄ haloalkylthio, C₁-C₄ alkylsulphonyl, C₁-C₄ haloalkylsulphonyl or C₁-C₄ alkylamino, di(C₁-C₄ alkyl)amino,
Substituents on the nitrogen: C₁-C₆ alkyl, C₂-C₆ alkenyl, C₂-C₆ alkynyl, C₁-C₆ haloalkyl, C₂-C₆ haloalkenyl, C₂-C₆ haloalkynyl, C₁-C₄ alkoxy-C₁-C₄ alkyl, phenyl, benzyl, C₁-C₄ haloalkylsulphonyl, C₁-C₆ alkoxycarbonyl, C₁-C₆ haloalkoxycarbonyl, phenylsulphonyl, C₁-C₄ alkylsulphonyl, -C(=O)H, or C₁-C₃ alkylcarbonyl, and
Z⁴ is C(=O)H, C₇-C₈ cycloalkyl, C₃-C₆ cycloalkyl-C₃-C₆-cycloalkyl, C₃-C₆ cycloalkenyl, C₄-C₆ alkoxy-C₁-C₄ alkyl, C₃-C₆ alkoxy-C₂-C₄ alkyl, C₁-C₃ alkoxy-C₅-C₆ alkoxy-C₁-C₃ alkyl, C₁-C₄ alkylthio-C₁-C₂ alkyl, C₁-C₄ alkylsulphinyl-C₁-C₂ alkyl, C₁-C₄ alkylsulphonyl-C₁-C₂ alkyl, C₁-C₄ alkylamino-C₁-C₂ alkyl, C₁-C₂-dialkylamino-C₁-C₂ alkyl, C₁-C₄ haloalkylamino-C₁-C₂ alkyl, C₃-C₆ cycloalkylamino-C₁-C₂ alkyl, C₅-C₆ alkylcarbonyl, C₁-C₄ haloalkylcarbonyl, C₃-C₆ cycloalkylcarbonyl, C₃-C₆ cycloalkoxycarbonyl, C₃-C₆ cycloalkyl-C₁-C₂ alkoxycarbonyl, C₃-C₆ cycloalkylaminocarbonyl, C₁-C₄ haloalkoxy-C₁-C₂ alkyl, C₅-C₆-hydroxyalkyl, C₅-C₆ alkoxy, C₅-C₆ haloalkoxy, C₃-C₆ cycloalkoxy, C₃-C₆ halocycloalkoxy, C₃-C₆ cycloalkylalkoxy, C₂-C₆ alkenyloxy, C₂-C₆ haloalkenyloxy, C₂-C₆ alkynyloxy, C₂-C₆ haloalkynyloxy, C₁-C₄ alkoxy-C₁-C₄ alkoxy, C₁-C₄ haloalkylcarbonyloxy, C₃-C₆ cycloalkylcarbonyloxy, C₁-C₄ alkylcarbonyl-C₁-C₄ alkoxy, C₅-C₆ alkylthio, C₅-C₆ haloalkylthio, C₅-C₆ alkylsulphinyl, C₅-C₆ haloalkylsulphinyl, C₁-C₄ alkylsulphonyl, C₁-C₄ haloalkylsulphonyl, C₃-C₆ cycloalkylsulphonyl, tri(C₃-C₄ alkyl)silyl, C₁-C₄ alkylsulphonylamino or C₁-C₄ haloalkylsulphonylamino,
Z⁵ is benzyl, phenyl, C₅-C₆ alkoxy, C₅-C₆ haloalkoxy, C₂-C₄ alkenyloxy, C₂-C₄ alkynyloxy, C₅-C₆ alkylthio or C₅-C₆ haloalkylthio,
Z⁶ is
Substituents on the carbon: C₃-C₆ cycloalkylcyclopropyl, C₃-C₆ cycloalkylcyclohexyl or tri(C₃-C₄ alkyl)silyl,
Substituents on the nitrogen: C₂-C₆ alkenyl, C₂-C₆ alkynyl, C₁-C₆ haloalkyl, C₃-C₆ cycloalkyl, C₁-C₄ alkyl-C₃-C₆-cycloalkyl, C₃-C₆ cycloalkylcyclopropyl, C₃-C₆ cycloalkylcyclohexyl, C₃-C₆ cycloalkyl-C₁-C₄ alkyl, C₁-C₄ alkylsulphonyl, C(=O)H, benzyl or phenyl,
L³ is a direct bond, -CH₂-, sulphur or oxygen,
R³ and R⁴, identically or differently and independently of one another, are hydrogen, C₁-C₄ alkyl, C₂-C₄ alkenyl, C₂-C₄ alkynyl, C₁-C₄ haloalkyl, C₅-C₆ cycloalkyl, benzyl or phenyl.

3. Compounds of the formula (I) according to Claim 1, **characterized in that**
Y is oxygen;
R² is hydrogen;
Q is Q-3;
R⁵ is hydrogen;
L¹ is a direct bond;
R¹ is 4,5-dimethyl-2-(prop-2-yn-1-yloxy)phenyl or
R¹ is 2,6-difluoro-3-(prop-2-yn-1-yloxy)phenyl or
R¹ is 2,6-difluoro-4-(prop-2-yn-1-yloxy)phenyl or
R¹ is 2,6-difluoro-4-[(methylsulphonyl)amino]phenyl or
R¹ is 3-(allyloxy)-2,6-difluorophenyl.

4. Method for controlling phytopathogenic harmful fungi, **characterized in that** compounds of the formula (I) according to Claim 1 are delivered to the phytopathogenic harmful fungi and/or their habitat.

5. Composition for controlling phytopathogenic harmful fungi, **characterized by** the presence therein of at least one compound of the formula (I) according to Claim 1, in addition to extenders and/or surfactants.

6. Use of compounds of the formula (I) according to Claim 1 for controlling phytopathogenic harmful fungi in agriculture, horticulture and forestry, in materials protection and in the household and hygiene sector.

7. Method for producing compositions for controlling phytopathogenic harmful fungi, **characterized in that** compounds of the formula (I) according to Claim 1 are mixed with extenders and/or surfactants.

8. Use of compounds of the formula (I) according to Claim 1 for treating transgenic plants.

9. Use of compounds of the formula (I) according to Claim 1 for treating seed and also seed of transgenic plants.

## Revendications

1. Composés de formule (I) 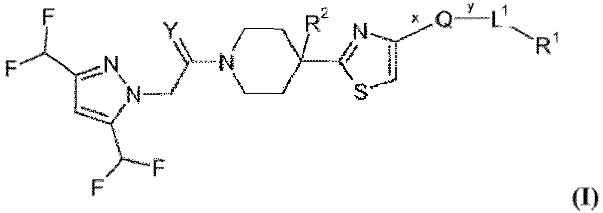 dans laquelle les radicaux ont les significations suivantes :
Y représente oxygène ou soufre,
R² représente hydrogène ou halogène,
Q représente 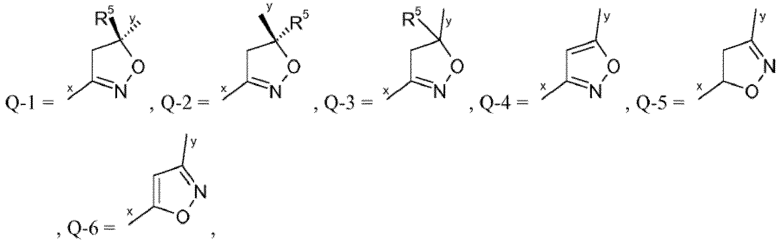
la liaison identifiée par « x » étant reliée directement au thiazole et la liaison identifiée par « y » étant reliée directement à L¹ ou R¹,
R⁵ représente hydrogène, cyano, alkyle en C₁-C₃ ou halogénoalkyle en C₁-C₃,
L¹ représente une liaison directe, -CH₂-, - (C=O) -, soufre ou oxygène,
R¹ représente phényle, qui contient au moins un substituant Z⁴ et qui contient en outre deux, trois ou quatre substituants supplémentaires, qui sont choisis indépendamment parmi Z⁴ et Z¹,
ou
R¹ représente naphtyle, dihydronaphtalényle, tétrahydronaphtalényle, hexahydronaphtalényle, octahydronaphtalényle ou indényle, qui contient au moins un substituant Z⁵ et qui contient en outre deux, trois ou quatre substituants supplémentaires, qui sont choisis indépendamment parmi Z⁵ et Z¹,
ou
R¹ représente un hétéroaryle à 5 ou 6 chaînons, substitué, éventuellement benzocondensé, qui contient au moins un substituant Z⁶ et qui contient en outre deux, trois ou quatre substituants supplémentaires, qui sont choisis indépendamment les uns des autres sur le carbone parmi Z⁶ et Z¹ et qui sont choisis indépendamment les uns des autres sur l'azote parmi Z⁶ et Z²,
ou
R¹ représente un cycloalkyle en C₃-C₈ ou un cycloalcényle en C₅-C₈, qui contient au moins un substituant Z⁷ et qui contient en outre deux, trois ou quatre substituants supplémentaires, qui sont choisis indépendamment les uns des autres parmi Z⁷ et Z¹,
Z¹ représente halogène, hydroxy, nitro, cyano, alkyle, alcényle, alcynyle, halogénoalkyle, halogénoalcényle, halogénoalcynyle, cycloalkyle, halogénocycloalkyle, alcoxy, alcoxyalkyle, hydroxyalkyle, halogénoalcoxy, alkylcarbonyle, alcoxycarbonyle, alkylcycloalkyle, cycloalcoxyalkyle, cycloalkylamino, alkylthio, halogénoalkylthio, cycloalkylthio, cycloalkylalkyle, alkylcarbonyloxy, alkylcarbonylamino, halogénoalkylcarbonylamino, alkylcarbonylthio, alkylsulfinyle, halogénoalkylsulfinyle, alkylsulfonyloxy, halogénoalkylsulfonyloxy, alkylcycloalkylalkyle,-C(=O)NR³R⁴, -NR³R⁴, tri(alkyle en C₁-C₂)silyle ou -L³Z³,
Z² représente hydrogène, halogène, hydroxy, nitro, cyano, alkyle, alcényle, alcynyle, halogénoalkyle, halogénoalcényle, halogénoalcynyle, cycloalkyle, halogénocycloalkyle, alcoxy, alcoxyalkyle, hydroxyalkyle, halogénoalcoxy, alkylcarbonyle, alcoxycarbonyle, alkylcycloalkyle, cycloalcoxyalkyle, cycloalkylamino, alkylthio, halogénoalkylthio, cycloalkylthio, cycloalkylalkyle, alkylcarbonyloxy, alkylcarbonylamino, halogénoalkylcarbonylamino, alkylcarbonylthio, alkylsulfinyle, halogénoalkylsulfinyle, alkylsulfonyloxy, halogénoalkylsulfonyloxy, alkylcycloalkylalkyle,-C(=O)NR³R⁴, -NR³R⁴ ou tri (alkyle en C₁-C₂)silyle,
Z³ représente un radical phényle, un radical naphtalényle ou un radical hétéroaryle à 5 ou 6 chaînons, qui peuvent chacun contenir 0, 1, 2 ou 3 substituants, les substituants étant choisis indépendamment les uns des autres dans la liste suivante :
substituants sur le carbone : halogène, cyano, nitro, hydroxy, amino, -SH, alkyle, alcényle, alcynyle, halogénoalkyle, halogénoalcényle, halogénoalcynyle, cycloalkyle, halogénocycloalkyle, alcoxyalkyle, alkylcarbonyle, halogénoalkylcarbonyle, alcoxycarbonyle, alcoxy, halogénoalcoxy, cycloalcoxy, halogénocycloalcoxy, alcényloxy, alcynyloxy, alcoxyalcoxy, alkylamino, dialkylamino, alkylthio, halogénoalkylthio, alkylsulfinyle, halogénoalkylsulfinyle, alkylsulfonyle, halogénoalkylsulfonyle, trisilylalkyle ou phényle,
substituants sur l'azote : hydrogène, -C(=O)H, alkyle, alcényle, alcynyle, halogénoalkyle, halogénoalcényle, halogénoalcynyle, cycloalkyle, halogénocycloalkyle, alkylcycloalkyle, cycloalkylalkyle, alcoxyalkyle, alkylsulfonyle, halogénoalkylsulfonyle, cycloalkylsulfonyle, phénylsulfonyle, alkylcarbonyle, halogénoalkylcarbonyle, alcoxycarbonyle, halogénoalcoxycarbonyle, cycloalcoxycarbonyle,-C(=O)NR³R⁴, phényle ou benzyle,
Z⁴ représente SH, C(=O)H, cycloalkyle en C₇-C₈, halogénocycloalkyle en C₇-C₈, cycloalkylcycloalkyle, halogénocycloalkylalkyle, cycloalcényle, halogénocycloalcényle, alcoxyalkyle en C₅-C₆, alcoxyalcoxyalkyle, alkylthioalkyle, alkylsulfinylalkyle, alkylsulfonylalkyle, alkylaminoalkyle, dialkylaminoalkyle, halogénoalkylaminoalkyle, cycloalkylaminoalkyle, alkylcarbonyle en C₅-C₆, halogénoalkylcarbonyle, cycloalkylcarbonyle, cycloalcoxycarbonyle, cycloalkylalcoxycarbonyle, cycloalkylaminocarbonyle, halogénoalcoxyalkyle, hydroxyalkyle en C₅-C₆, alcoxy en C₅-C₆, halogénoalcoxy en C₅-C₆, cycloalcoxy, halogénocycloalcoxy, cycloalkylalcoxy, alcényloxy, halogénoalcényloxy, alcynyloxy, halogénoalcynyloxy, alcoxyalcoxy, halogénoalkylcarbonyloxy, cycloalkylcarbonyloxy, alkylcarbonylalcoxy, alkylthio en C₅-C₆, halogénoalkylthio en C₅-C₆, alkylsulfinyle en C₅-C₆, halogénoalkylsulfinyle en C₅-C₆, alkylsulfonyle, halogénoalkylsulfonyle en C₁-C₄, cycloalkylsulfonyle, tri(alkyle en C₃-C₄)silyle, alkylsulfonylamino ou halogénoalkylsulfonylamino,
Z⁵ représente tri(alkyle en C₂-C₄)silyle, benzyle, phényle, SH, alcoxy en C₅-C₆, halogénoalcoxy en C₅-C₆, alcényloxy en C₂-C₆, alcynyloxy en C₂-C₆, alkylthio en C₅-C₆ ou halogénoalkylthio en C₅-C₆,
Z⁶ représente
substituants sur le carbone : SH, cycloalkylcycloalkyle ou tri (alkyle en C₃-C₄) silyle,
substituants sur l'azote : alcényle, alcynyle, halogénoalkyle, halogénoalcényle, halogénoalcynyle, cycloalkyle, halogénocycloalkyle, alkylcycloalkyle, cycloalkylalkyle, cycloalkylcycloalkyle, alkylsulfonyle, C(=O)H, benzyle ou phényle,
Z⁷ représente cyano, halogène, hydroxy, oxo, alkyle en C₁-C₄, halogénoalkyle en C₁-C₄, alcoxy en C₁-C₄, halogénoalcoxy en C₁-C₄, alkylthio en C₁-C₄, halogénoalkylthio en C₁-C₄ ou phényle,
L³ représente une liaison directe, -CH₂-, -C(=O)-, soufre, oxygène, -C(=O)O-, -C(=O)NH- -OC(=O)- ou-NHC(=O)-,
R³ et R⁴ sont identiques ou différents, et représentent indépendamment l'un de l'autre hydrogène, alkyle, alcényle, alcynyle, halogénoalkyle, cycloalkyle, benzyle ou phényle,
ainsi que les sels, complexes métalliques et N-oxydes des composés de formule (I).

2. Composés de formule (I) selon la revendication 1, **caractérisés en ce que**
Y représente oxygène ou soufre,
R² représente hydrogène ou halogène,
Q représente Q-1, Q-2, Q-3, Q-4, Q-5 et Q-6, la liaison identifiée par « x » étant reliée directement au thiazole et la liaison identifiée par « y » étant reliée directement à L¹ ou R¹,
R⁵ représente hydrogène, cyano, méthyle, éthyle, trifluorométhyle ou difluorométhyle,
L¹ représente une liaison directe ou oxygène,
R¹ représente phényle, qui contient au moins un substituant Z⁴ et qui contient en outre deux ou trois substituants supplémentaires, qui sont choisis indépendamment les uns des autres parmi Z⁴ et Z¹, ou
R¹ représente naphtyle, dihydronaphtalényle, tétrahydronaphtalényle, hexahydronaphtalényle, octahydronaphtalényle ou indényle, qui contient au moins un substituant Z⁵ et qui contient en outre deux ou trois substituants supplémentaires, qui sont choisis indépendamment les uns des autres parmi Z⁵ et Z¹, ou
R¹ représente un hétéroaryle à 5 ou 6 chaînons, substitué, éventuellement benzocondensé, qui contient au moins un substituant Z⁶ et qui contient en outre deux ou trois substituants supplémentaires, qui sont choisis indépendamment les uns des autres sur le carbone parmi Z⁶ et Z¹ et qui sont choisis indépendamment les uns des autres sur l'azote parmi Z⁶ et Z², ou
R¹ représente un cycloalkyle en C₃-C₈ ou un cycloalcényle en C₅-C₈, qui contient au moins un substituant Z⁷ et qui contient en outre deux ou trois substituants supplémentaires, qui sont choisis indépendamment les uns des autres parmi Z⁷ et Z¹, ou
Z² représente hydrogène, halogène, cyano, hydroxy, nitro, -C(=O)NR³R⁴, -NR³R⁴, alkyle en C₁-C₆, alcényle en C₂-C₆, alcynyle en C₂-C₆, halogénoalkyle en C₁-C₆, halogénoalcényle en C₂-C₆, halogénoalcynyle en C₂-C₆, cycloalkyle en C₃-C₆, halogénocycloalkyle en C₃-C₈, alcoxy en C₁-C₆-alkyle en C₁-C₆, alcoxycarbonyle en C₁-C₆, alcoxy en C₁-C₄, halogénoalcoxy en C₁-C₄, alkylcarbonyloxy en C₁-C₆, alkylthio en C₁-C₄, halogénoalkylthio en C₁-C₄, cycloalkylthio en C₃-C₆ ou tri (alkyle en C₁-C₂)silyle,
Z³ représente un radical phényle, un radical naphtalényle ou un radical hétéroaryle à 5 ou 6 chaînons, qui peuvent contenir jusqu'à deux substituants, les substituants étant choisis indépendamment les uns des autres dans la liste suivante :
substituants sur le carbone : halogène, cyano, nitro, hydroxy, amino, -SH, alkyle en C₁-C₄, alcényle en C₂-C₄, alcynyle en C₂-C₄, halogénoalkyle en C₁-C₄, halogénoalcényle en C₂-C₄, halogénoalcynyle en C₂-C₄, alcoxyalkyle en C₂-C₄, alkylcarbonyle en C₁-C₆, halogénoalkylcarbonyle en C₁-C₆, alcoxycarbonyle en C₁-C₆, alcoxy en C₁-C₄, halogénoalcoxy en C₁-C₄, alcényloxy en C₂-C₆, alcynyloxy en C₂-C₆, alkylthio en C₁-C₄, halogénoalkylthio en C₁-C₄, alkylsulfonyle en C₁-C₄, halogénoalkylsulfonyle en C₁-C₄ ou alkylamino en C₁-C₄, di(alkyle en C₁-C₄)amino,
substituants sur l'azote : alkyle en C₁-C₆, alcényle en C₂-C₆, alcynyle en C₂-C₆, halogénoalkyle en C₁-C₆, halogénoalcényle en C₂-C₆, halogénoalcynyle en C₂-C₆, alcoxy en C₁-C₄-alkyle en C₁-C₄, phényle, benzyle, halogénoalkylsulfonyle en C₁-C₄, alcoxycarbonyle en C₁-C₆, halogénoalcoxycarbonyle en C₁-C₆, phénylsulfonyle,, alkylsulfonyle en C₁-C₄, -C(=O)H ou alkylcarbonyle en C₁-C₃, et
Z⁴ représente C(=O)H, cycloalkyle en C₇-C₈, cycloalkyle en C₃-C₆-cycloalkyle en C₃-C₆, cycloalcényle en C₃-C₆, alcoxy en C₄-C₆-alkyle en C₁-C₄, alcoxy en C₃-C₆-alkyle en C₂-C₄, alcoxy en C₁-C₃-alcoxy en C₅-C₆-alkyle en C₁-C₃, alkylthio en C₁-C₄-alkyle en C₁-C₂, alkylsulfinyle en C₁-C₄-alkyle en C₁-C₂, alkylsulfonyle en C₁-C₄-alkyle en C₁-C₂, alkylamino en C₁-C₄-alkyle en C₁-C₂, dialkylamino en C₁-C₂-alkyle en C₁-C₂, halogénoalkylamino en C₁-C₄-alkyle en C₁-C₂, cycloalkylamino en C₃-C₆-alkyle en C₁-C₂, alkylcarbonyle en C₅-C₆, halogénoalkylcarbonyle en C₁-C₄, cycloalkylcarbonyle en C₃-C₆, cycloalcoxycarbonyle en C₃-C₆, cycloalkyle en C₃-C₆-alcoxycarbonyle en C₁-C₂, cycloalkylaminocarbonyle en C₃-C₆, halogénoalcoxy en C₁-C₄-alkyle en C₁-C₂, hydroxyalkyle en C₅-C₆, alcoxy en C₅-C₆, halogénoalcoxy en C₅-C₆, cycloalcoxy en C₃-C₆, halogénocycloalcoxy en C₃-C₆, cycloalkylalcoxy en C₃-C₆, alcényloxy en C₂-C₆, halogénoalcényloxy en C₂-C₆, alcynyloxy en C₂-C₆, halogénoalcynyloxy en C₂-C₆, alcoxy en C₁-C₄-alcoxy en C₁-C₄, halogénoalkylcarbonyloxy en C₁-C₄, cycloalkylcarbonyloxy en C₃-C₆, alkylcarbonyle en C₁-C₄-alcoxy en C₁-C₄, alkylthio en C₅-C₆, halogénoalkylthio en C₅-C₆, alkylsulfinyle en C₅-C₆, halogénoalkylsulfinyle en C₅-C₆, alkylsulfonyle en C₁-C₄, halogénoalkylsulfonyle en C₁-C₄, cycloalkylsulfonyle en C₃-C₆, tri (alkyle en C₃-C₄) silyle, alkylsulfonylamino en C₁-C₄ ou halogénoalkylsulfonylamino en C₁-C₄,
Z⁵ représente benzyle, phényle, alcoxy en C₅-C₆, halogénoalcoxy en C₅-C₆, alcényloxy en C₂-C₄, alcynyloxy en C₂-C₄, alkylthio en C₅-C₆ ou halogénoalkylthio en C₅-C₆,
Z⁶ représente
substituants sur le carbone : cycloalkylcyclopropyle en C₃-C₆, cycloalkylcyclohexyle en C₃-C₆ ou tri (alkyle en C₃-C₄) silyle,
substituants sur l'azote: alcényle en C₂-C₆, alcynyle en C₂-C₆, halogénoalkyle en C₁-C₆, cycloalkyle en C₃-C₆, alkyl en C₁-C₄-cycloalkyle en C₃-C₆, cycloalkylcyclopropyle en C₃-C₆, cycloalkylcyclohexyle en C₃-C₆, cycloalkyle en C₃-C₆-alkyle en C₁-C₄, alkylsulfonyle en C₁-C₄, C(=O)H, benzyle ou phényle,
L³ représente une liaison directe, -CH₂-, soufre, oxygène,
R³ et R⁴ sont identiques ou différents, et représentent indépendamment l'un de l'autre hydrogène, alkyle en C₁-C₄, alcényle en C₂-C₄, alcynyle en C₂-C₄, halogénoalkyle en C₁-C₄, cycloalkyle en C₅-C₆, benzyle ou phényle.

3. Composés de formule (I) selon la revendication 1, **caractérisés en ce que**
Y représente oxygène ;
R² représente hydrogène ;
Q représente Q-3 ;
R⁵ représente hydrogène ;
L¹ représente une liaison directe ;
R¹ représente 4,5-diméthyl-2-(prop-2-in-1-yloxy)phényle, ou
R¹ représente 2,6-difluoro-3-(prop-2-in-1-yloxy)phényle, ou
R¹ représente 2,6-difluoro-4-(prop-2-in-1-yloxy)phényle, ou
R¹ représente 2,6-difluoro-4-[(méthylsulfonyl)amino]phényle, ou
R¹ représente 3-(allyloxy)-2,6-difluorophényle.

4. Procédé de lutte contre des champignons phytopathogènes, **caractérisé en ce que** des composés de formule (I) selon la revendication 1 sur appliqués sur les champignons phytopathogènes et/ou leur habitat.

5. Agent de lutte contre des champignons phytopathogènes, **caractérisé par** une teneur en au moins un composé de formule (I) selon la revendication 1, en plus d'extendeurs et/ou de substances tensioactives.

6. Utilisation de composés de formule (I) selon la revendication 1 pour lutter contre des champignons phytopathogènes dans l'agriculture, l'horticulture et la sylviculture, dans la protection des matériaux et dans le domaine ménager et de l'hygiène.

7. Procédé de fabrication d'agents de lutte contre des champignons phytopathogènes, **caractérisé en ce que** des composés de formule (I) selon la revendication 1 sont mélangés avec des extendeurs et/ou des substances tensioactives.

8. Utilisation de composés de formule (I) selon la revendication 1 pour le traitement de plantes transgéniques.

9. Utilisation de composés de formule (I) selon la revendication 1 pour le traitement de graines et de graines de plantes transgéniques
